# EUROPÄISCHE PATENTSCHRIFT

(11) **EP 0 304 402 B1**
(45) Veröffentlichungstag und Bekanntmachung des Hinweises auf die Patenterteilung: **06.05.1992**
(21) Anmeldenummer: 88810549.1
(22) Anmeldetag: 12.08.1988
(51) Int. Cl.: C07C 335/20, C07C 335/32, C07C 267/00, C07C 233/43, C07C 331/28, A01N 47/30, A01N 47/42, A01N 47/40

(54) **Anilinophenylthioharnstoffe, -isothioharnstoffe und -carbodiimide, ihre Herstellung und Verwendung in der Schädlingsbekämpfung**
Anilinophenyl thioureas, isothioureas and carbodiimides, their preparation and their use in pest-control
Anilinophénylthiourées-isothiourées et carbodiimides, leur préparation et leur utilisation comme pesticide

(30) Priorität: 21.08.1987 CH 3213/87; 21.06.1988 CH 2382/88
(43) Veröffentlichungstag der Anmeldung: 22.02.1989
(73) Patentinhaber: CIBA-GEIGY AG, 4002 Basel (CH)
(72) Erfinder: Böger, Manfred, D-7858 Weil am Rhein 5 (DE); Drabek, Jozef, Dr., CH-4104 Oberwil (CH); Ehrenfreund, Josef, Dr., CH-4123 Allschwil (CH)

(56) Entgegenhaltungen:
- EP-A- 0 282 452
- EP-A- 0 296 120
- EP-A- 0 304 025
- EP-A- 0 307 361
- WO-A-86/05781
- BE-A- 888 179
- FR-A- 2 174 473
- FR-A- 2 465 720

## Beschreibung

Die vorliegende Erfindung betrifft neue substituierte Anilinophenylthioharnstoffe, -isothioharnstoffe und - carbodiimide, ihre Salze mit organischen und anorganischen Säuren, Verfahren und Zwischenprodukte zu ihrer Herstellung, Schädlingsbekämpfungsmittel, die diese Verbindungen enthalten, sowie ihre Verwendung in der Schädlingsbekämpfung.

Die erfindungsgemässen Verbindungen entsprechen der Formel

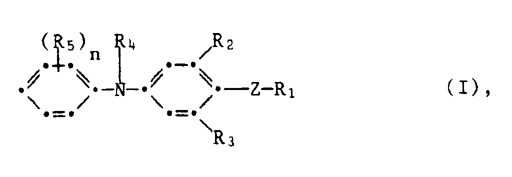

worin

R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-alkyl substituiertes C₃-C₈-Cycloalkyl oder C₃-C₈-Cycloalkyl-C₁-C₄-alkyl;

R₂ und R₃ je für Cₗ-C₅-Alkyl oder C₅-Cₛ-Cycloalkyl;

R₄ für Wasserstoff, Methyl oder -CHO;

R₅je für Halogen, C₁-C₄-Alkyl, ein- oder mehrfach durch Halogen substituiertes C₁-C₄-Alkyl, C₁-C₄-Alkyl, ein- oder mehrfach durch Halogen substituiertes C₁-C₄-Alkyl oder für eine -(CH=CH)₂, (̵CH₂)̵₃, (̵CH₂)̵₄-Brücke in 2,3- oder 3,4-Stellung;

n für 0, 1, 2, 3 oder 4;

Z für -NH-CS-NH-, -N=C(SR₆)-NH- oder -N=C=N-; und

R₆ für C₁-C₆-Alkyl oder Allyl stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl usw. und ihre Isomeren genannt.

Die als Substituenten in Betracht kommenden Alkoxyle können geradkettig oder verzweigt sein. Als Beispiele solcher Alkoxyle seien Methoxy, Aethoxy, Propoxy, Isopropoxy und Butoxy und seine Isomeren genannt.

Die als Substituenten in Betracht kommenden ein- oder mehrfach durch Halogen und/oder C₁-C₆-Alkoxy substituierten C₁-C₁₂-Alkyle können geradkettig oder verzweigt und nur teilweise oder auch perhalogeniert und/oder ein- bis fünffach durch C₁-C₆-Alkoxy substituiert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. CHF₂ oder CF₃; das ein- bis fünffach durch Fluor, Chlor und/oder Brom substituierte Aethyl wie z.B. CH₂CF₃, CF₂CF₃, CF₂CCI₃, CF₂CHCl₂, CF₂CHF₂, CF₂CFCI₂, CF₋ ₂CHBr₂, CF₂CHClF, CF₂CHBrF oder CCIFCHCIF; das ein- bis siebenfach durch Fluor, Chlor und/oder Brom substituierte Propyl oder Isopropyl wie z.B. CH₂CHBrCH₂Br, CF₂CHFCF₃, CH₂CF₂CF₃ oder CH(CF₃)₂; das einbis neunfach durch Fluor, Chlor und/oder Brom substituierte Butyl oder eines seiner Isomeren wie z.B. CF(CF₃- )CHFCF₃ oder CH₂(CF₂)₂CF₃; Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl, 1,2-Dimethoxyäthyl, 1,3-Dimethoxypropyl oder 2,4-Dimethoxybutyl.

Bei den als Substituenten in Betracht kommenden Cycloalkylen handelt es sich beispielsweise um Cyclopropyl, Cyclobutyl, Cyclopentyl oder Cyclohexyl. Diese können ein- oder mehrfach durch einen C₁-C₃-Alkylrest substituiert und/oder über eine C₁-C₄-Alkylenbrücke mit dem Rest des Moleküls verbunden sein.

Die Verbindungen der Formel in denen Z für -N=C(SR₆)-NH- steht, können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Jodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N=C(SRₛ)-NH- steht, können in den tautomeren Formen 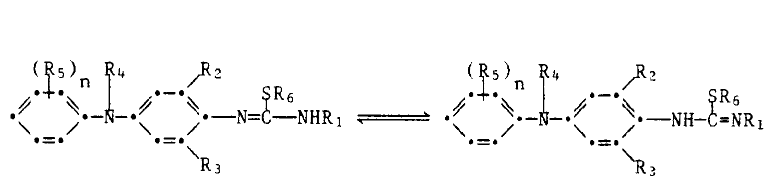 vorliegen. Die Erfindung umfasst sowohl die einzelnen Tautomeren, als auch Tautomerengemische.

Der mit R₅ substituierte Phenylrest kann, je nach der Grösse von n, mehrfach durch R₅ substituiert sein. Ist n grösser als 1, so können die verschiedenen R₅ gleiche oder verschiedene Bedeutung haben.

Unter den Verbindungen der Formel stehen diejenigen im Vordergrund, in denen R₁ für C₁-C₈-Alkyl, ein-oder mehrfach durch Halogen und/oder Cₗ-C₅-Alkoxy substituiertes C₁-C₈-Alkyl oder C₃-C₈-Cycloalkyl; R₂ für Cₗ-C₅-Alkyl; R₃ für C₁-C₅-Alkyl oder C₅-Cₛ-Cycloalkyl; R₄ für Wasserstoff, Methyl oder -CHO; R₅je für Halogen, C₁-C₃-Alkyl oder für eine (̵CH=CH)̵₂ oder (̵CH₂)̵₃ Brücke in 2,3- oder 3,4-Stellung; n für 0, 1 oder 2; Z für -NH-CS-NH-, N=C(SR₆)-NH- oder -N=C=N-; und R₆ für C₁-C₄-Alkyl oder Allyl stehen.

Dabei sind diejenigen Verbindungen der Formel I bevorzugt, in denen
a) R₁ für C₁-C₅-Alkyl oder Cyclopentyl; R₂ und R₃ für C₁-C₄-Alkyl; R₄ für Wasserstoff, Methyl oder -CHO;
R₅ für Methyl; n für 0 oder 1; und Z für -NH-CS-NH- stehen oder
b) R₁ für C₁-C₅-Alkyl oder Cyclopentyl; R₂ und R₃ für C₁-C₄-Alkyl; R₄ für Wasserstoff, Methyl oder -CHO;
R₅ für Methyl; n für 0 oder 1; Z für -NH-C(SRₛ)-NH-; und R₆ für C₁-C₄-Alkyl stehen oder
c) R₁ für C₁-C₅-Alkyl oder Cyclopentyl; R₂ und R₃ für C₁-C₄-Alkyl; R₄ für Wasserstoff, Methyl oder -CHO; R₅ für Methyl; n für 0 oder 1; und Z für -N=C=N- stehen.

Die erfindungsgemässen Verbindungen der Formel können nach im Prinzip bekannten Verfahren hergestellt werden, indem man z.B.
A) ein Isothiocyanat der Formel II 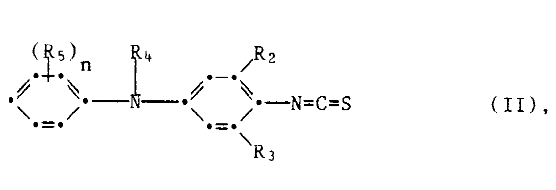 mit einem Amin der Formel III 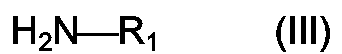 zum Thioharnstoff umsetzt und gegebenenfalls
B) den erhaltenen Thioharnstoff mit einer Verbindung der Formel IV 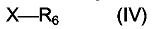 zum Isothioharnstoff umsetzt oder
C) den erhaltenen Thioharnstoff durch Abspalten von Schwefelwasserstoff in das Carbodiimid überführt. R₁, R₂, R₃, R₄, R₅, R₆ und n haben dabei die angegebene Bedeutung und X steht für eine geeignete Abgangsgruppe wie z.B. ein Halogenatom, insbesondere Chlor, Brom oder Jod, oder Alkylsulfat.

Das Verfahren Awird üblicherweise unter normalem Druck, und in Gegenwart eines organischen Lösungs-oder Verdünnungsmittels durchgeführt. Die Temperatur beträgt dabei zwischen 0 und +150_{°}C, vorzugsweise +10 bis 70°C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid.

Das Verfahren C wird zweckmässigerweise in einem aprotischen organischen Lösungs- oder Verdünnungsmittel unter normalem Druck durchgeführt. Die Temperatur beträgt dabei zwischen 0 und +150_{°}C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6,1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel 11 können nach im Prinzip bekannten Methoden hergestellt werden, z.B. indem man ein Aminodiphenylamin der Formel V

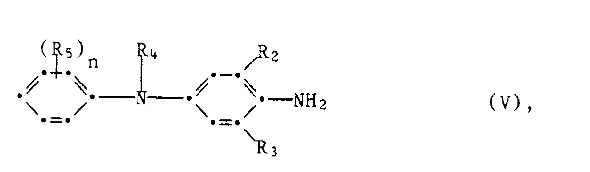

mit Thiophosgen umsetzt, wobei R₂, R₃, R₄, R₅ und n die für Formel angegebene Bedeutung haben.

Das Verfahren zur Herstellung der Verbindungen der Formel II wird zweckmässigerweise in Gegenwart einer organischen oder anorganischen Base, wie. z.B. Triäthylamin oder Calciumcarbonat, und eines gegenüber den Reaktionsteilnehmern inerten Lösungs- oder Verdünnungsmittels unter normalem Druck durchgeführt. Die Temperatur beträgt dabei zwischen 0 und +100_{°}C, vorzugsweise Siedetemperatur des verwendeten Lösungs- oder Verdünnungsmittels oder +20 bis 80°C. Geeignete Lösungs- und Verdünnungsmittel sind u.a. Aether oder ätherartige Verbindungen, wie z.B. Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon; oder chlorierte Kohlenwasserstoffe wie z.B. Dichlormethan. Die Herstellung kann auch in Anwesenheit von Wasser im 2-Phasensystem erfolgen.

Eine andere Möglichkeit zur Herstellung der Isothiocyanate der Formel II besteht im Umweg über den entsprechenden, einseitig unsubstituierten Thioharnstoff. Dabei wird ein Anilin der Formel V mit Ammoniumrhodanid in saurem, vorzugsweise mineralsaurem Medium zum entsprechenden Thioharnstoff umgesetzt, der seinerseits beim Erhitzen auf +130 bis 200°C Ammoniak abspaltet und in ein Isothiocyanat der Formel II übergeht (vgl. Saul Patai "The chemistry of cyanates and their thio derivatives", John Wiley and Sons, 1977; Chemistry and Industry, July 3,1954, p. 735. J.N. Baxter et al., "New method of preparation of aryl isothiocyanates").

Die Aminodiphenylamine der Formel V können nach im Prinzip bekannten Methoden hergestellt werden, z.B. indem man ein Anilin der Formel VI

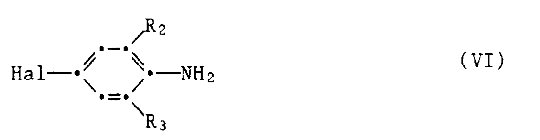

mit einem Formanilid der Formel VII 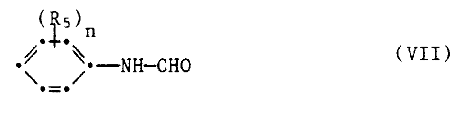 umsetzt, wobei R₂, R₃, R₅ und n die für Formel 1 angegebene Bedeutung haben und Hal für Halogen, insbe-4

sondere für Chlor oder Brom steht. Diese N-Formyl-aminodiphenylamine werden gegebenenfalls durch Erwärmen in alkalischer Lösung in die entsprechenden N-unsubstituierten Aminodiphenylamine (R₄ = H) oder durch Reduktion z.B. mit einem Boran-methylsulfid-Komplex (Tetrahedron Letters, Vol. 23, Nr. 33, pp. 3315-3318) oder durch katalytische Hydrierung, wobei die üblichen Hydrierungskatalysatoren, insbesondere Rheniumoxide verwendet werden können, in die entsprechenden N-methyl-aminodiphenylamine (R₄ = CH₃) übergeführt.

Das Verfahren zur Herstellung der Aminodiphenylamine der Formel V erfolgt zweckmässigerweise in Analogie zur Goldberg-Diphenylamin-Synthese (J. Amer. Chem., Soc., 1928, 50, 859) bei Temperaturen zwischen +150 und 180°C und in Gegenwart eines Schwermetall-Katalysators wie z.B. Kupferpulver.

Die Verbindungen der Formeln 11 und V sind neu und bilden ebenfalls einen Gegenstand der vorliegenden Erfindung. Die Verbindungen der Formeln 111, IV, VI und VII sind dagegen bekannt oder können nach im Prinzip bekannten Methoden hergestellt werden.

Aus der FR-Patentanmeldung 2.465.720 bzw. der BE-PS 888.179 sind substituierte N-Phenoxyphenyl-N'- alkyl-thioharnstoffe und -isothioharnstoffe mit insektizider und akarizider Wirkung bekannt. Ferner werden in der PCT-Patentanmeldung WO 86/05781 pestizid wirksame N-Phenoxyphenyl-N'-benzoylharnstoffe beschrieben. Demgegenüber unterscheiden sich die erfindungsgemässen Thioharnstoff- und Isothioharnstoff-Verbindungen der Formel 1 strukturell im wesentlichen durch das Vorliegen einer N-Anilinophenylgruppierung.

Ueberraschenderweise wurde gefunden, dass die erfindungsgemässen Verbindungen der Formel 1 bei günstiger Warmblüter- und Pflanzenverträglichkeit wertvolle Wirkstoffe in der Schädlingsbekämpfung sind. So eignen sich die Verbindungen der Formel z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder-äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die alkali-, Erdalkali- oder gegebenenfalls substituierten Ammoniumsalze von höheren Fettsäuren (C_{1O}-C₂₂), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkalioder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seinen Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch",
Carl Hanser Verlag München/Wien 1981.

Die pestiziden Zubereitungen enthalten in der Regel 0,1 bis 99 %, insbesondere 0,1 bis 95 %, Wirkstoff der Formel oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

### Beispiel 1: Herstellung

### 1.1. Zwischenprodukte

### 1.1.1. 4-(N-Phenyl-N-formylamino)-aniline

### 1.1.1.1. 2,6-Diäthyl-4-(N-phenyl-N-formylamino)-aniline

36,3 g Formanilid, 70 g 2,6-Diäthyl-4-bromanilin, 42,0 g Kaliumcarbonat und 1 g Kupferpulverwerden vorgelegt und 16 Stunden lang bei +160_{°}C und unter Stickstoff gerührt. Das erkaltete Reaktionsgemisch wird mit 200 ml Toluol und 200 ml Wasser versetzt und über Kieselgur filtriert. Die Toluolphase wird abgetrennt, über Natriumsulfat getrocknet und eingedampft. Der Rückstand wird in einem Hexan/Essigester-Gemisch (80:20) aufgenommen und erneut über Kieselgur filtriert. Das Filtrat wird eingedampft, mit Isopropanol versetzt, abgenutscht und mit Hexan gewaschen. Die Titelverbindung der Formel

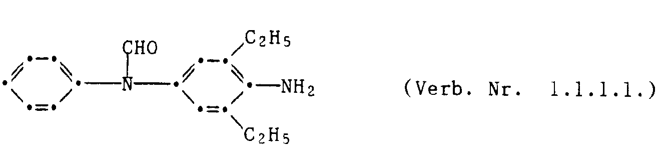

liegt in Form eines beigefarbenen Pulvers vor; Smp. 105-107°C.

Auf analoge Weise werden die folgenden Verbindungen hergestellt:

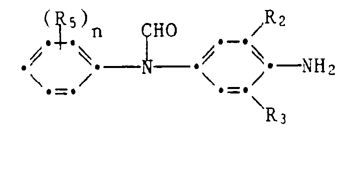

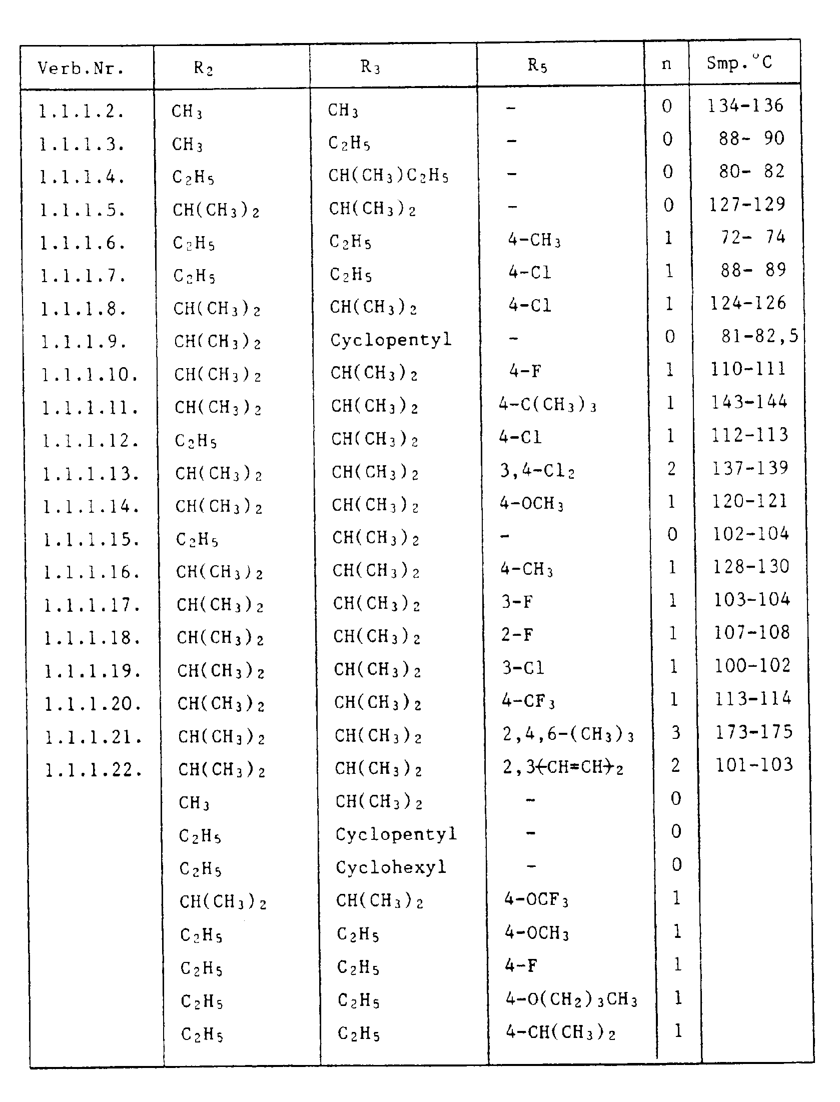

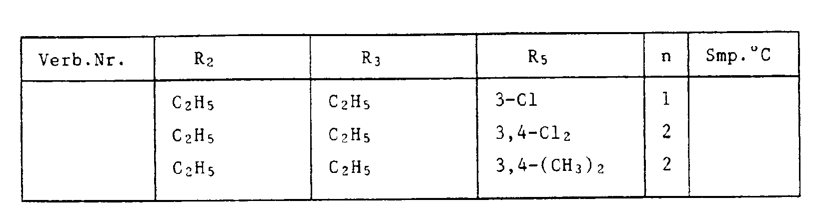

### 1.1.2. 4-Aminodiphenylamine

### 1.1.2.1. 2,6-Diäthyl-4-(N-phenylamino)-anilin

6,8 g 2,6-Diäthyl-4-(N-phenyl-N-formylamino)-anilin, 60 ml 10%ige wässrige Natronlauge und 5 ml Polyäthylenglykol (M 200) werden vorgelegt und 2 Stunden lang bei +100_{°}C gerührt. Das erkaltete Reaktionsgemisch wird mit 150 ml Toluol versetzt und die abgetrennte organische Phase dreimal mit Wasser gewaschen, über Natriumsulfat getrocknet und eingedampft. Der Rückstand wird aus einem Toluol/ Hexan-Gemisch umkristallisiert. Die Titelverbindung der Formel 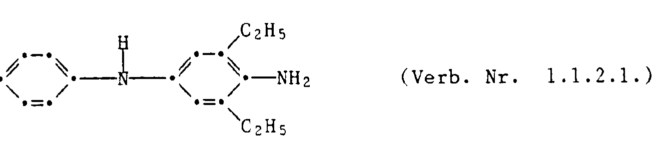 liegt in Form eines beigefarbenen Pulvers vor; Smp. 42-44°C.

Auf analoge Weise die folgenden Verbindungen hergestellt:

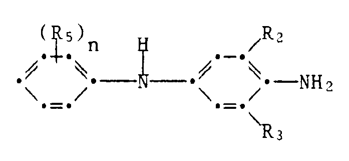

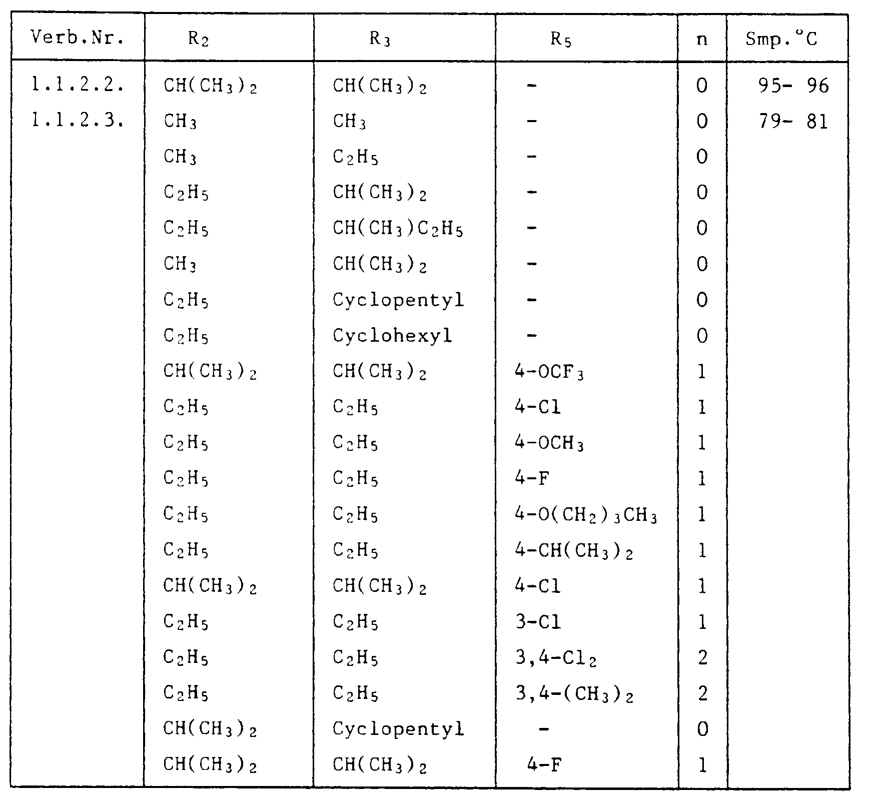

### 1.1.3. 4-(N-Phenyl-N-methylamino)-aniline)

### 1.1.3.1. 2,6-Diäthyl-4-(N-phenyl-N-methylamino)-anilin

A. 7,3 g 2,6-Diäthyl-4-(N-phenyl-N-formylamino)-anilin gelöst in 30 ml Tetrahydrofuran werden unter Rühren und schwachem Stickstoffstrom tropfenweise bei 0°C und 6,3 ml des Boran-dimethylsulfid-Komplexes (CH₃)₂S.BH₃ versetzt. Das Reaktionsgemisch wird 3 Stunden lang unter Rückfluss gerührt, dann auf 0°C gekühlt, tropfenweise mit 30 ml Methanol versetzt und schliesslich zur Trockne eingedampft. Der Rückstand wird in 80 ml Toluol und 30 ml Wasser aufgenommen, die organische Phase abgetrennt und über Natriumsulfat und Kieselgel getrocknet. Nach Entfernung des Lösungsmittels am Vakuum liegt die Titelverbindung der Formel 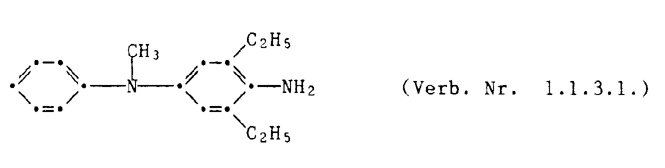 als braunes Oel vor, Brechungsindex nö: 1,5908.

### 1.1.3.2. 2,6-Diisopropyl-4-(N-phenyl-N-methylamino)-anilin

B. 10,4 g 2,6-Diisopropyl-4-(N-phenyl-N-methylamino)-anilin werden in 100 ml Tetrahydrofuran gelöst und in Gegenwart von 0,7 g Re₂0₇ bei +245_{°}C und 150 bar während 20 Stunden im Autoklaven hydriert. Das erkaltete Reaktionsgemisch wird über Kieselgur filtriert und das Filtrat eingedampft. Der ölige Rückstand wird alsdann in einem Lösungsmittelgemisch Hexan/Essigester (98:2) aufgenommen und über Kieselgel chromatographiert. Nach dem Eindampfen des Lösungsmittels liegt die Titelverbindung der Formel 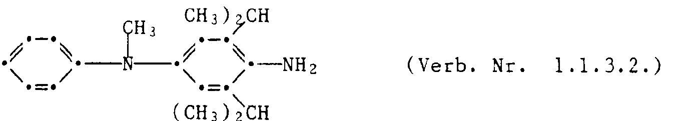 als hellbraunes Oel vor; Brechungsindex nö: 1,5865.

In Analogie zur Methode A oder B können die folgenden Verbindungen hergestellt werden:

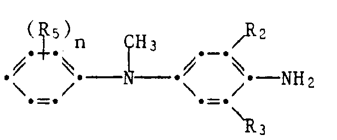

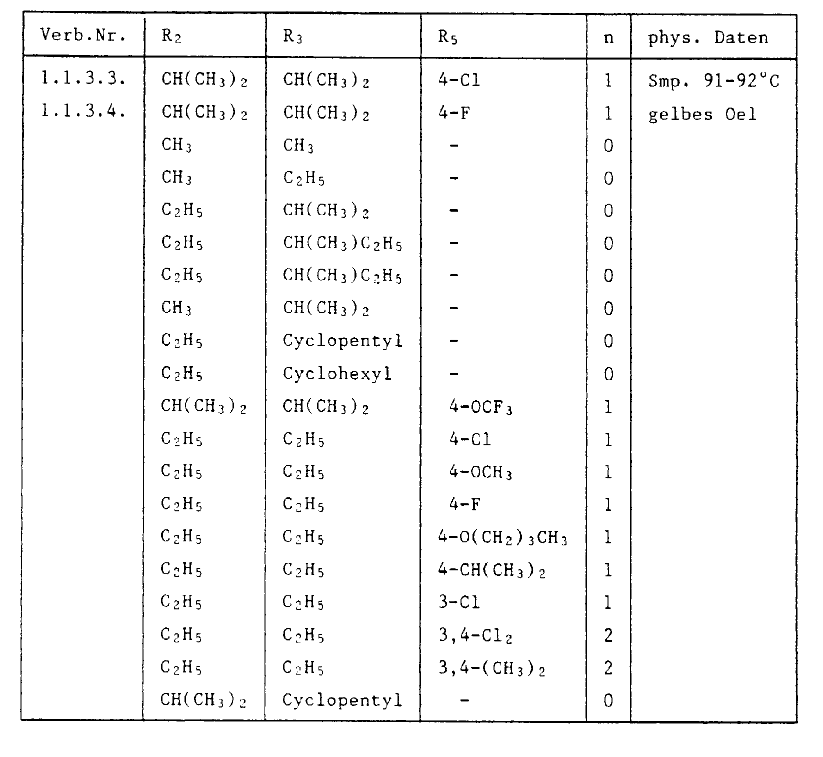

### 1.1.4. Arylaminophenylisothiocyanate

### 1.1.4.1. 2,6-Diäthyl-4-(N-formylanilino)-phenyl-isothiocyanat

24,0 g 2,6-Diäthyl-4-(N-phenyl-N-formylamino)-anilin gelöst in 80 ml Dichlormethan werden unter Rühren tropfenweise bei 0 bis +10°C zu 13,4 g Thiophosgen, 100 ml Dichlormethan, 60 ml Wasser und 19 g Calciumcarbonat gegeben. Das Reaktionsgemisch wird 2 Stunden lang bei Raumtemperatur gerührt und dann filtriert. Die organische Phase wird abgetrennt, mit 50 ml Wasser gewaschen, über Natriumsulfat getrocknet und anschliessend eingedampft. Die Titelverbindung der Formel 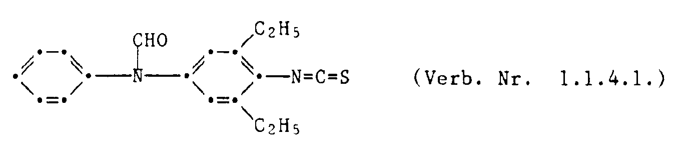 liegt als kristallines Pulver vor; Smp. 77-78°C.

Auf analoge Weise werden die folgenden Verbindungen hergestellt:

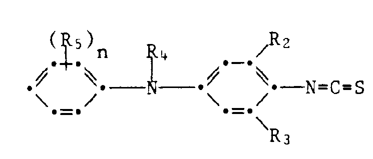

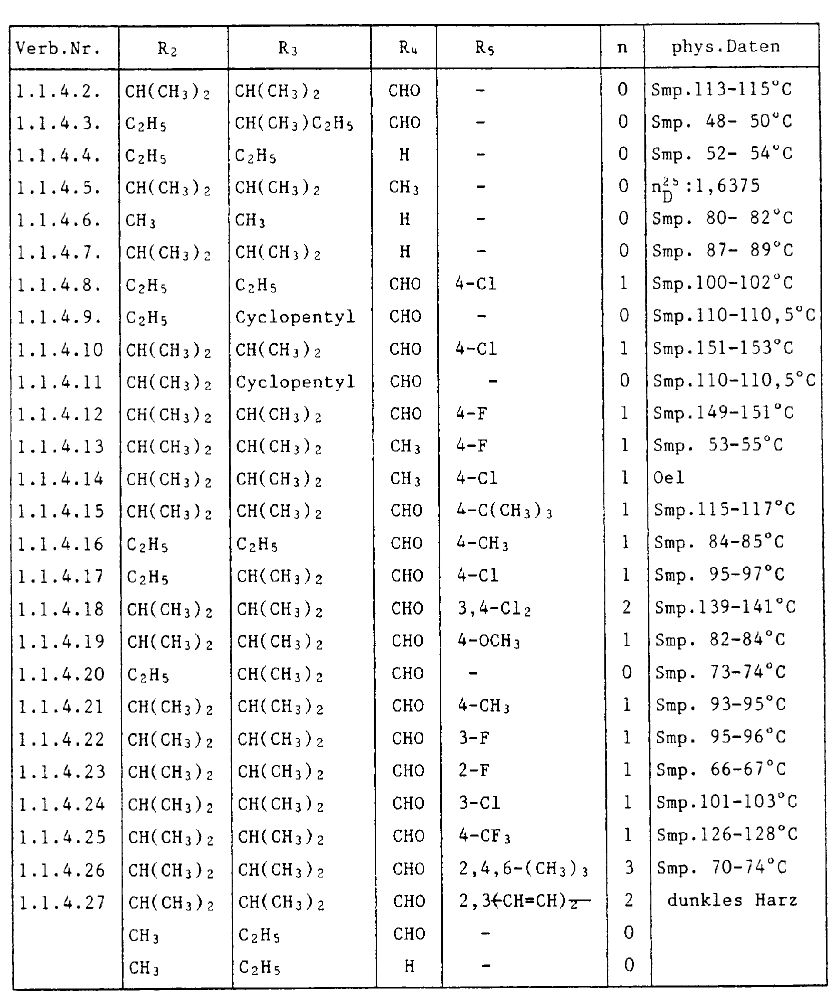

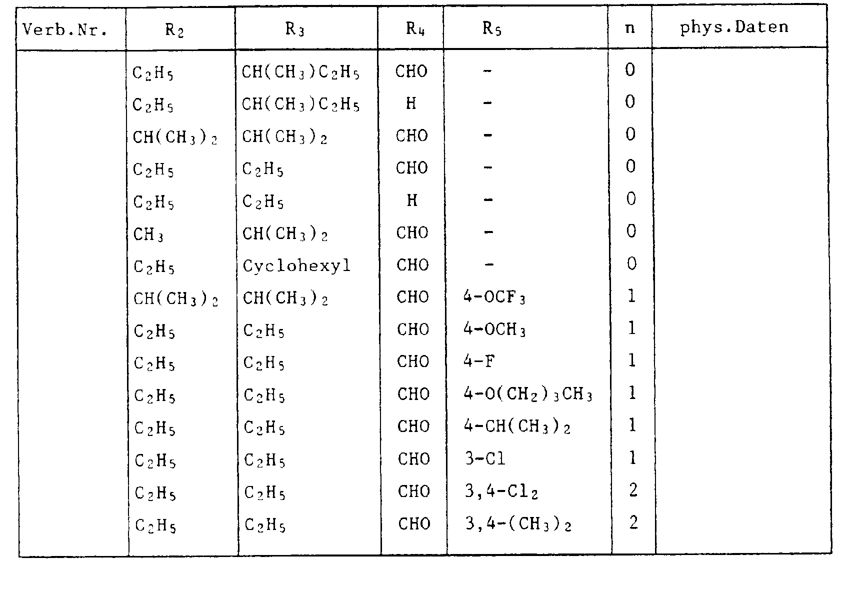

### 1.2. Endprodukte

### 1.2.1. Arylaminophenylthioharnstoffe

### 1.2.1.1. N-[2,6-Diäthyl-4-(N-formylanilino)pheny]-N-tert.butyl-thioharnstoff

14,0 g 2,6-Diäthyl-4-(N-formylanilino)-phenyl-isothiocyanat werden in 30 ml Toluol und 100 ml Hexan gelöst und unter Rühren bei +50_{°}C tropfenweise mit 3,7 g tert.Butylamin versetzt. Das Reaktionsgemisch wird 2 Stunden lang bei +60_{°}C gerührt. Alsdann wird der entstandene Niederschlag abfiltriert, mit Hexan gewaschen und getrocknet. Die Titelverbindung der Formel 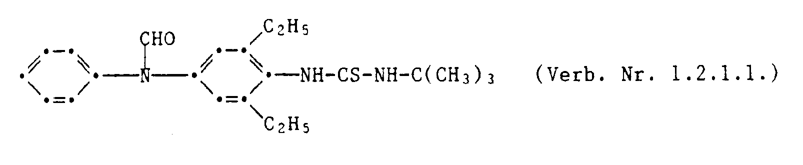 liegt als hellbeiges Pulver vor; Smp. 160-162°C.

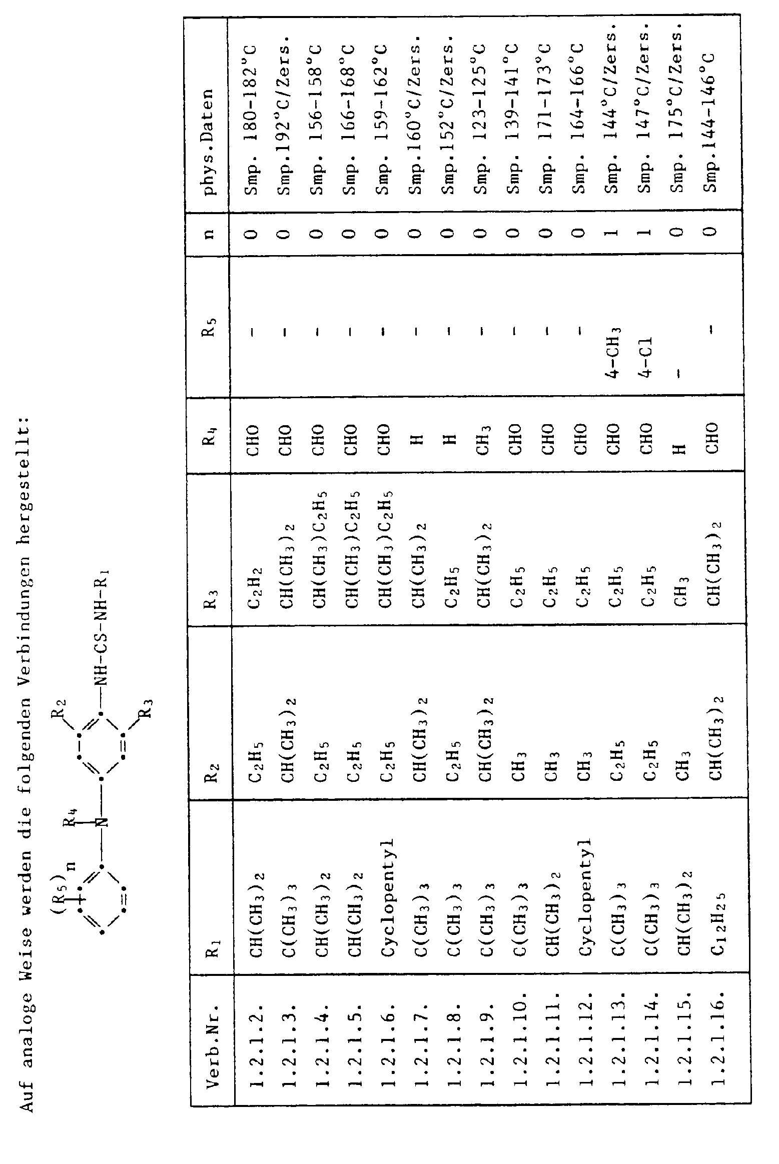

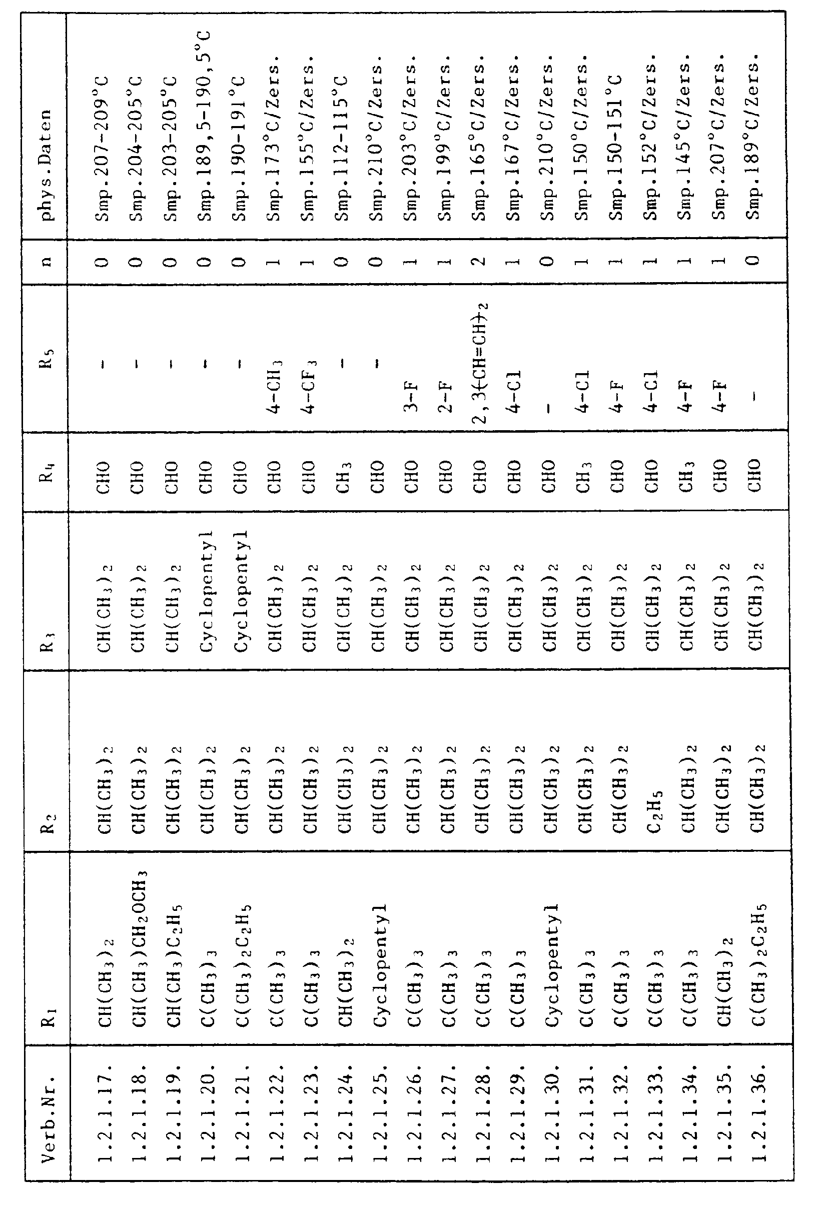

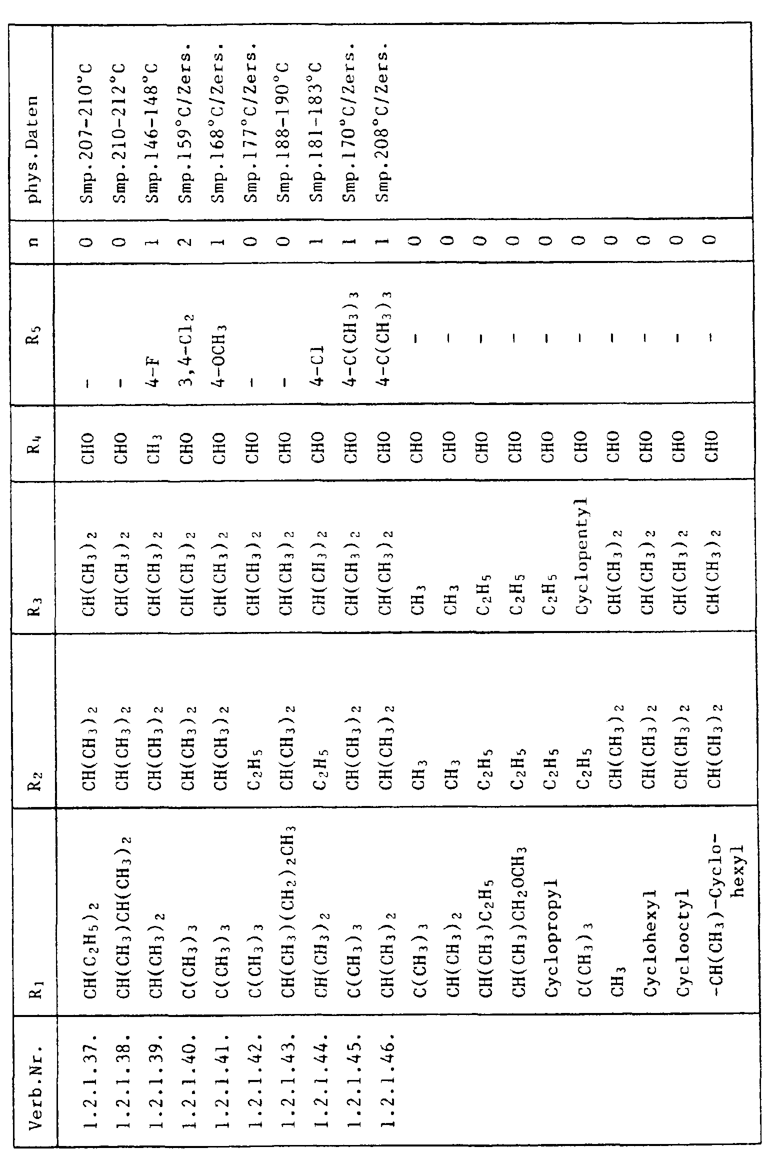

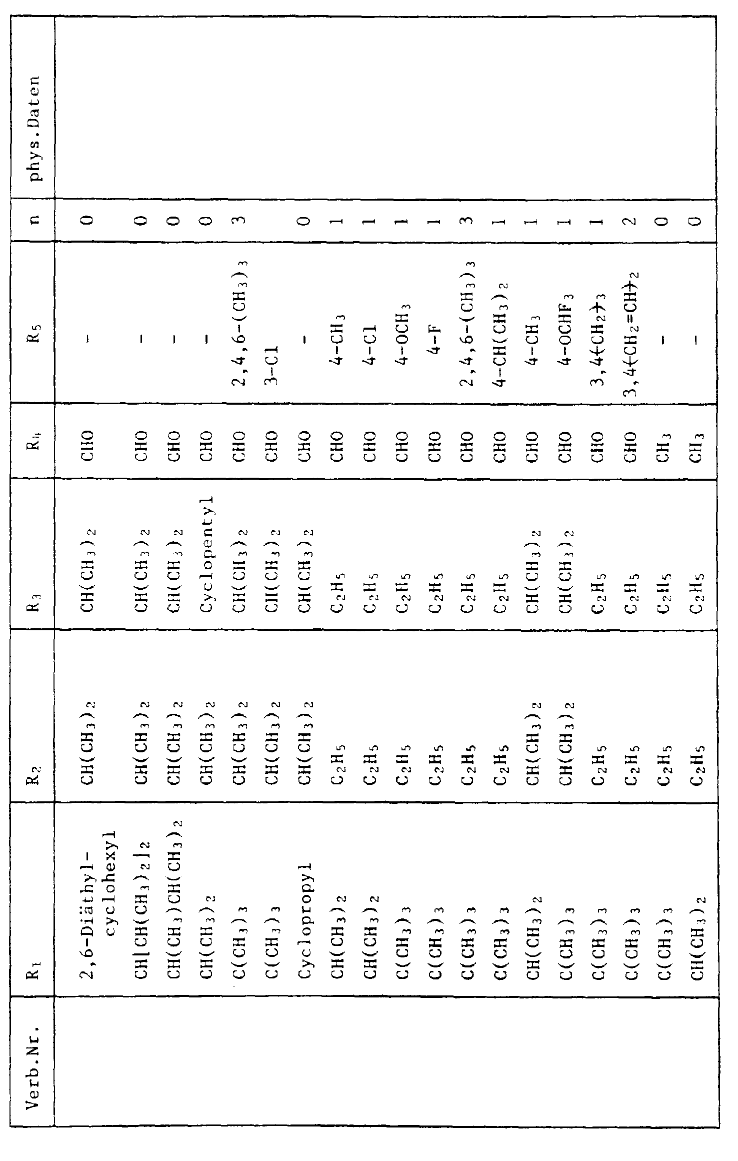

### 1.2.2. Arylaminophenylisothioharnstoffe

### 1.2.2.1. N-[2,6-Diäthyl-4-(N-formylanilino)pheny]-N'-tert.butyl-S-methyl-isothioharnstoff

5,2 g N-[2,6-Diäthyl-4-(N-formylanilino)-phenyl]-N'-tert.butyl-thioharnstoff werden in 50 ml Aethanol vorgelegt, bei Raumtemperatur mit 2,6 g Methyljodid versetzt und während 2 Stunden unter leichtem Rückfluss gerührt. Anschliessend wird das Lösungsmittel abgedampft und der Rückstand in 50 ml Dichlormethan und 40 ml 10%iger wässriger Sodalösung aufgenommen. Die organische Phase wird abgetrennt, über Natriumsulfat getrocknet und am Rotationsverdampfer eingedampft. Der Rückstand wird aus Toluol umkristallisiert. Die Titelverbindung der Formel

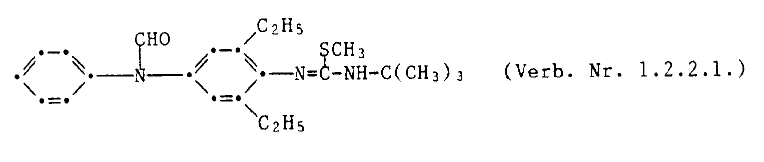

liegt als weisses Pulver vor; Smp. 168-170°C.

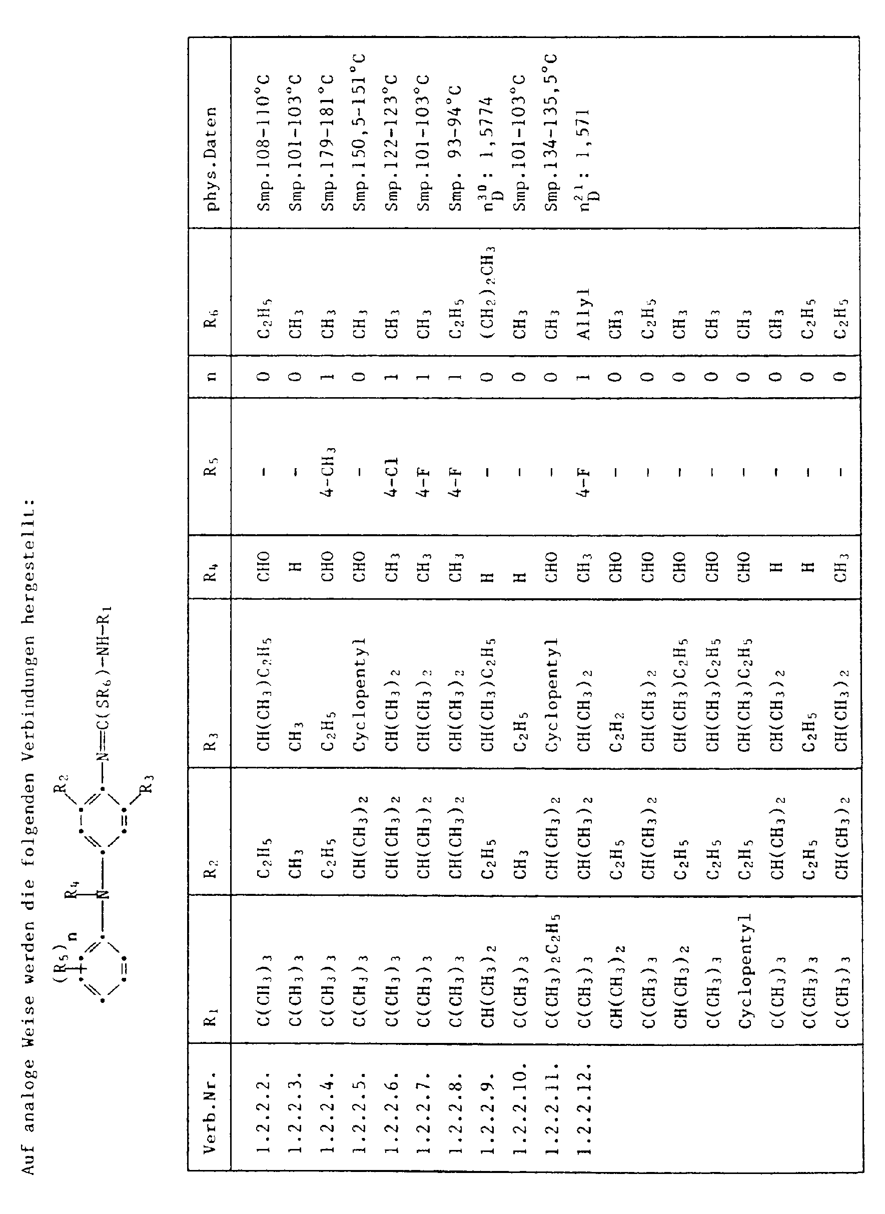

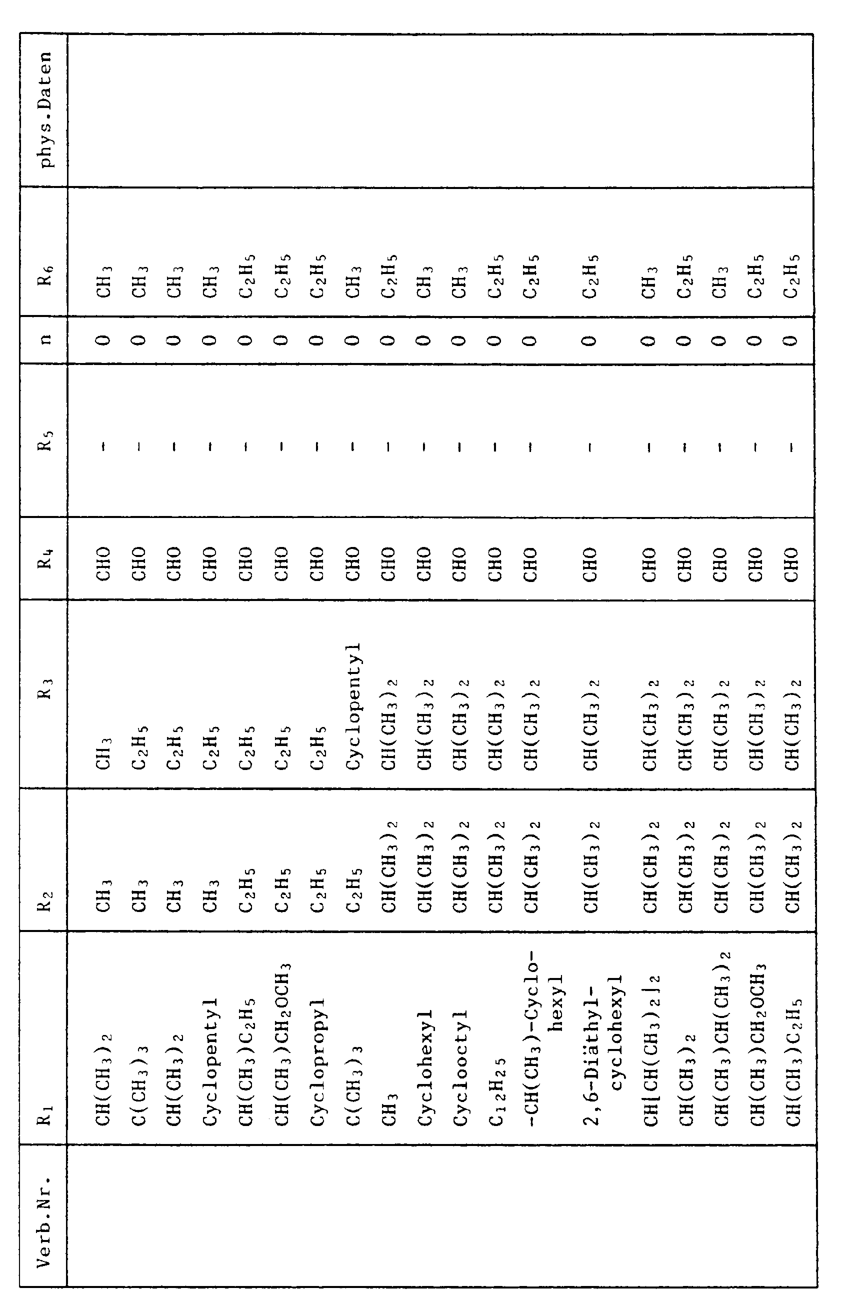

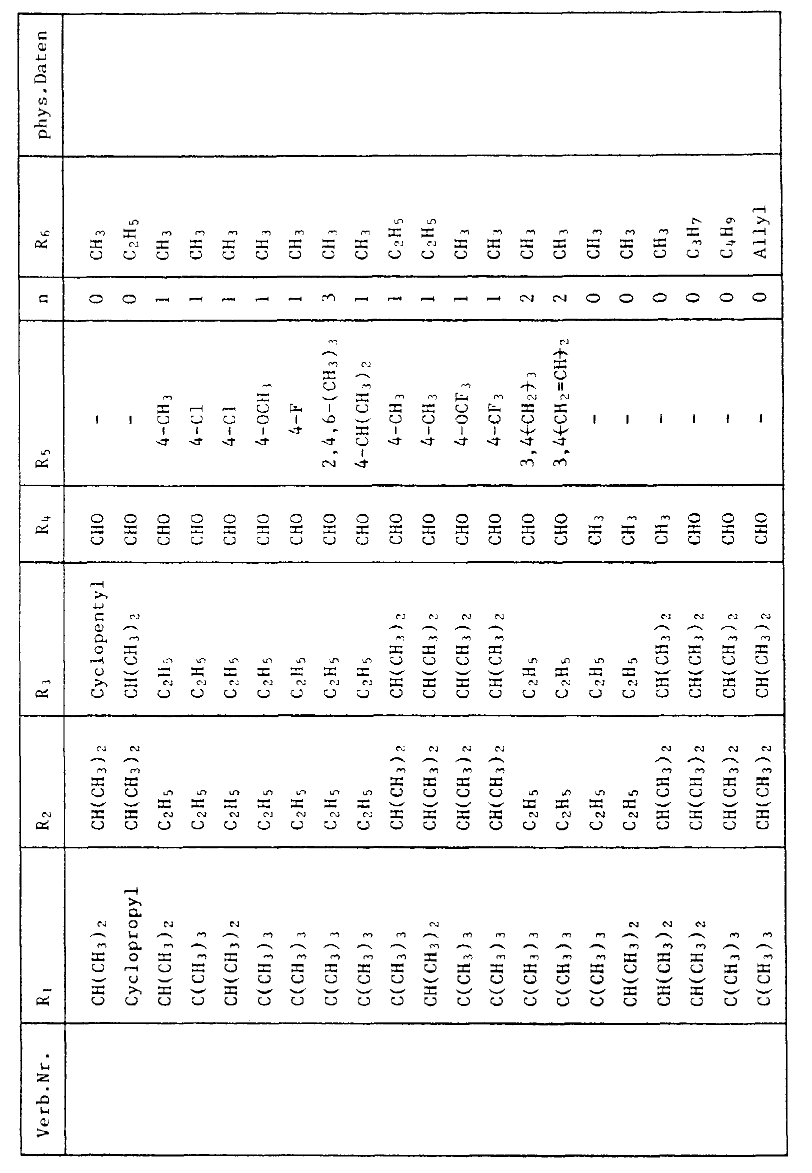

### 1.2.3. Arylaminophenylcarbodiimide

### 1.2.3.1. N-[2,6-Diäthyl-4-(N-formylanilino)pheny]-N-tert.butyl-carbodiimid

6,0 g N-[2,6,-Diäthyl-4-(N-formylanilino)-phenyl]-N'-tert.butyl-thioharnstoff und 4,8 g 2-Chlor-1-methylpyri- diniumjodid werden in 30 ml Acetonitril vorgelegt, bei Raumtemperatur tropfenweise unter Rühren mit 3,2 g Triäthylamin versetzt und 1 Stunde lang bei +70_{°}C gerührt. Anschliessend wird das Reaktionsgemisch am Rotationsverdampfer bei +50_{°}C eingedampft und der Rückstand in 50 ml Hexan und 30 ml kaltem Wasser aufgenommen. Die Hexanphase wird abgetrennt, mit Wasser gewaaschen, über Natriumsulfat getrocknet und schliesslich eingedampft. Die Titelverbindung der Formel

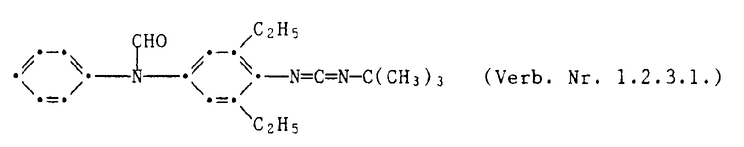

liegt als gelbliches Oel vor; Brechungsindex n 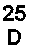 1,5840.

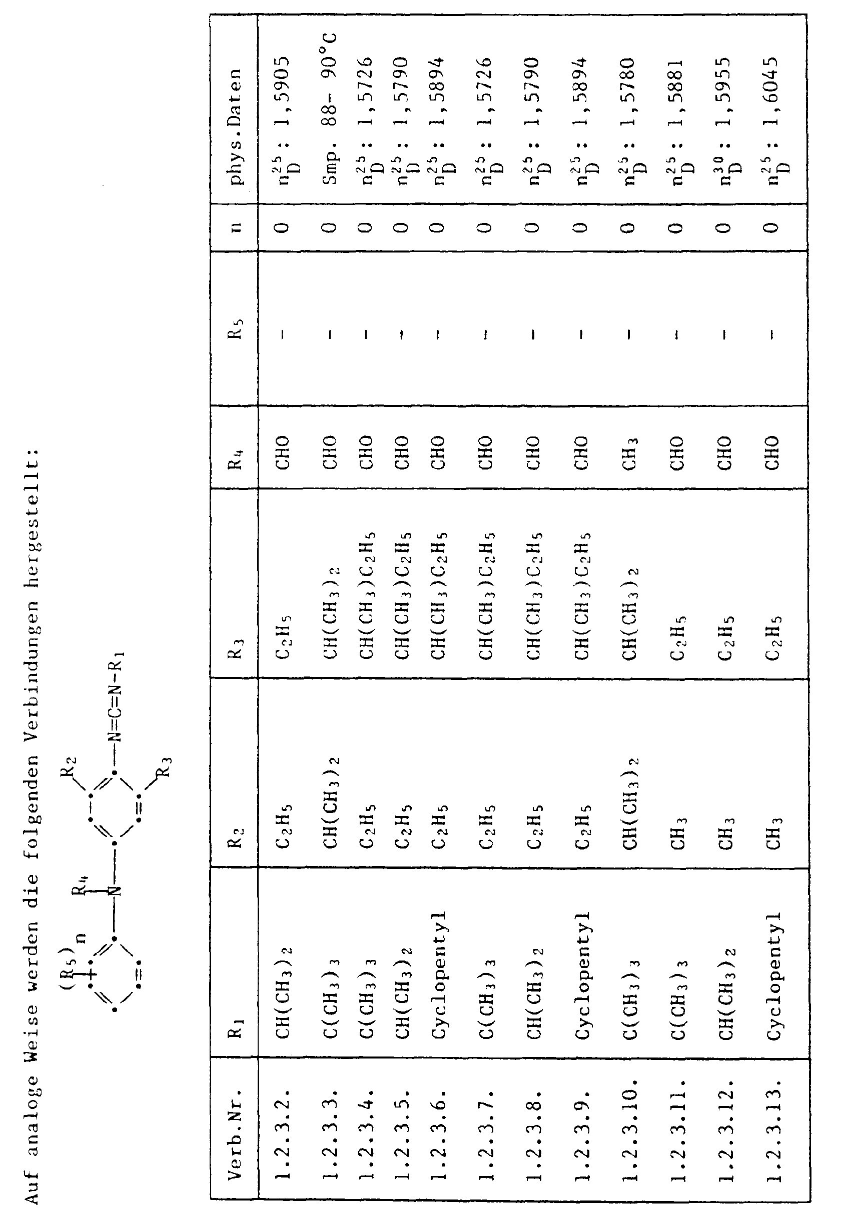

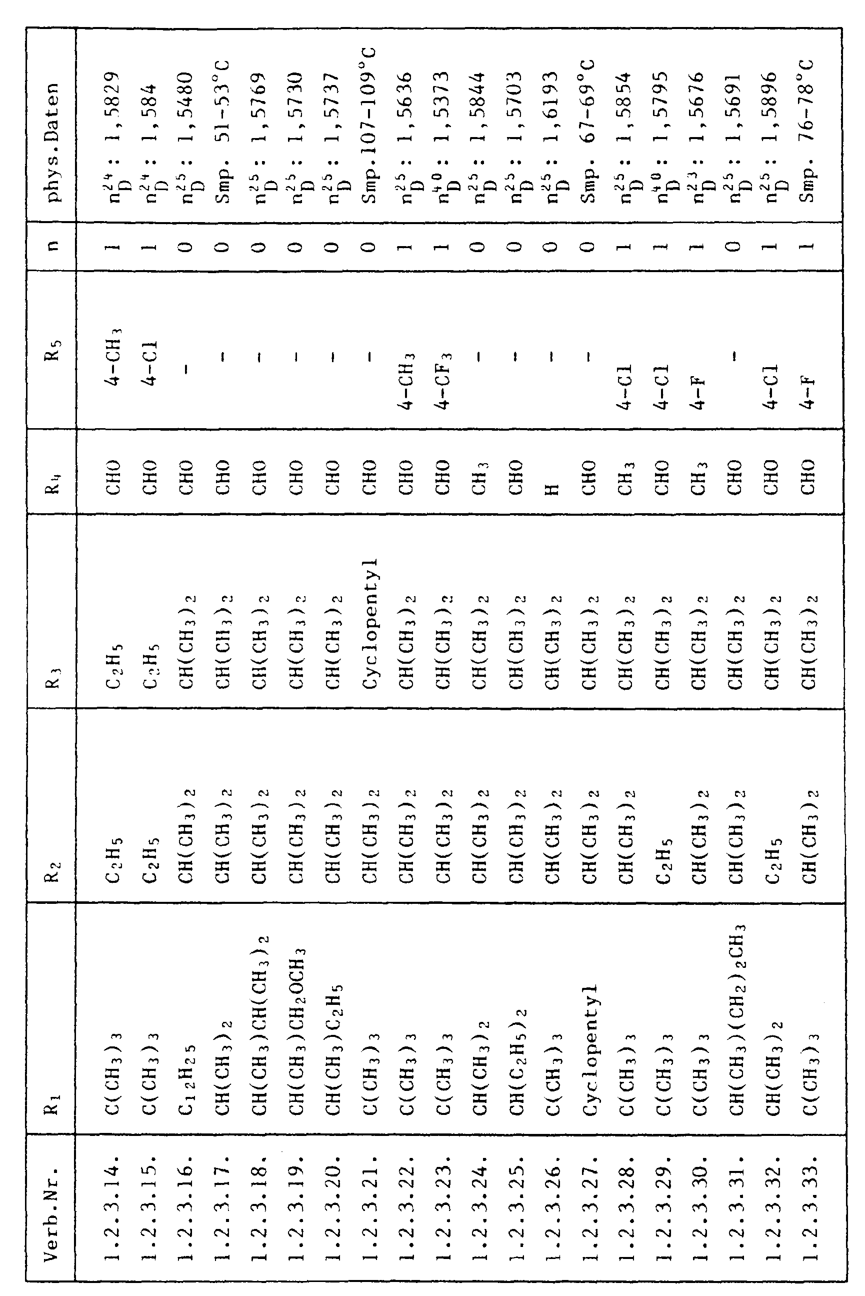

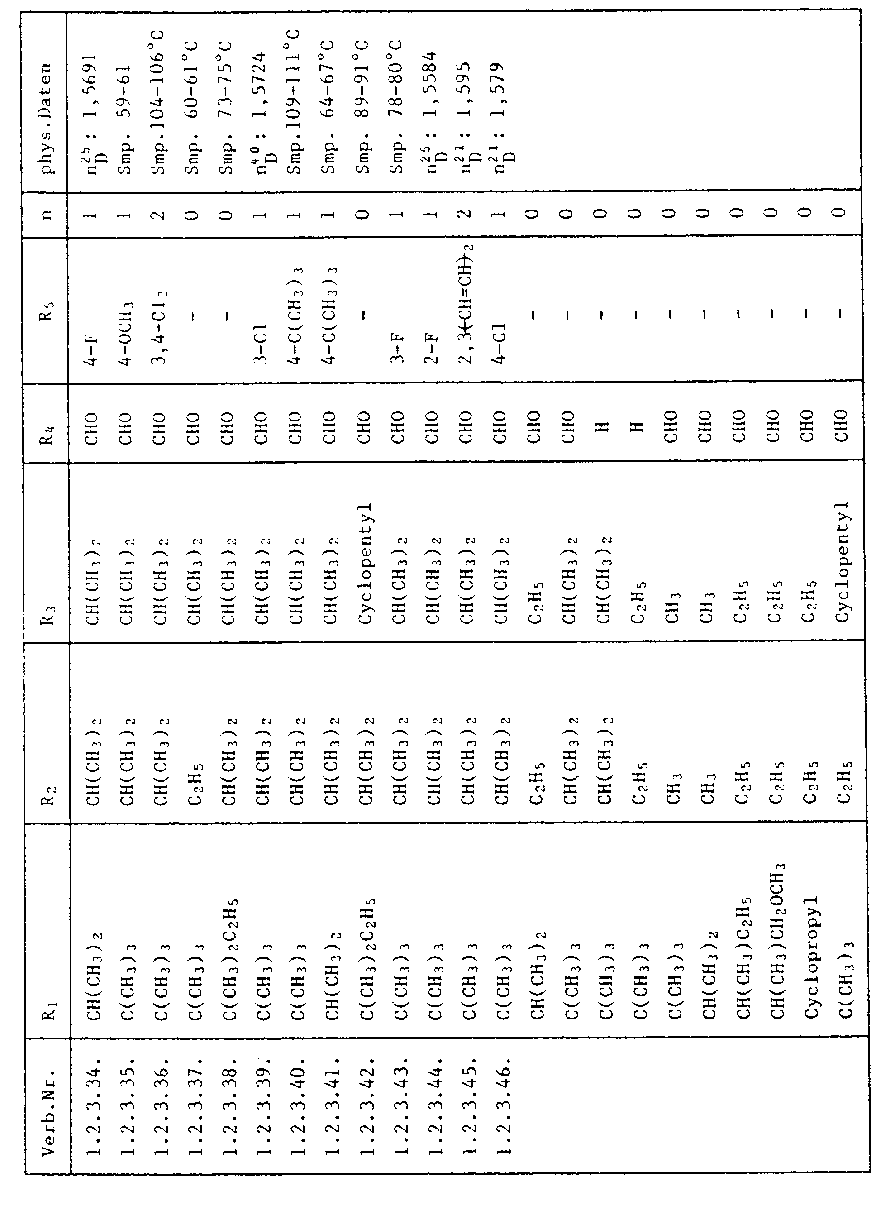

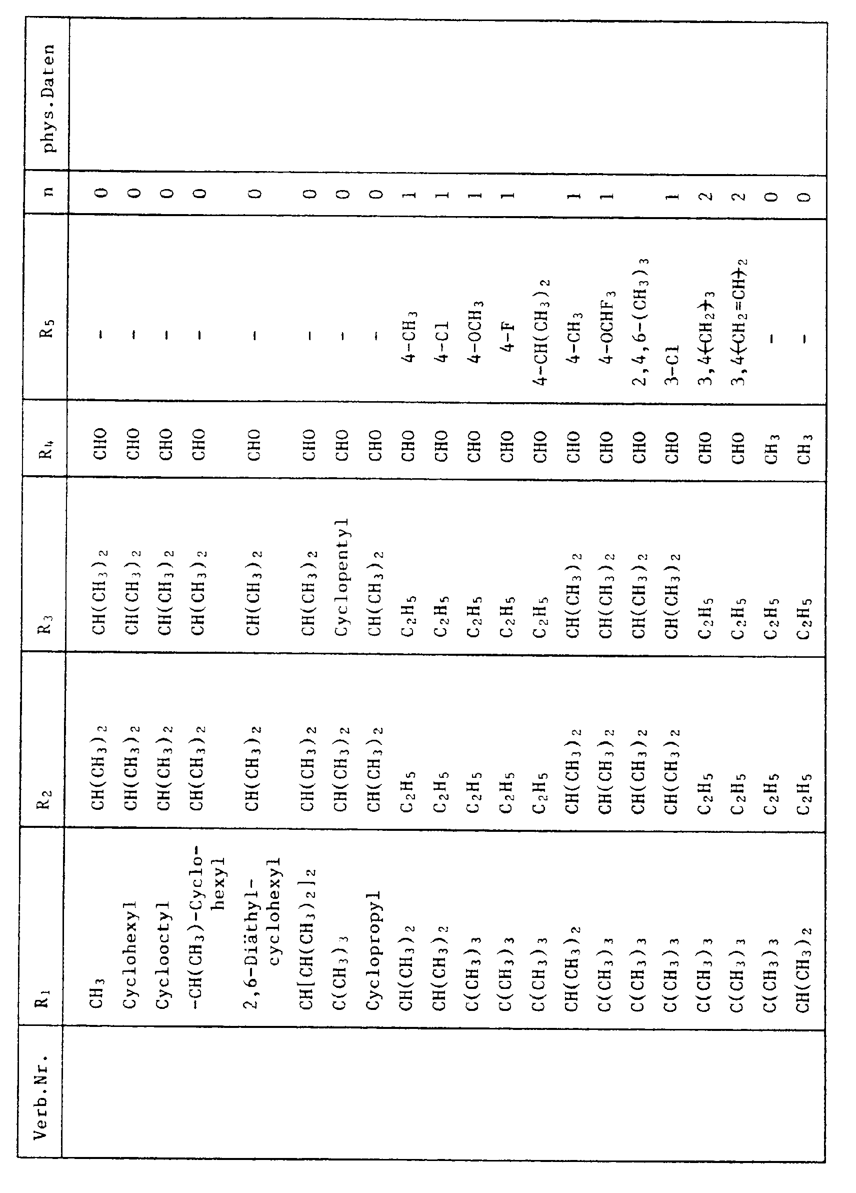

### Beispiel 2: Formulierungen von Wirkstoffen der Formel I gemäss den Herstellungsbeispielen 1.2.

### (% = Gewichtsprozent)

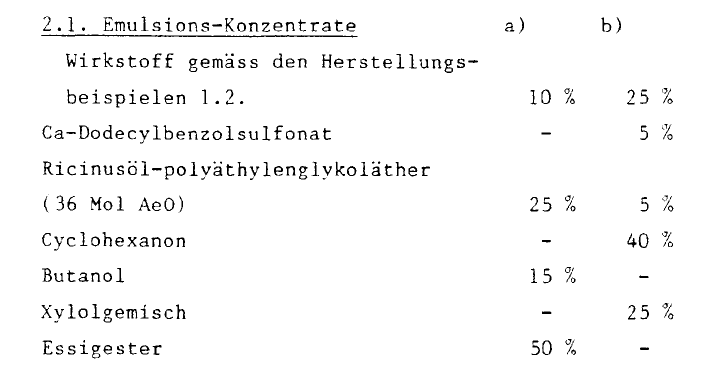

Aus solchen Konzentraten können durch Verdünnen mit Wasser Emulsionen jeder gewünschten Konzentration hergestellt werden.

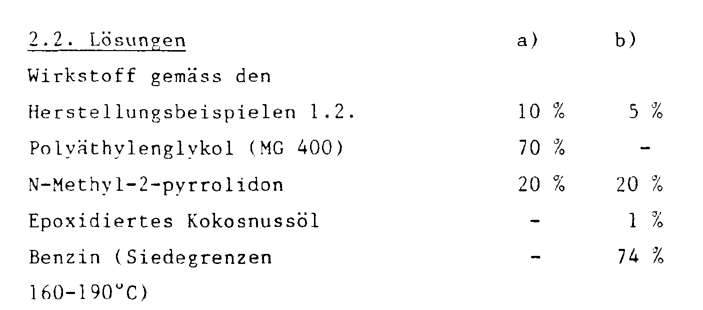

Die Lösungen sind zur Anwendung in Form kleinster Tropfen geeignet.

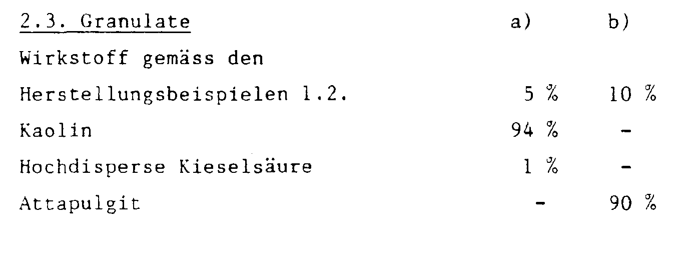

Der Wirkstoff wird in Methylenchlorid gelöst, auf den Träger aufgesprüht und das Lösungsmittel anschliessend im Vakuum abgedampft.

### 2.4. Extruder Granulat

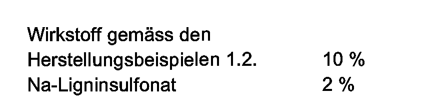

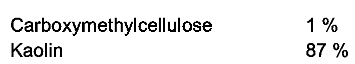

Der Wirkstoff wird mit den Zusatzstoffen vermischt, vermahlen und mit Wasser angefeuchtet. Dieses Gemisch wird extrudiert und anschliessend im Luftstrom getrocknet.

### 2.5. Umhüllungs-Granulat

### Wirkstoff gemäss den

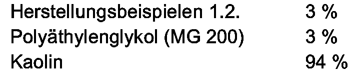

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

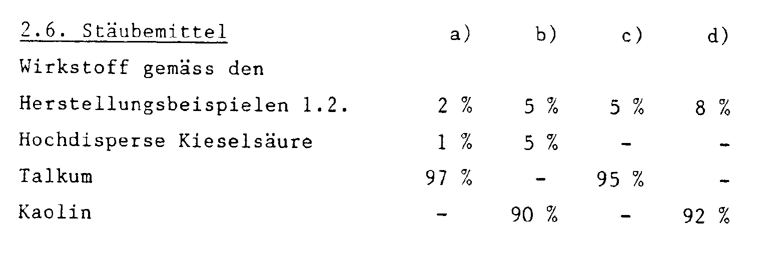

Durch inniges Vermischen der Trägerstoffe mit dem Wirkstoff undgegebenenfalls Vermahlen auf einer geeigneten Mühle erhält man gebrauchsfertige Stäubemittel.

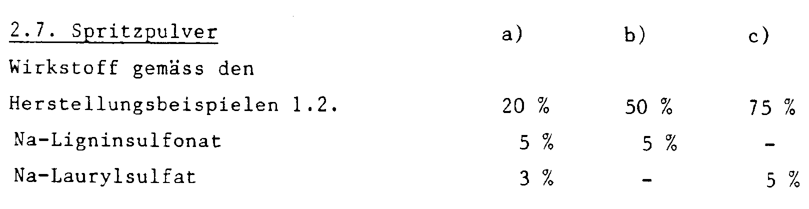

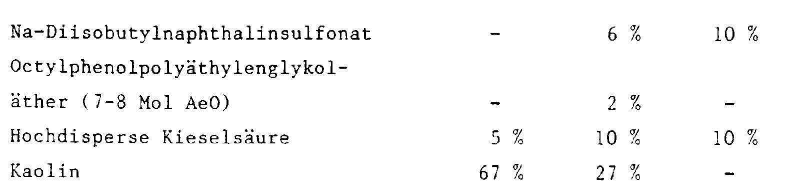

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

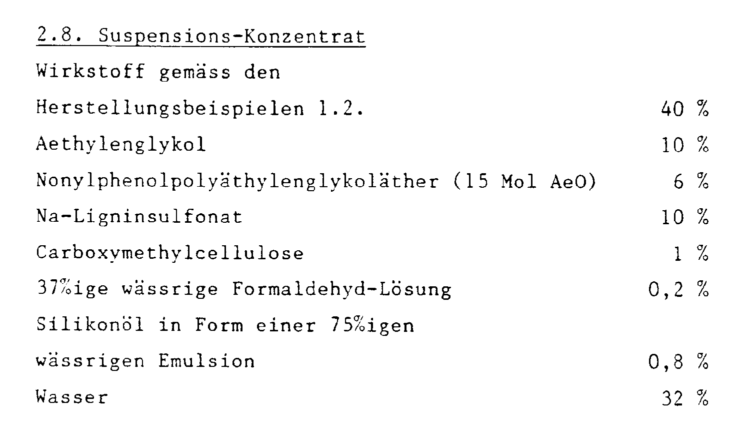

Der fein gemahlene Wirkstoff wird mit den Zusatzstoffen innig vermischt. Man erhält so ein SuspensionsKonzentrat, aus welchem durch Verdünnen mit Wasser Suspensionen jeder gewünschten Konzentration hergestellt werden können.

### Beispiel 3: Biologische Prüfungen

### 3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss den Beispielen 1.2. zeigen gute Wirkung im obigen Test.

### 3.2. Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.2. zeigen in diesem Test gute Wirkung gegen Lucilia sericata.

### 3.3. Wirkung gegen Zecken in verschiedenen Entwicklungsstadien

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die Testtiere werden für kurze Zeit in wässrige Emulsionen der zu untersuchenden Substanzen mit einer Konzentration von 400 ppm getaucht. Die in Teströhrchen befindlichen Emulsionen werden dann mit Watte aufgenommen und die benetzten Testtiere in den so kontaminierten Röhrchen belassen. Die Auswertung (Mortalität in Prozenten) erfolgt für Larven nach 3 Tagen und für Nymphen und Imagines nach 14 Tagen.

Verbindungen gemäss den Beispielen 1.2. zeigen gute Wirkung im obigen Test.

### 3.4. Frassgift-Wirkung auf Spodoptera littoralis-Larven (L₁)

Baumwollpflanzen im Keimblattstadium werden mit einerwässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs-und Häutungsstörungen der angesetzten Larven bestimmt.

Verbindungen gemäss den Beispielen 1.2. zeigen gute Wirkung in diesem Test.

### 3.5. Frassgift-Wirkung auf Spodoptera littoralis- und Heliothis virescens-Larven (L₃)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

### 3.6. Frassgift-Wirkung auf Plutella xylostella-Larven (L₂)

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Chinakohlpflanzen mit 10 Plutella xylostella-Larven im zweiten larvalen Stadium besiedelt. Der Versuch wird bei 26°C und ca. 60% relativer Lufteuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

### 3.7. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit 20 Nymphen der Testtiere im zweiten oder dritten Stadium. Um die Zikaden am Entweichen zu hindern, wird über jede besiedelte Pflanze ein Plexiglaszylinder gestülpt und dieser mit einem Gaze-Deckel abgedeckt. Die Nymphen werden für 6 Tage an der behandelten Pflanze, die mindestens einmal nachgegossen werden muss, gehalten. Der Versuch wird bei einer Temperaturvon ca. 23°C, bei 55 % relativer Luftfeuchtigkeit und mit einer Beleuchtungsperiode von 16 Stunden durchgeführt.

Verbindungen gemäss den Beispielen 1.2. zeigen in diesem Test gute Wirkung.

### 3.8. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N₂- bis N₃- Stadium besiedelt und mit einem Plastikzylinder abgedeckt. Der Versuch wird bei 26°C und ca. 60 % relativer Luftfeuchtigkeit mit einer Belechtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.2. zeigen im obigen Test 80 - 100%ige Wirkung (Mortalität) gegen Nilaparvata lugens.

### 3.9. Wirkung gegen Bodeninsekten (Diabrotica balteata)

Es werden 350 ml Erde (bestehend aus 95 Vol.-% Sand und 5 vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50 % gehalten. Sechs Tage nach dem Ansatz wird die in den Bechern enthaltene Erde abgesiebt und die zurückbleibenden Larven werden auf ihre Mortalität geprüft.

Verbindungen gemäss den Beispielen 1.2. zeigen gute Wirkung im obigen Test.

### 3.10. Kontaktwirkung auf Aphis craccivora

In Töpfen angezogene 4-5 Tage alte Erbsenkeimlinge (Pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach 3 und 5 Tagen. Der Versuch wird bei ca. 21 °C und einer relativen Luftfeuchtigkeit von etwa 55 % durchgeführt.

Verbindungen gemäss den Beispielen 1.2. zeigen gute Wirkung in diesem Test.

### 3.11. Kontaktwirkung auf Myzus persicae

Etwa 4 bis 5 Tage alte, in Wasser angezogene Erbsenkeimlinge (Pisum sativum) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe direkt besprüht. Man verwendet pro Testsubstanz zwei Pflanzen. Eine Auswertung der erzielten Abtötungsrate erfolgt 3 und 5 Tage nach Applikation. Der Versuch wird bei ca. 21°C und etwa 60 % relativer Lufteuchtigkeit durchgeführt.

Verbindungen gemäss den Beispielen 1.2. zeigen gute Wirkung in diesem Test.

### 3.12 Wirkung gegen Tetranychus urticae (OP-sensibel)

Die Primärblättervon Phaseolus vulgaris-Pflanzen werden 24 Stunden vor dem Versuch auf akarizide Wirkung mit einem infestierten Blattstück aus einer Massenzucht von Tetranychus urticae (OP-sens.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht. Während des Versuchsverlaufs stehen die Pflanzen in Gewächshauskabinen bei ca. 25°C mit etwa 50 % rel. Luftfeuchtigkeit.

Nach 6 Tagen werden Imagines und Larven (alle beweglichen Stadien) sowie abgelegte Eier unter dem Binokular auf lebende und tote Individuen ausgewertet.

Verbindungen gemäss den Beispielen 1.2. zeigen in diesem Versuch gute Wirkung.

### 3.13. Wirkung gegen Panonychus ulmi (OP- und Carbamat-resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 100 ppm der zu prüfenden Verbindung bis zur Tropfnässe besprüht. Die behandelten Pflanzen werden dann während 14 Tagen bei ca. 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2. zeigen gute Wirkung im obigen Test.

### 3.14. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss den Beispielen 1.2. zeigen gute Wirkung in diesem Test

### 3.15 Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen gemäss den Beispielen 1.2. zeigen 80 - 100%ige Wirkung (Mortalität).

### 3.16. Wirkung gegen sensible und resistente Adulte von Bemisia tabaci

Baumwollblätter werden in eine Testlösung enthaltend 400 ppm Wirkstoff eingetaucht. Auf die behandelten trockenen Blätterwerden dann in zugedeckten Petri-Schalen 20 - 50 sensible resp. resistente Adulte von Bemisia tabaci gegeben. Nach 24 Stunden wird die Mortalität durch Auszählen bestimmt.

Verbindungen gemäss den Beispielen 1.2. zeigen gute Wirkung im obigen Test.

### 3.17. Ovizide Wirkung gegen Tetranychus cinnabarinus

Zur Eiablage werden Buschbohnen im 2-Blatt-Stadium mit 20 adulten Weibchen pro Blatt eines OP-toleranten Tetranychus cinnabarinus-Stammes besiedelt. Nach 24 Stunden, wenn sich 50 bis 100 höchstens 24 Stunden alte Eier auf der Testpflanze befinden, werden die Weibchen wieder entfernt, und die Pflanzen in einer Spritzkabine mit einer wässrigen Zubereitung enthaltend 200 ppm Wirkstoff (erhalten aus einer 20%igen Spritzpulver) bis zur Tropfnässe besprüht. Eine Auswertung der erzielten Abtötungsrate erfolgt nach 6 Tagen. Der Versuch wird bei ca. 25°C und etwa 50 % relativer Lufteuchtigkeit durchgeführt.

Verbindungen gemäss den Beispielen 1.2. zeigen gute Wirkung in diesem Test.

## Patentansprüche (Patentansprüche für folgende(n) Vertragsstaat(en) : AT, BE, CH, DE, FR, GB, GR, IT, LI, NL)

1. Verbindungen der Formel I 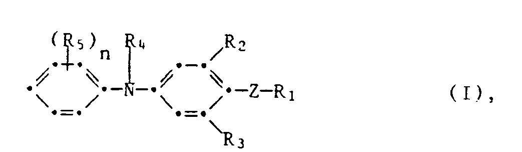 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 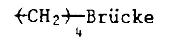 in 2,3- oder 3,4-Stellung;
n für 0, 1, 2, 3 oder 4,
Z für -NH-CS-NH-, -N=C(SRₛ)-NH- oder -N=C=N- und
R₆ für C₁-C₆-Alkyl oder Allyl stehen, sowie ihre Salze mit organischen oder anorganischen Säuren.

2. Verbindungen der Formel I gemäss Anspruch 1, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder C₅-C₆-Cycloalkyl; R₄ für Wasserstoff, Methyl oder-CHO; R₅ je für Halogen, C₁-C₃-Alkyl oder für eine (̵CH=CH)̵₂ oder 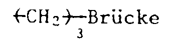 in 3,4-Stellung; n für 0, 1 oder 2; Z für -NH-CS-NH-, -N=C(SR₆)-NH- oder -N=C=N-; und R₆ für C₁-C₄-Alkyl stehen.

3. Verbindungen der Formel gemäss Anspruch 2, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -NH-CS-NH-stehen.

4. Verbindungen der Formel gemäss Anspruch 2, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; Z für -N=C(SR₆)-NH-; und R₆ für C₁-C₄-Alkyl stehen.

5. Verbindungen der Formel gemäss Anspruch 2, worin R₁ für C₁-C₅-Alkyl oder Cyclopentyl; R₂ und R₃ je für C₁-C₄-Alkyl; R₄ für Wasserstoff, Methyl oder-CHO; R₅ für Methyl; n für oder 1; und Z für-N=C=N-stehen.

6. Die Verbindungen gemäss einem der Ansprüche 1 bis 3 der Formeln

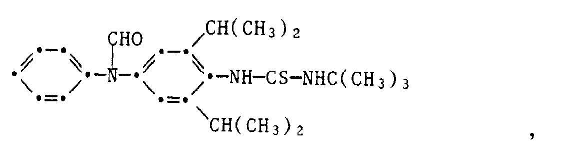

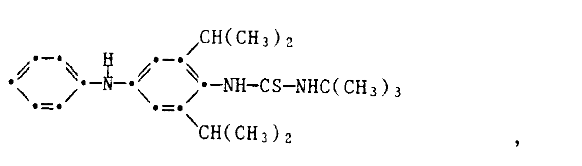

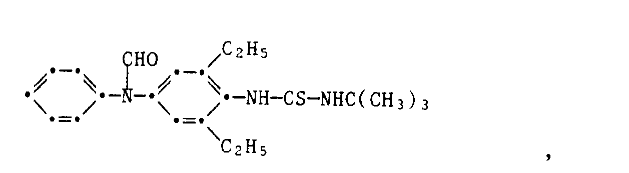

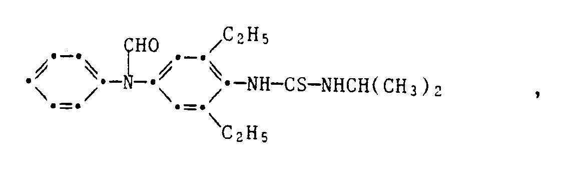

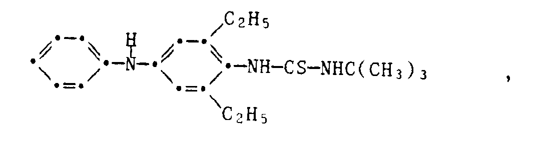

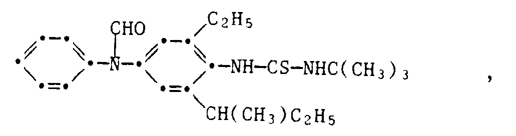

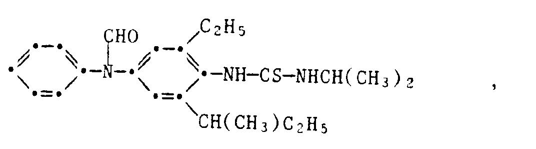

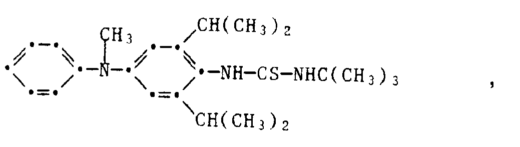

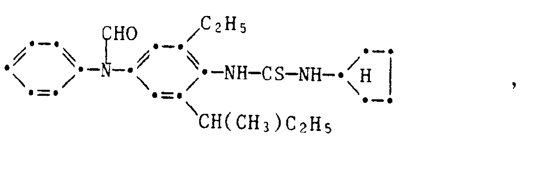

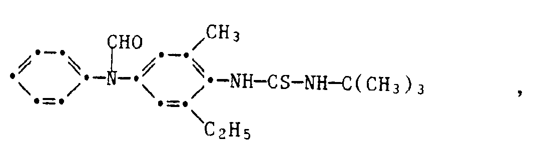

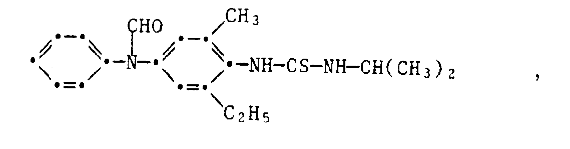

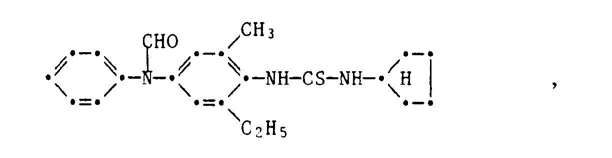

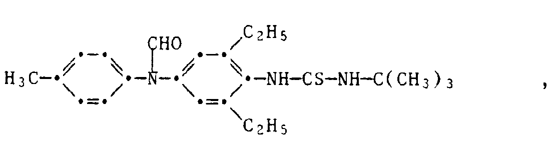

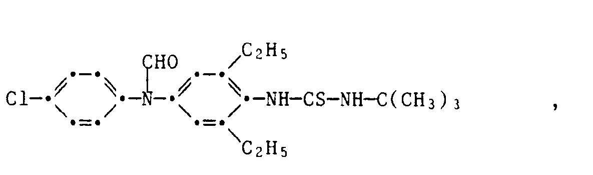

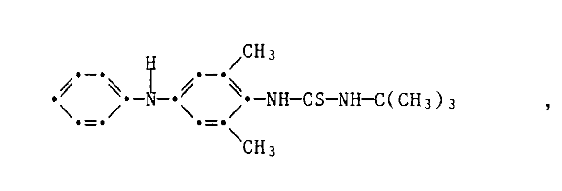

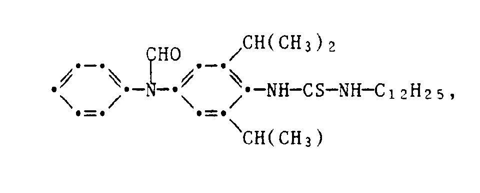

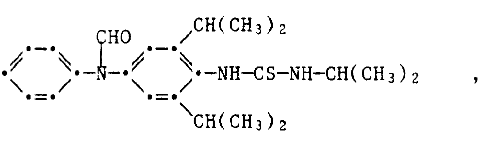

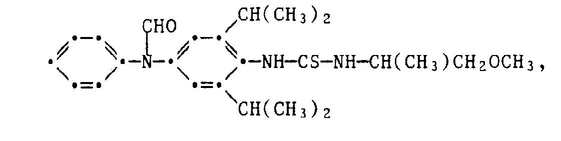

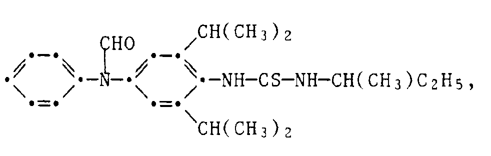

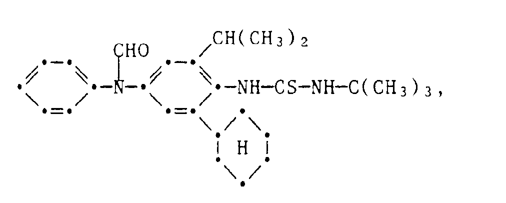

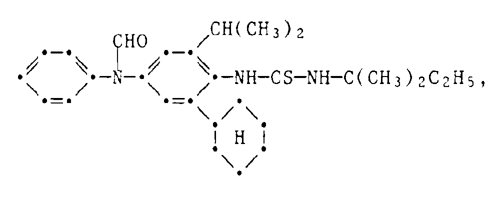

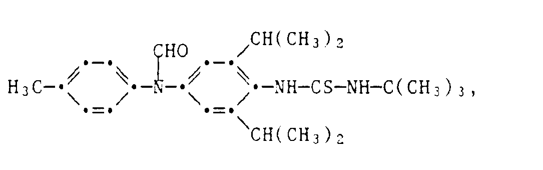

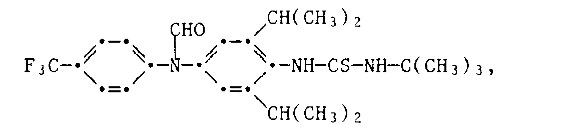

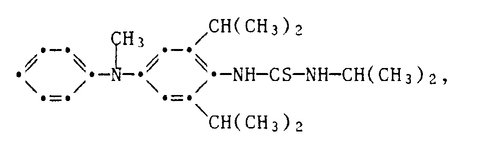

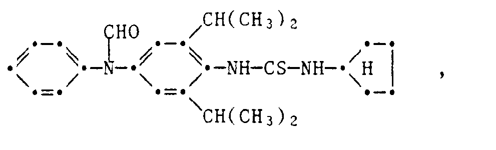

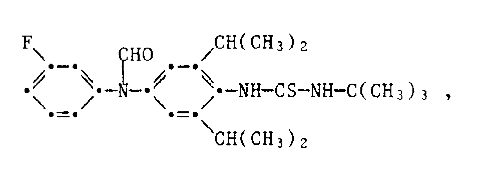

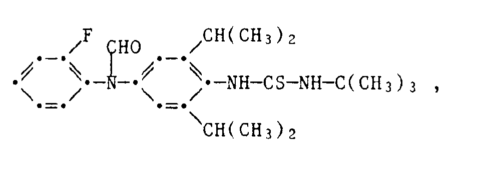

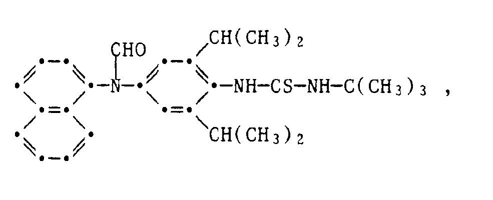

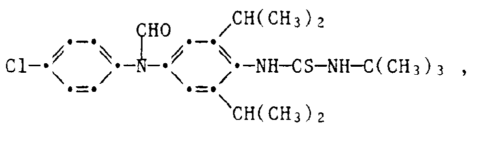

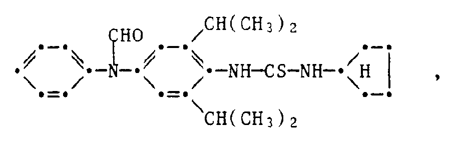

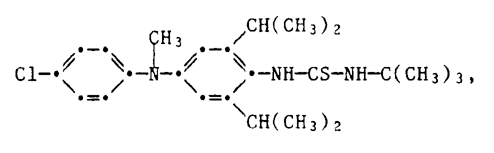

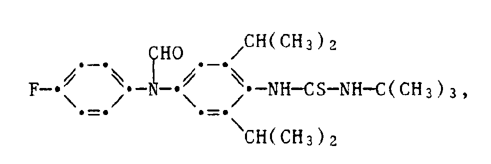

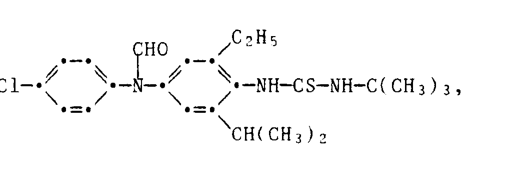

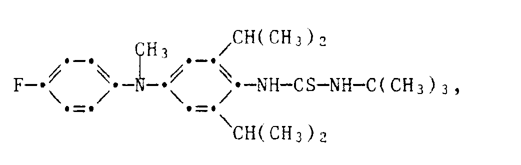

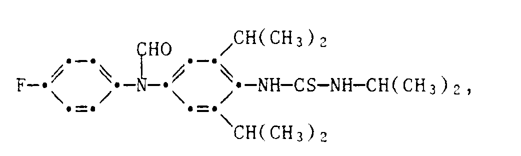

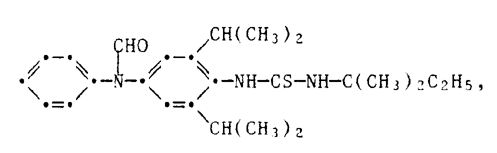

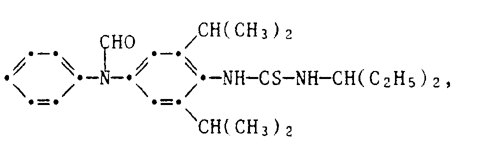

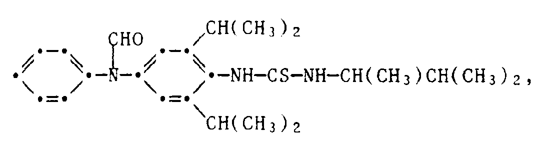

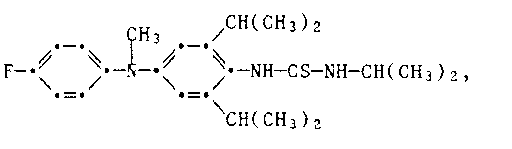

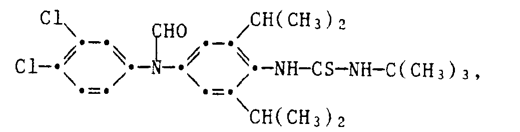

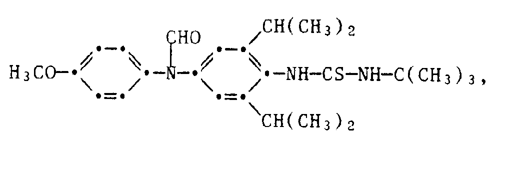

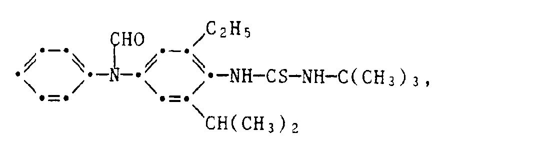

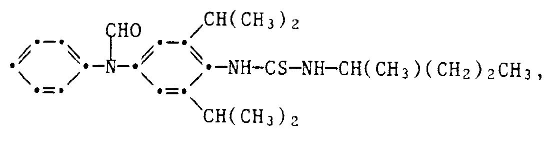

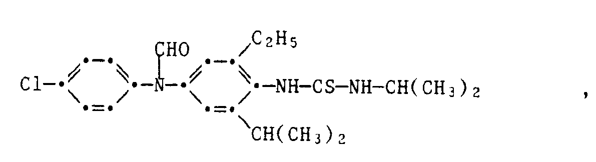

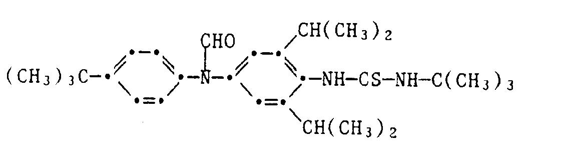

und

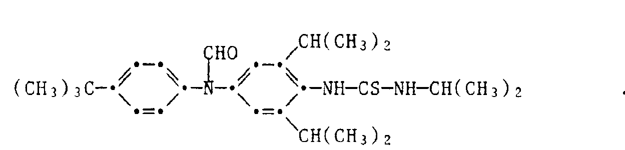

7. Die Verbindungen gemäss einem der Ansprüche 1, 2 oder 4 der Formeln 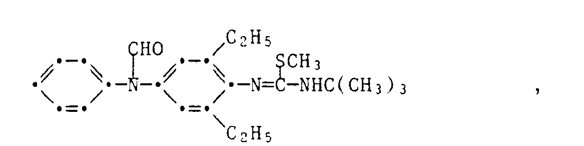 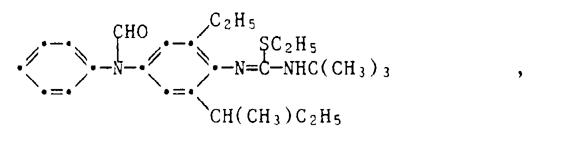 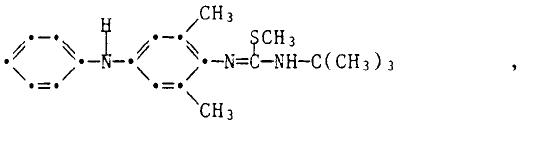 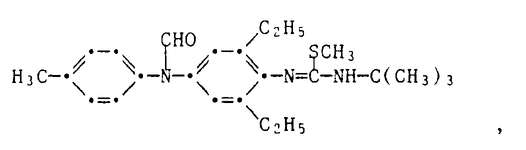 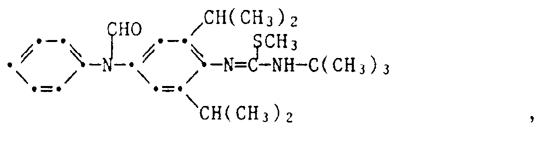 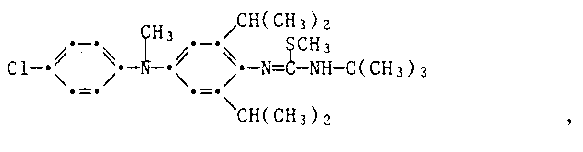 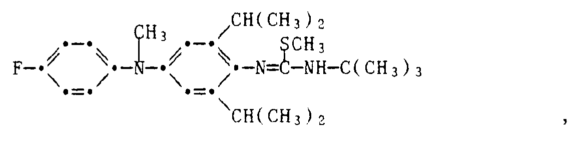 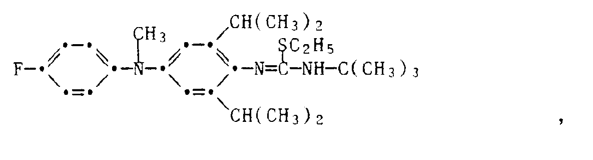 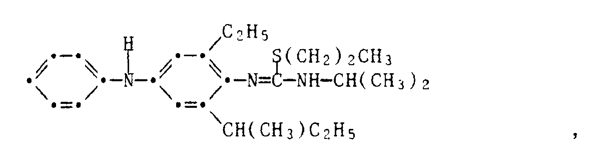 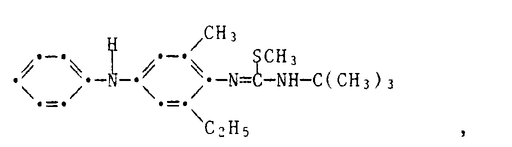 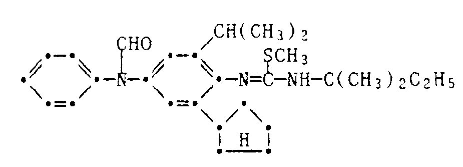 und ,

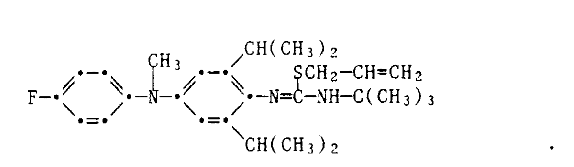

8. Die Verbindungen gemäss einem der Ansprüche 1, 2 oder 5 der Formeln 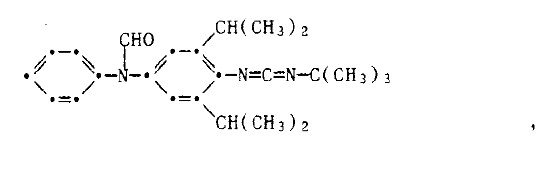 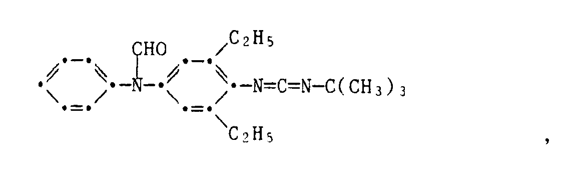 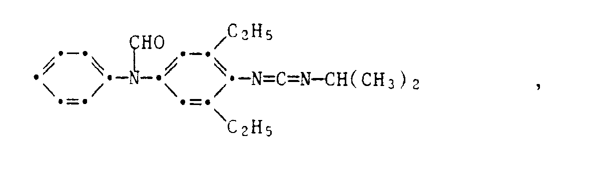 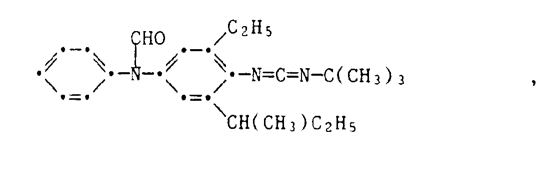 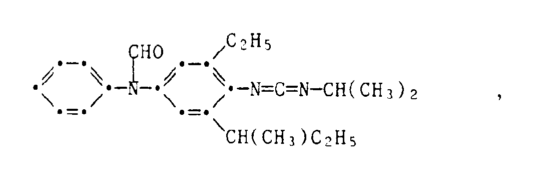 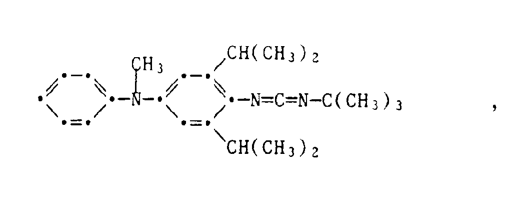 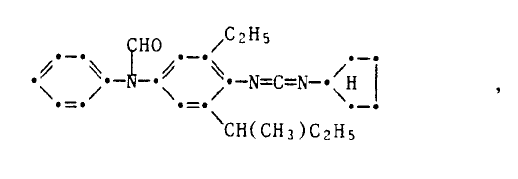 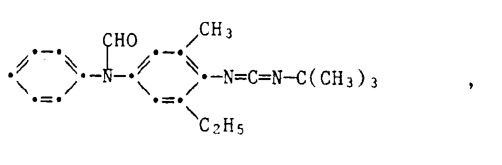 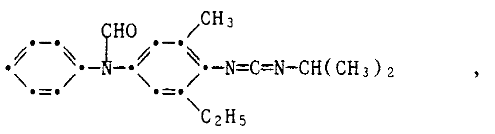 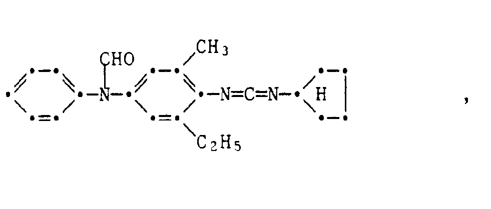 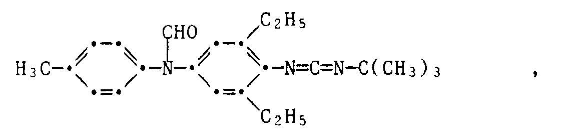 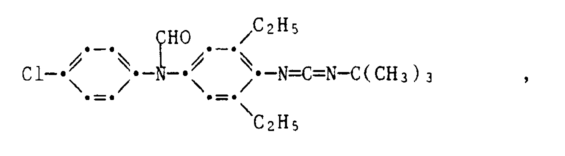 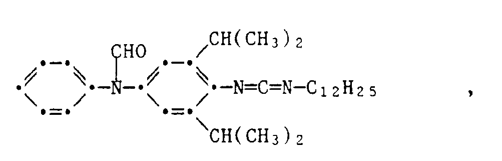 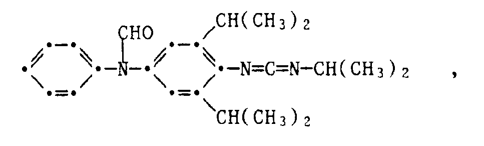 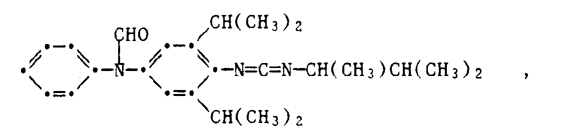 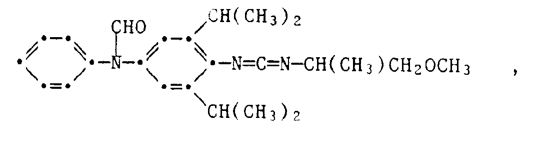 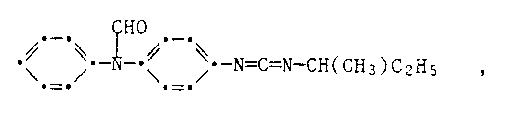 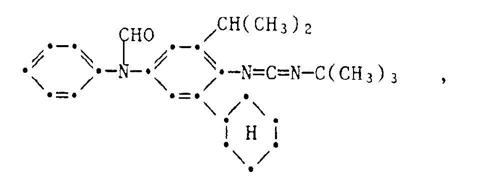 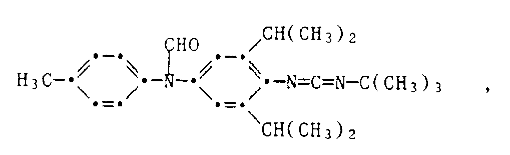 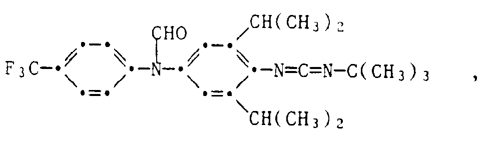 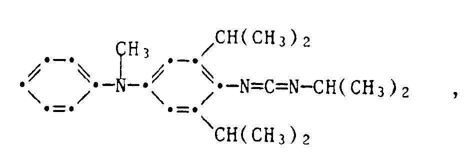 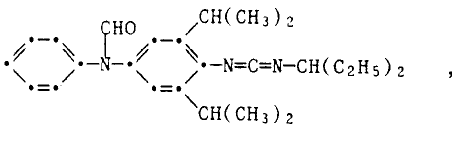 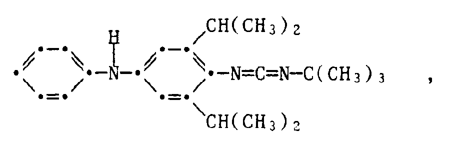 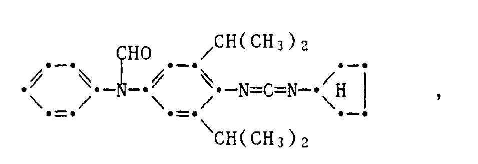 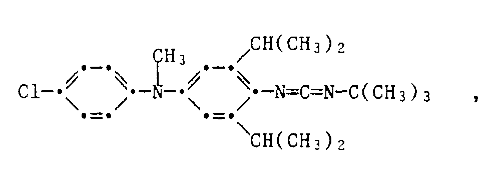 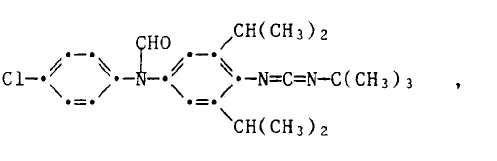 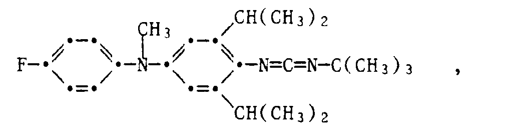 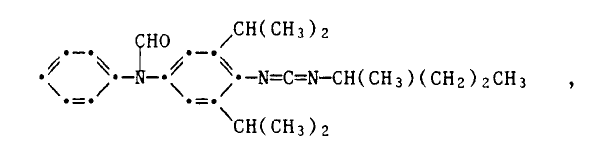 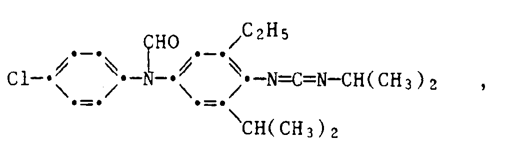 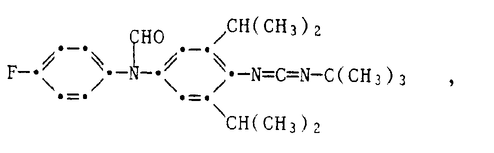 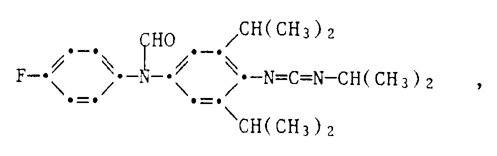 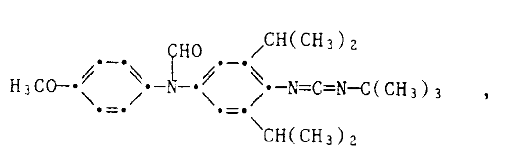 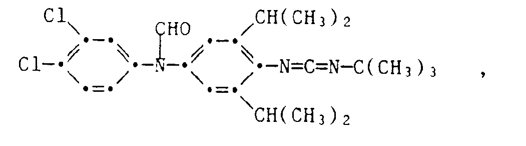 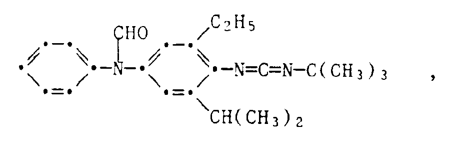 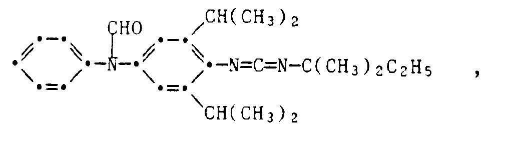 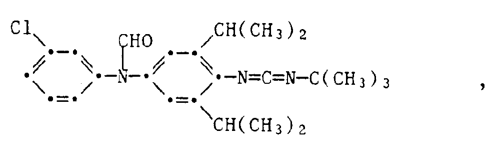 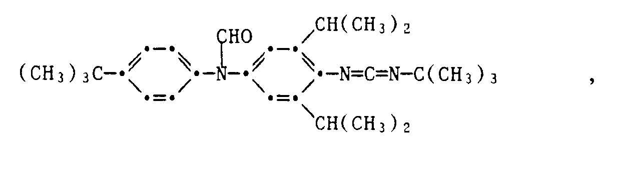 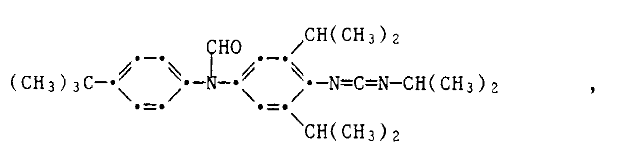 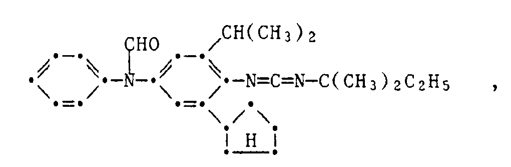 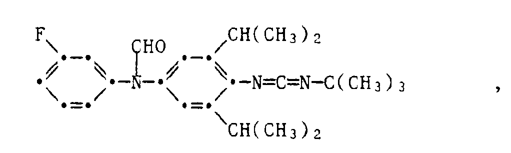 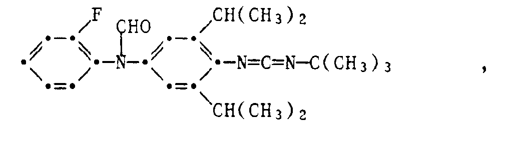 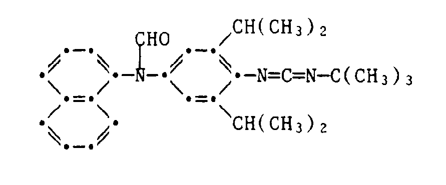 und

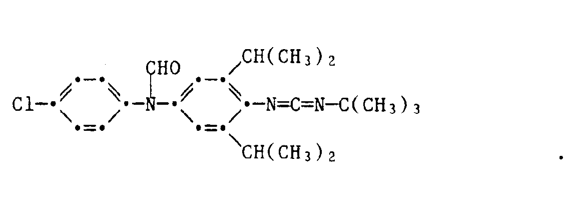

9. Verfahren zur Herstellung einer Verbindung der Formel I 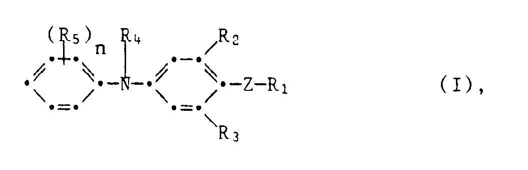 worin

R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes, C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 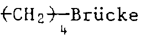 in 2,3- oder 3,4-Stellung;
n für 0, 1, 2, 3 oder 4,
Z für -NH-CS-NH-, -N=C(SRₛ)-NH- oder -N=C=N- und
R₆ für C₁-C₆-Alkyl oder Allyl stehen, dadurch gekennzeichnet, dass man
A) ein Isothiocyanat der Formel II 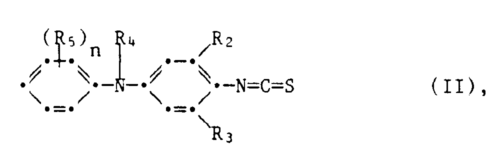 mit einem Amin der Formel III 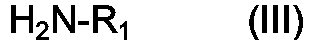 in einem organischen Lösungs- oder Verdünnungsmittel bei Normaldruck und einer Temperatur von 0 bis +150_{°}C zum Thioharnstoff und gegebenenfalls
B) den erhaltenen Thioharnstoff mit einer Verbindung der Formel IV 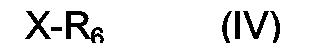 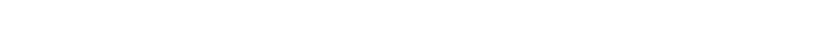 in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, wobei R₁, R₂, R₃, R₄, R₅, R₆ und n die angegebene Bedeutung haben und X für eine Abgangsgruppe steht.

10. Schädlingsbekämpfungsmittel, welches als aktive Komponente mindestens eine Verbindung der Formel 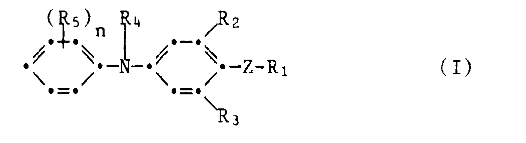 enthält, worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 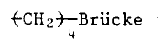 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen oder eines ihrer Salze mit einer organischen oder anorganischen Säure zusammen mit geeigneten Trägern und/oder Zuschlagstoffen.

11. Schädlingsbekämpfungsmittel gemäss Anspruch 10, welches als aktive Komponente mindestens eine Verbindung der Formel enthält, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 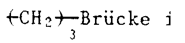 in 3,4-Stellung; n für 0, 1 oder 2; Z für -NH-CS-NH-, -N=C(SR₆)-NH- oder -N=C=N-; und R₆ für C₁-C₄-Alkyl stehen.

12. Schädlingsbekämpfungsmittel gemäss Anspruch 11, worin R₁ für C₁-C₅-Alkyl oder Cyclopentyl; R₂ und R₃ je für C₁-C₄-Alkyl; R₄ für Wasserstoff, Methyl oder-CHO; R₅ für Methyl; n für 0 oder 1; und Z für -NH-CS-NH-stehen.

13. Schädlingsbekämpfungsmittel gemäss Anspruch 11, worin R₁ für C₁-C₅-Alkyl oder Cyclopentyl; R₂ und R₃je für C₁-C₄-Alkyl; R₄ für Wasserstoff, Methyl oder-CHO; R₅ für Methyl; n für 0 oder 1; Z für-N=C(SR₆)-NH-; und R₆ für C₁-C₄-Alkyl stehen.

14. Schädlingsbekämpfungsmittel gemäss Anspruch 11, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -N=C=N-stehen.

15. Verwendung einer Verbindung der Formel I 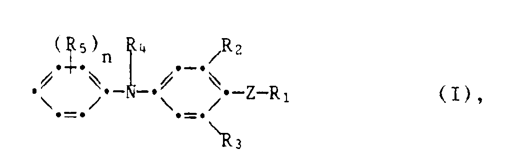 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein-oder mehrfach durch Halogen substituiertes C₁-C₄-Alkoxy oder für eine (̵CH=CH)̵₂, (̵CH₂)̵₃, 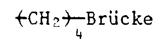 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen, oder eines ihrer Salze mit einer organischen oder anorganischen Säure zur Bekämpfung von Schädlingen an Tieren und Pflanzen.

16. Verwendung gemäss Anspruch 15 zur Bekämpfung von Insekten und Arachniden.

17. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel

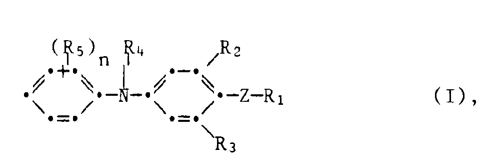

worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 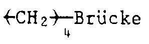 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen, oder einem ihrer Salze mit einer organischen oder anorganischen Säure in Kontakt bringt.

18. Verbindung der Formel V 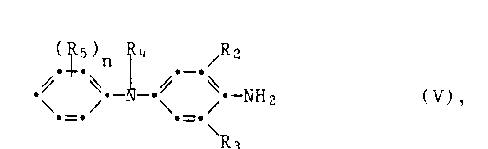 worin
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy oder für eine (̵CH=CH)̵₂, (̵CH₂)̵₃ oder 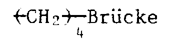 in 2,3- oder 3,4-Stellung und
n für 0, 1, 2, 3 oder 4 stehen.

19. Verbindungen der Formel V gemäss Anspruch 18, worin R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 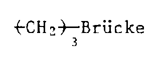 in 3,4-Stellung; und n für 0, 1 oder 2 stehen.

20. Verbindungen der Formel V gemäss Anspruch 19, worin R₂ und R₃ je für C₁-C₄-Alkyl; R₄ für Wasserstoff, Methyl oder -CHO; R₅ für Methyl; und n für 0 oder 1 stehen.

21. Die Verbindungen gemäss Anspruch 18 der Formeln

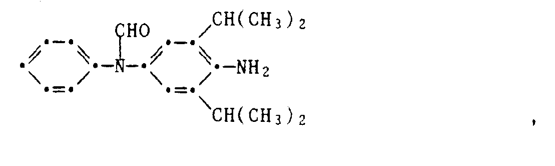

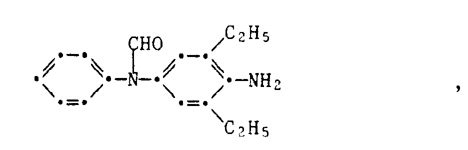

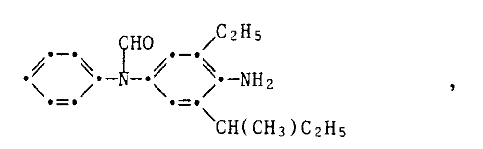

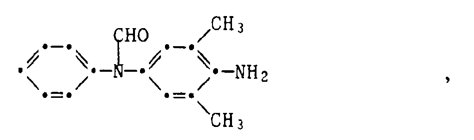

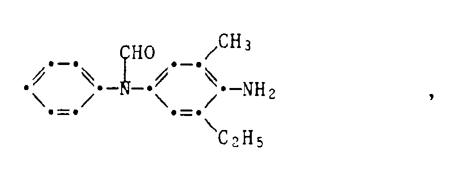

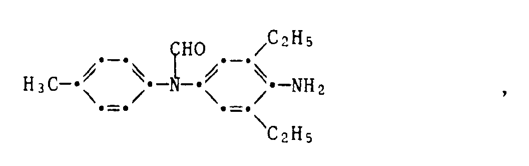

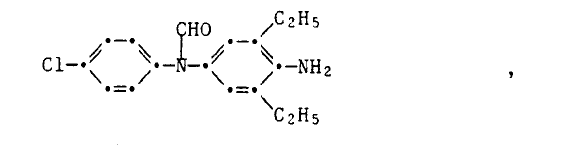

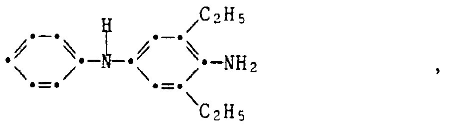

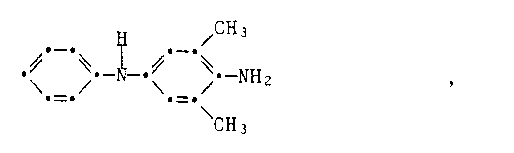

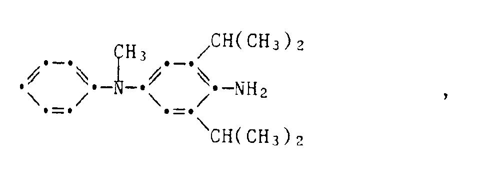

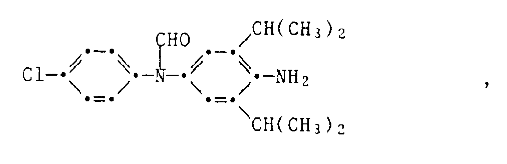

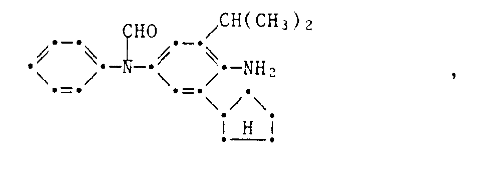

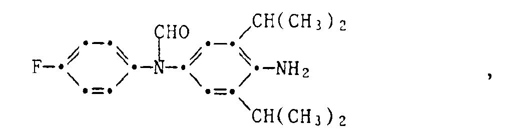

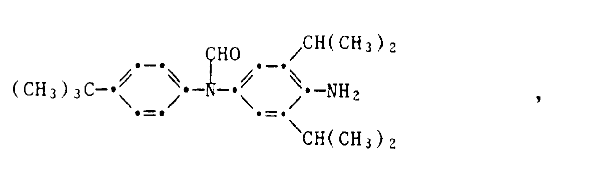

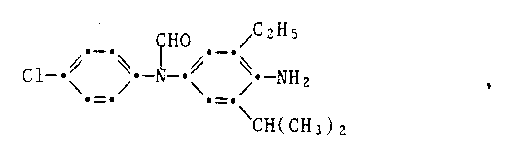

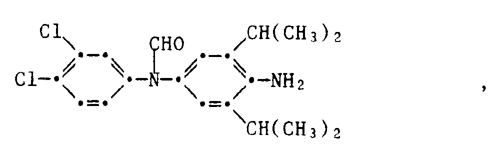

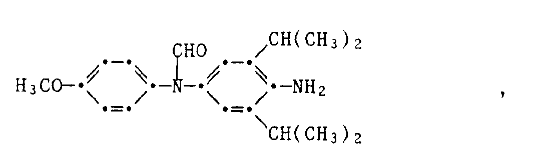

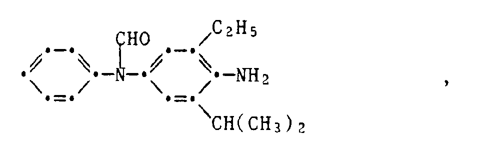

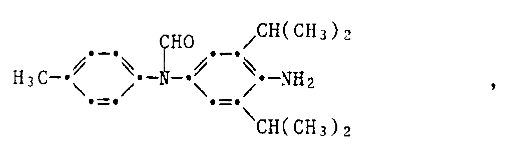

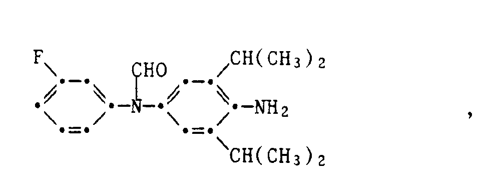

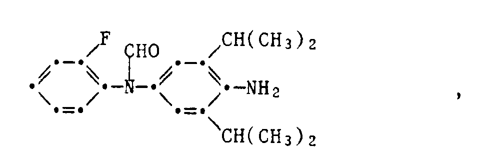

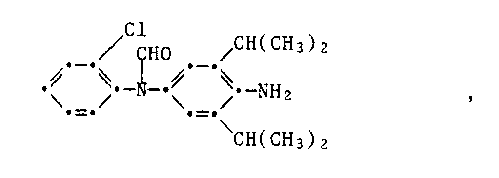

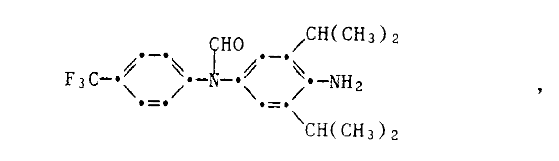

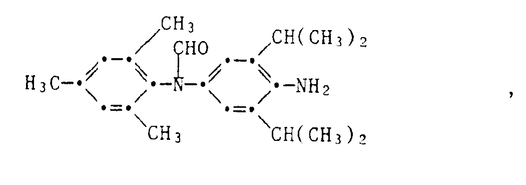

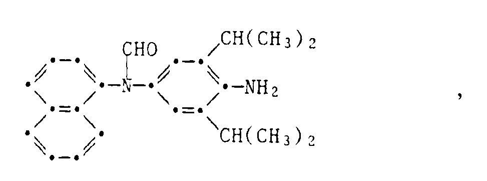

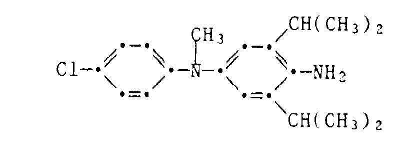

und,

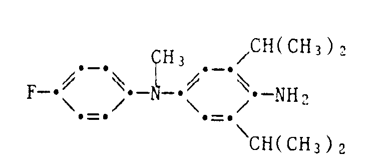

22. Verbindungen der Formel II 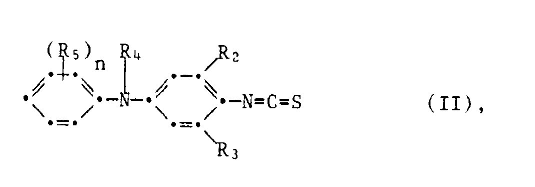 worin
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy oder für eine (̵CH=CH)̵₂, (̵CH₂)̵₃ oder 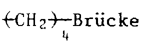 in 2,3 oder 3,4-Stellung und
n für 0, 1, 2, 3 oder 4 stehen.

23. Verbindungen der Formel gemäss Anspruch 22, worin R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 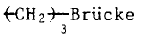 in 3,4-Stellung; und n für 0, 1 oder 2 stehen.

24. Verbindungen der Formel II gemäss Anspruch 23, worin R₂ und R₃ je für C₁-C₄-Alkyl; R₄ für Wasserstoff, Methyl oder -CHO; R₅ für Methyl; und n für 0 oder 1 stehen.

25. Die Verbindungen gemäss Anspruch 22 der Formeln

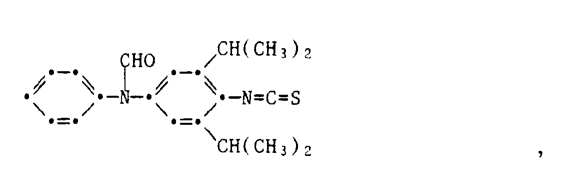

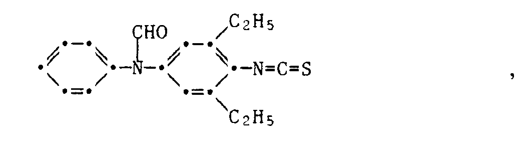

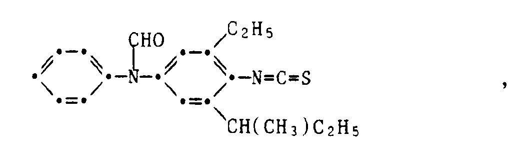

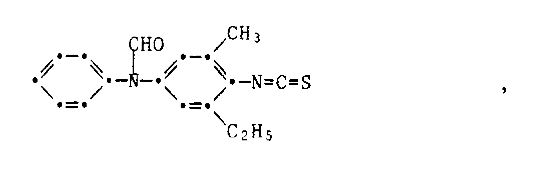

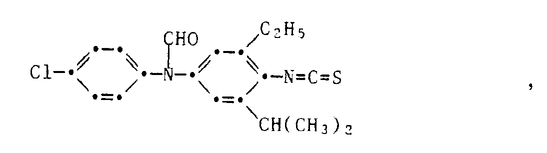

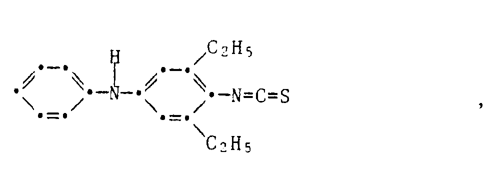

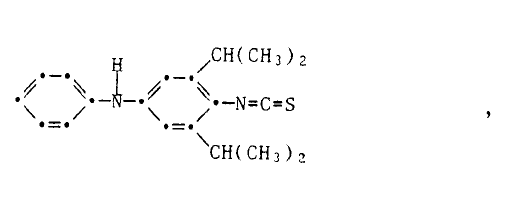

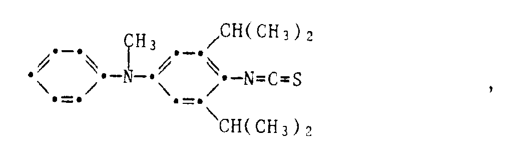

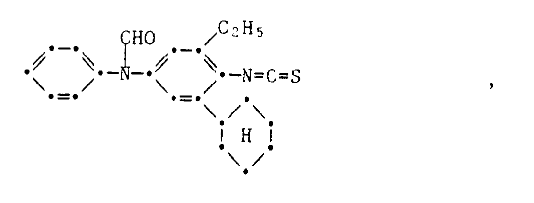

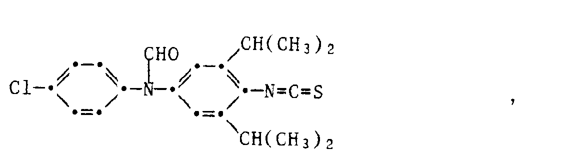

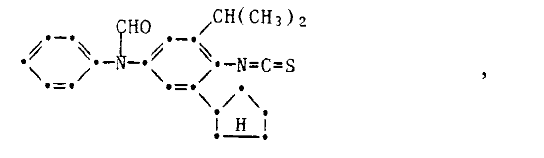

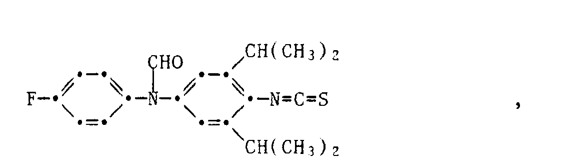

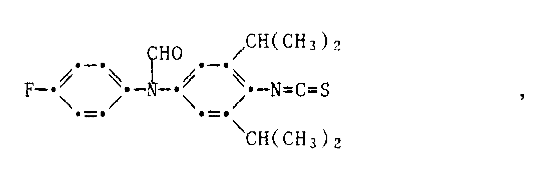

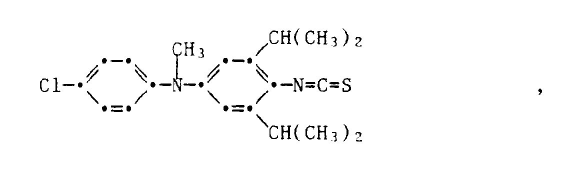

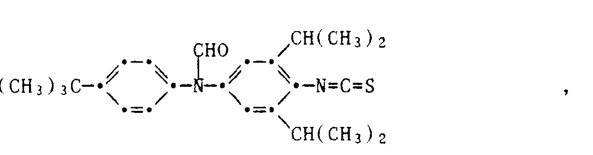

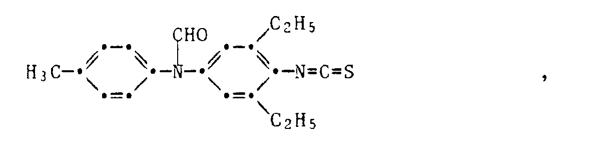

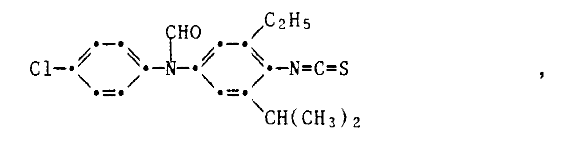

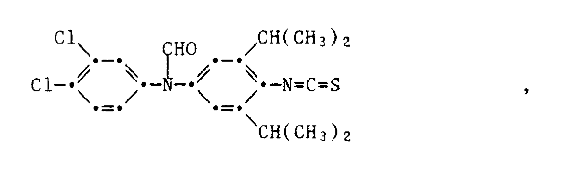

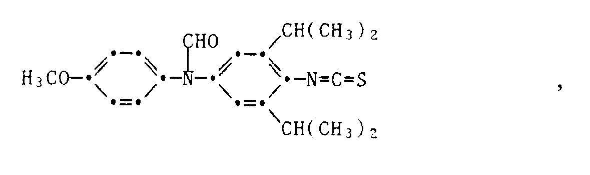

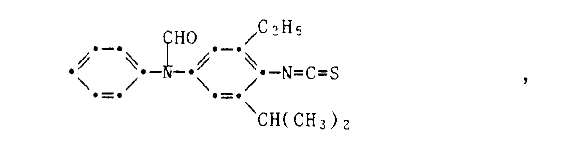

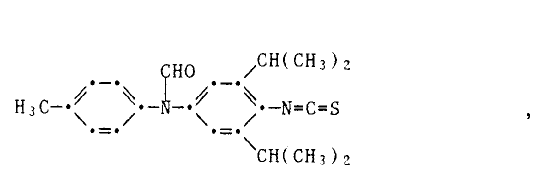

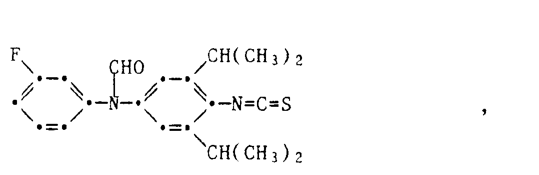

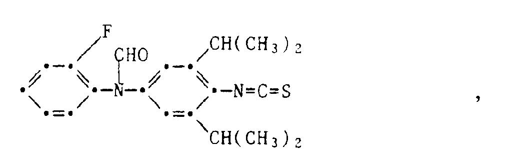

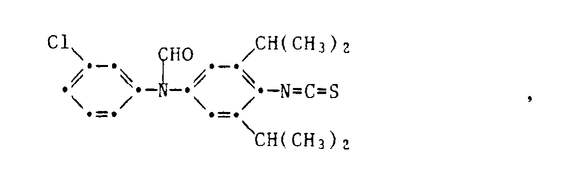

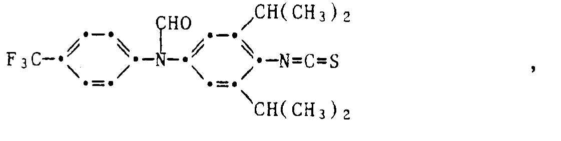

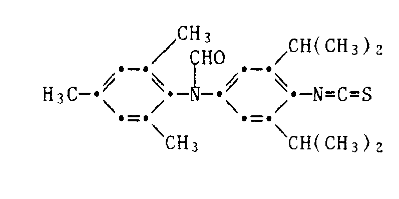

und,

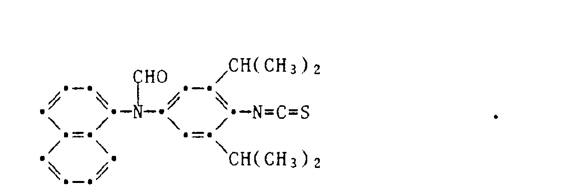

## Patentansprüche (Patentansprüche für folgende(n) Vertragsstaat(en) : ES)

1. Verfahren zur Herstellung einer Verbindung der Formel I 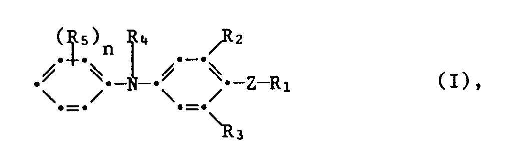 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 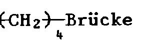 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen, dadurch gekennzeichnet, dass man
A) ein Isothiocyanat der Formel II 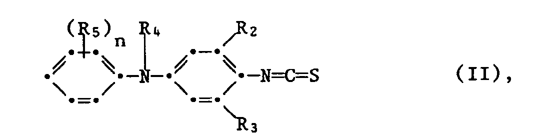 mit einem Amin der Formel III 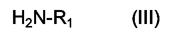 in einem organischen Lösungs- oder Verdünnungsmittel bei Normaldruck und einer Temperatur von 0 bis +150_{°}C zum Thioharnstoff und gegebenenfalls
B) den erhaltenen Thioharnstoff mit einer Verbindung der Formel IV X-R₆ (IV) in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, wobei R₁, R₂, R₃, R₄, R₅, R₆ und n die angegebene Bedeutung haben und X für eine Abgangsgruppe steht.

2. Verfahren gemäss Anspruch 1, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 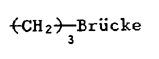 in 3,4-Stellung; n für 0, 1 oder 2; Z für -NH-CS-NH-, -N=C(SR₆)-NH- oder -N=C=N-; und R₆ für C₁-C₄-Alkyl stehen.

3. Verfahren gemäss Anspruch 2, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -NH-CS-NH-stehen.

4. Verfahren gemäss Anspruch 2, worin R₁ für C₁-C₅-Alkyl oder Cyclopentyl; R₂ und R₃ je für C₁-C₄-Alkyl; R₄ für Wasserstoff, Methyl oder-CHO; R₅ für Methyl; n für 0 oder 1; Z für-N=C(SR₆)-NH-; und R₆ für C₁-C₄-Alkyl stehen.

5. Verfahren gemäss Anspruch 2, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -N=C=N-stehen.

6. Verfahren gemäss einem der Ansprüche 1 bis 3 zur Herstellung der Verbindungen der Formeln

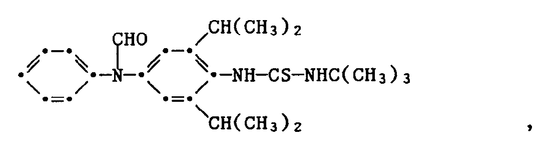

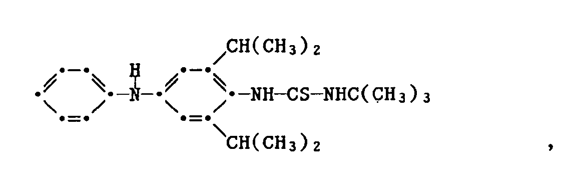

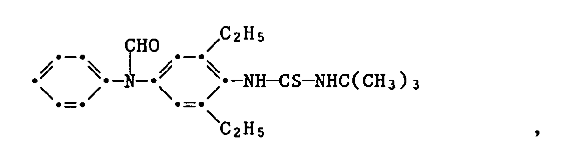

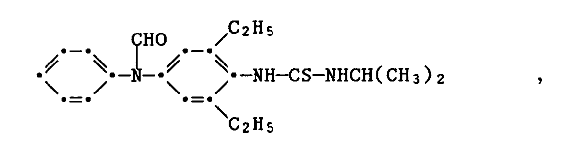

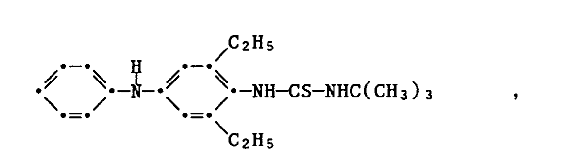

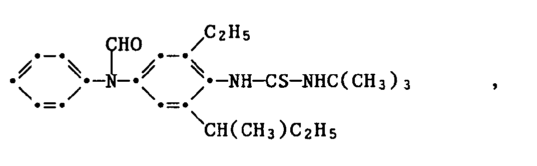

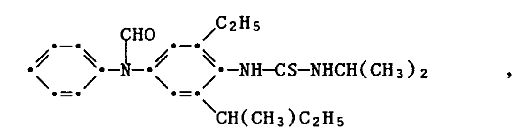

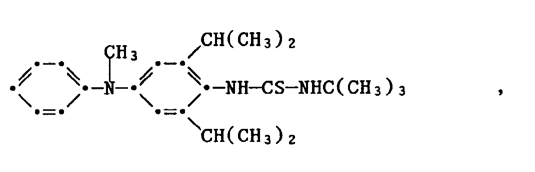

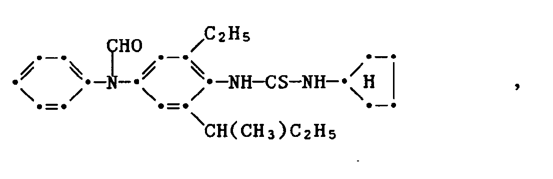

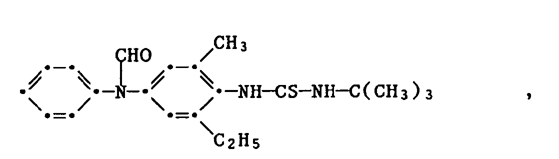

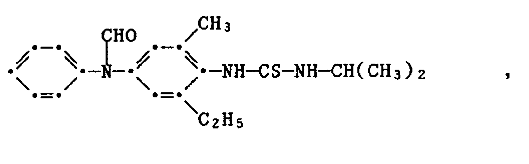

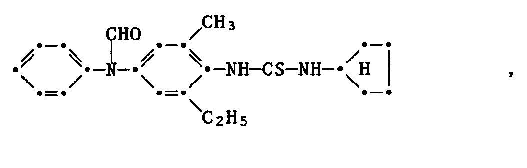

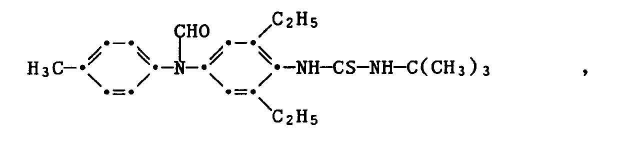

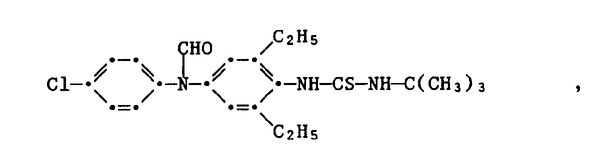

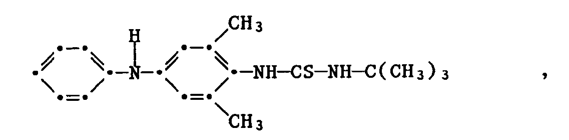

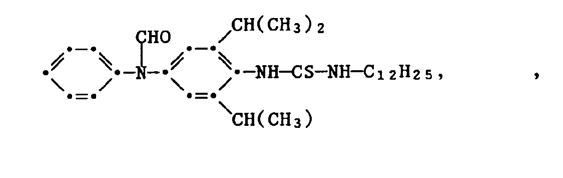

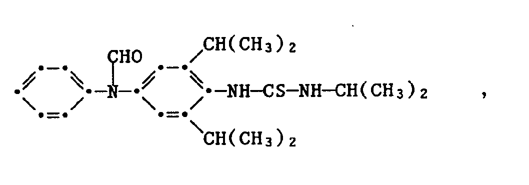

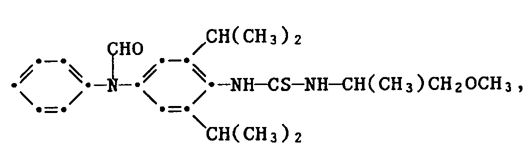

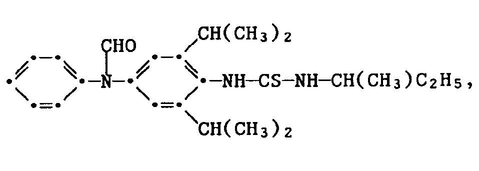

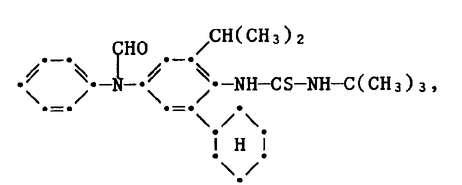

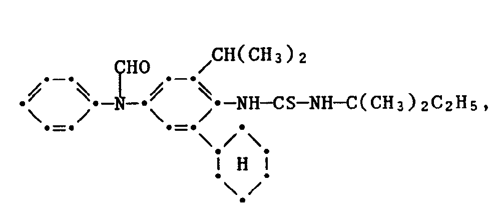

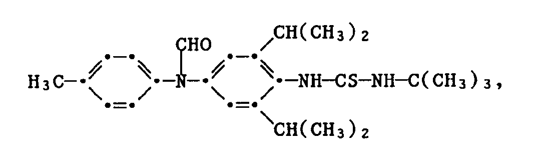

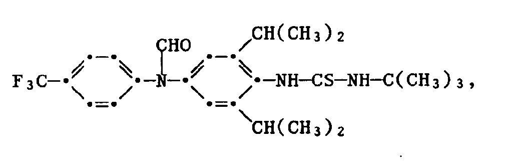

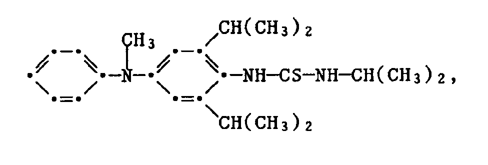

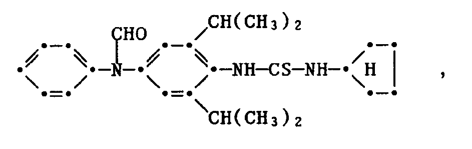

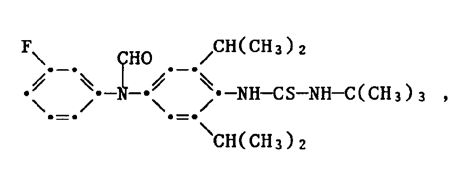

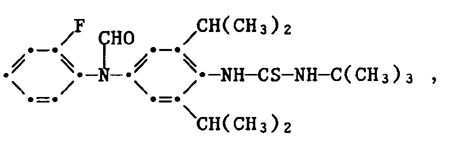

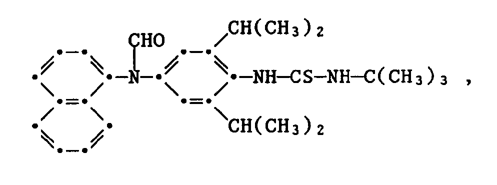

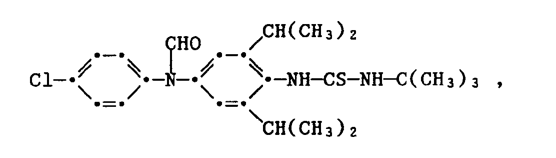

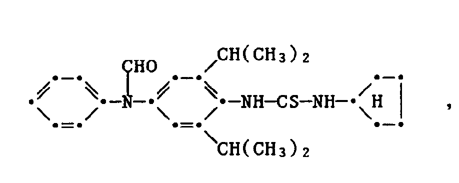

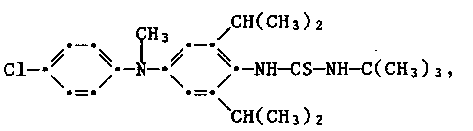

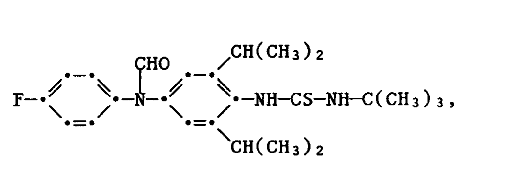

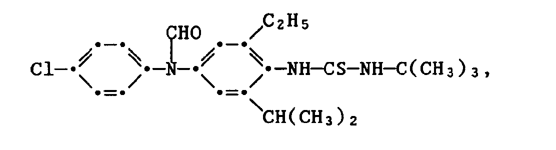

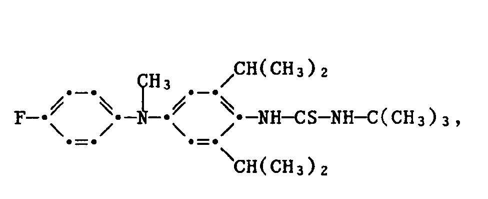

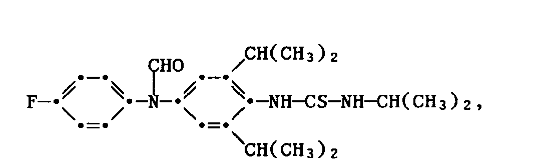

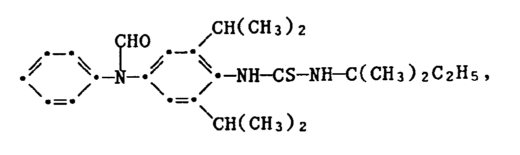

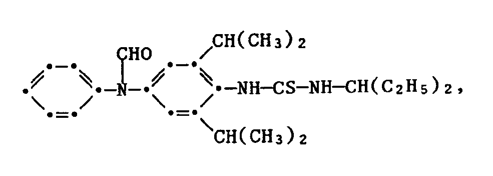

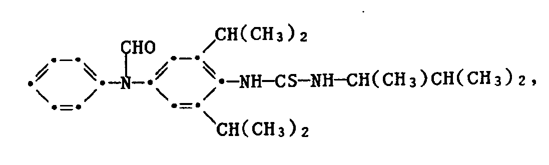

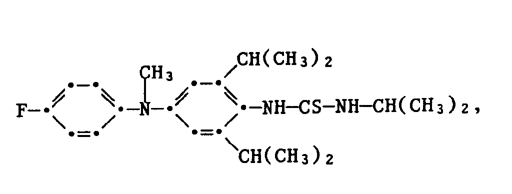

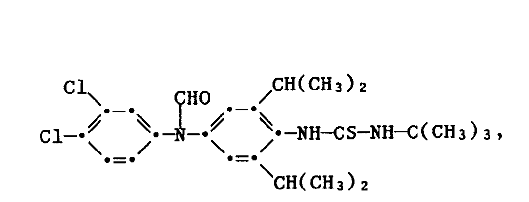

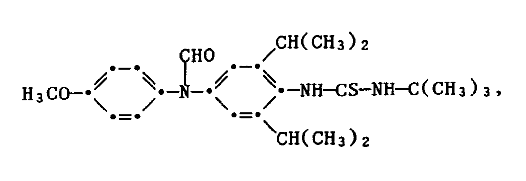

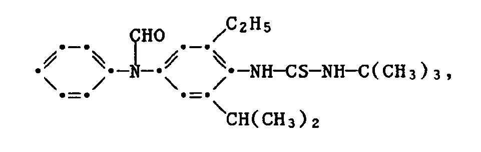

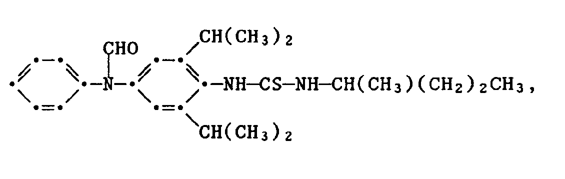

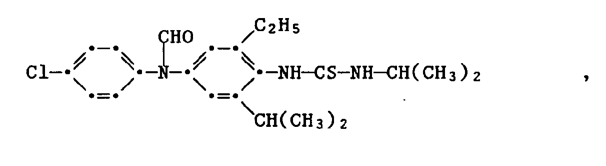

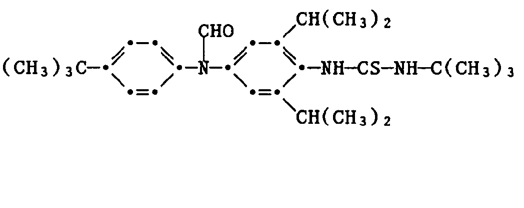

und

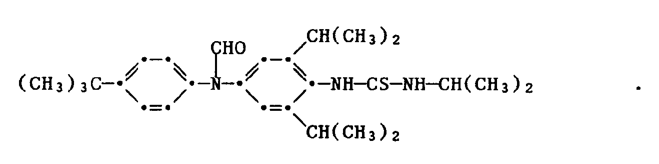

7. Verfahren gemäss einem der Ansprüche 1, 2 oder 4 zur Herstellung der Verbindungen der Formeln 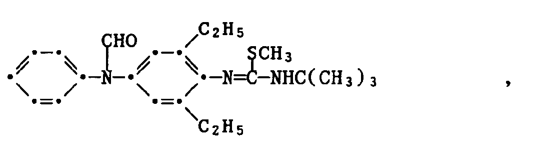 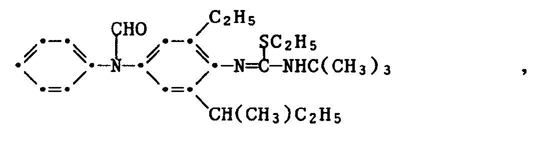 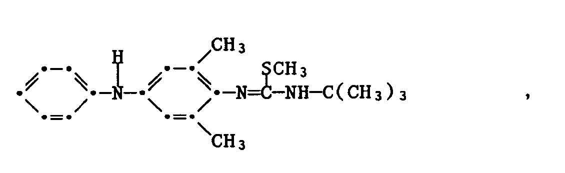 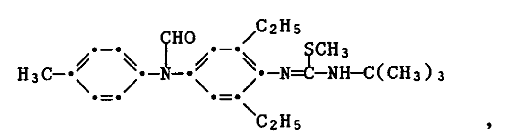 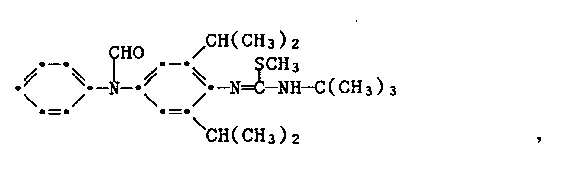 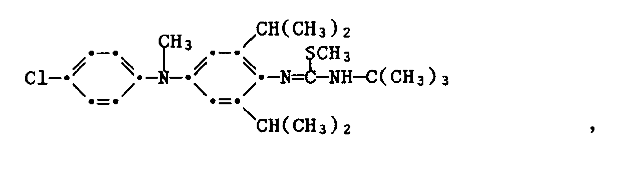 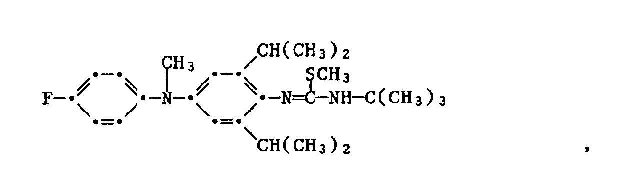 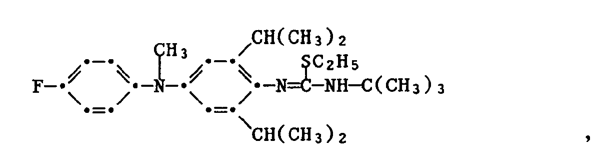 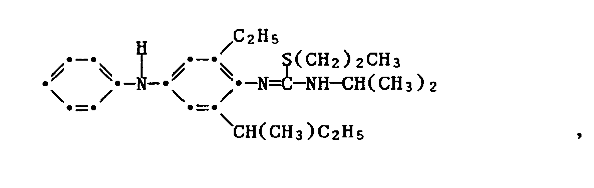 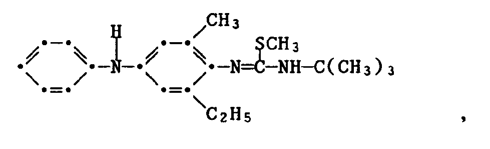 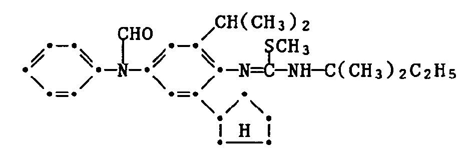 und

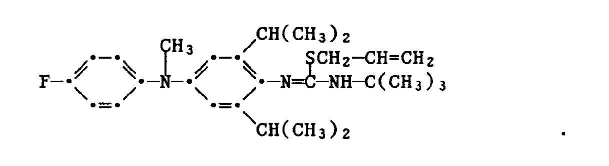

8. Verfahren gemäss einem der Ansprüche 1, 2 oder 5 zur Herstellung der Verbindungen der Formeln 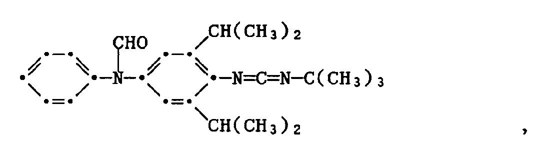 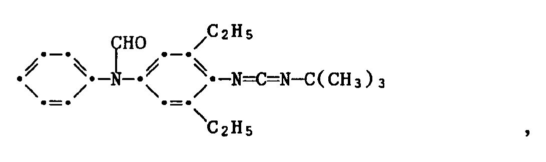 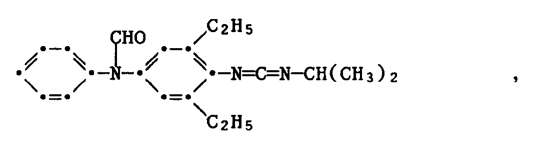 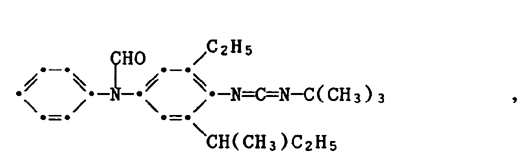 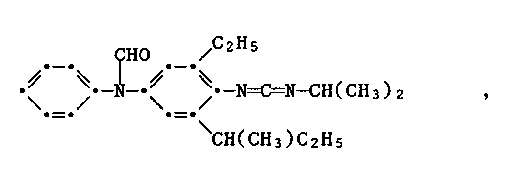 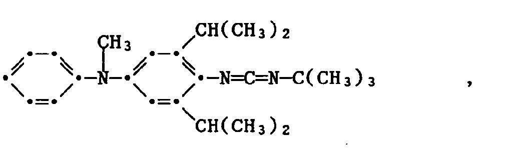 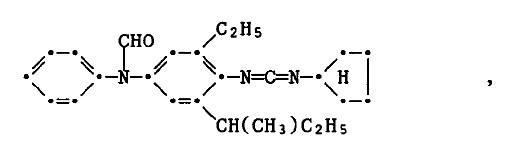 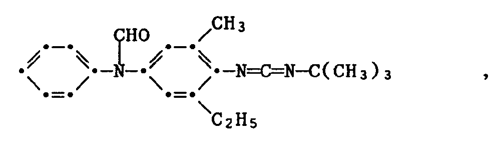 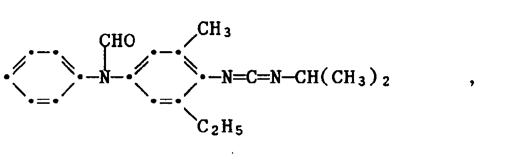 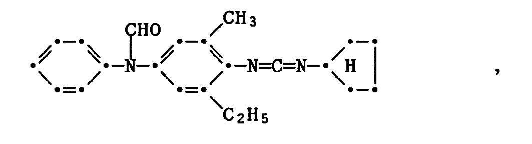 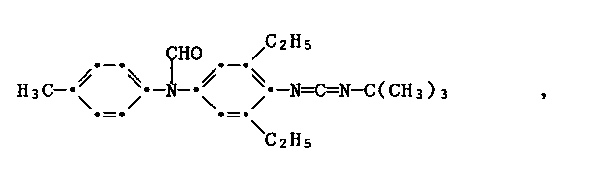 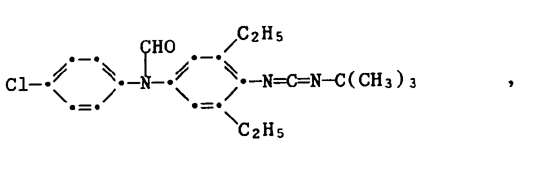 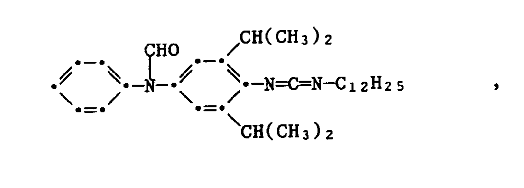 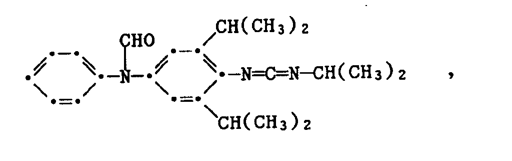 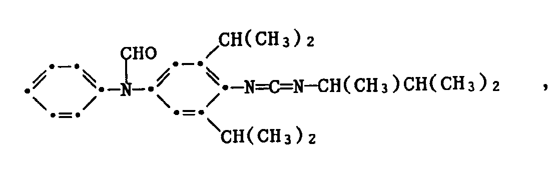 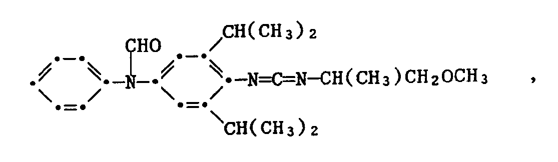 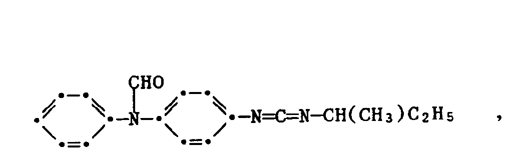 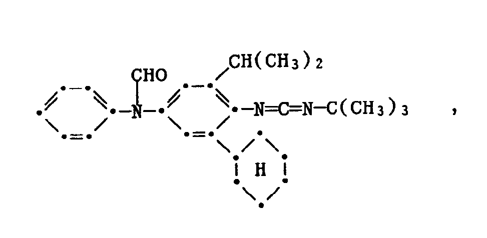 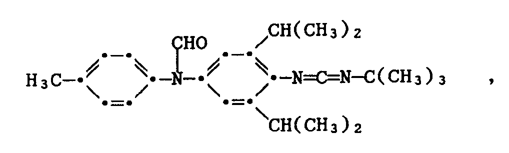 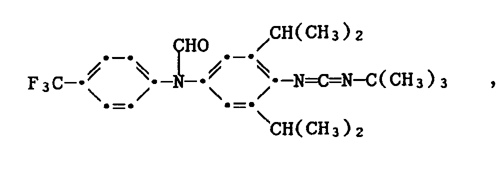 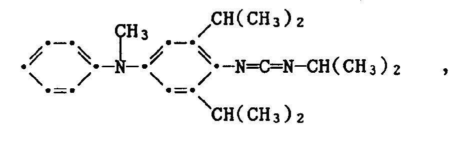 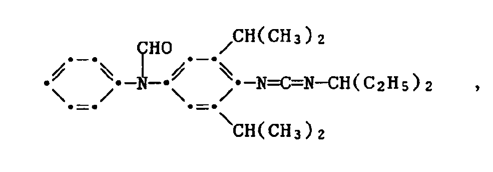 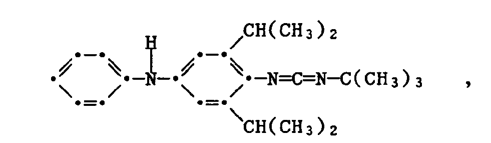 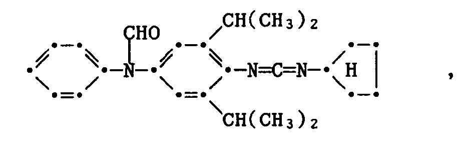 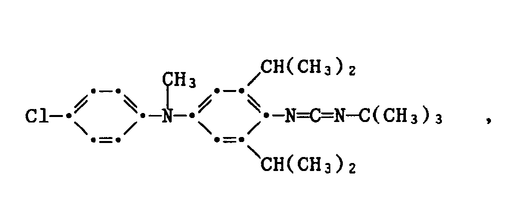 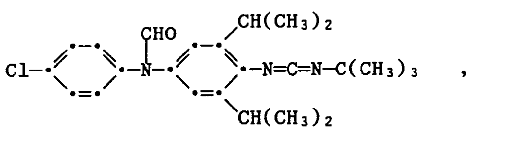 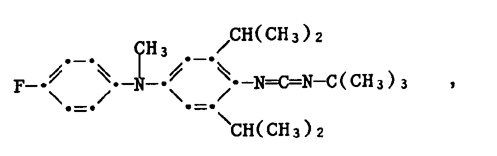 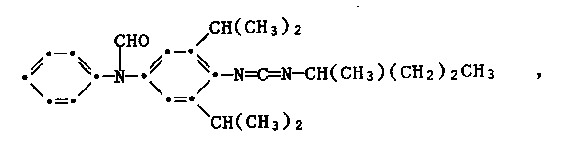 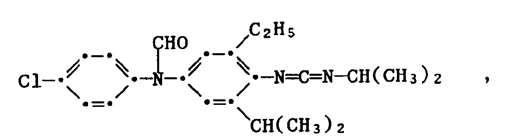 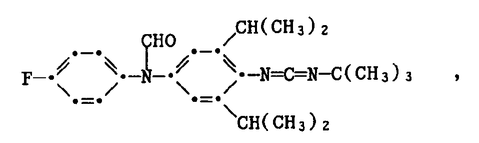 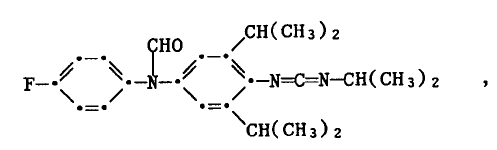 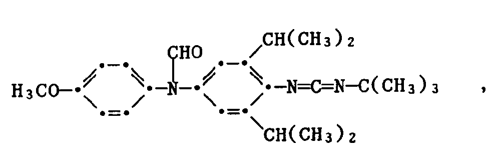 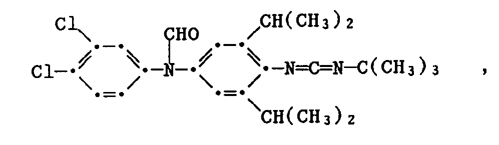 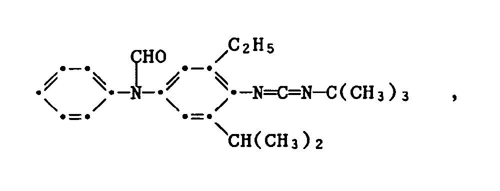 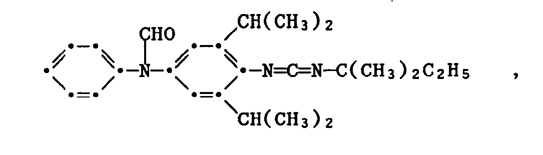 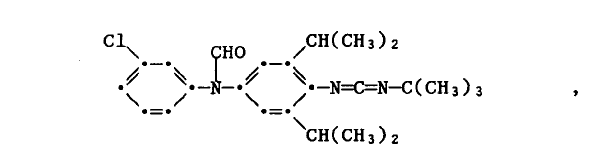 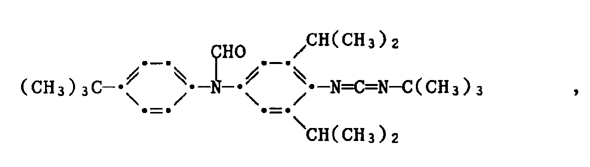 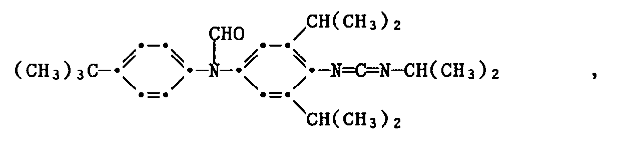 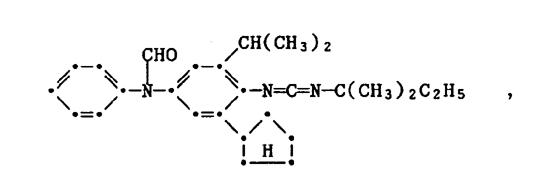 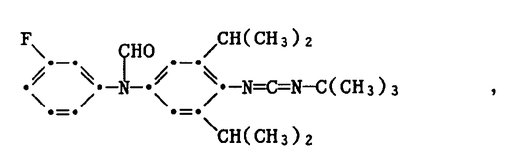 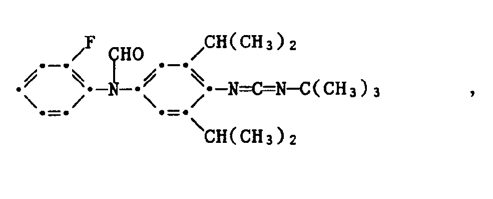 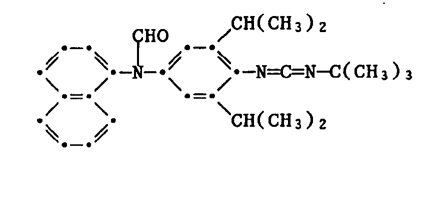 und

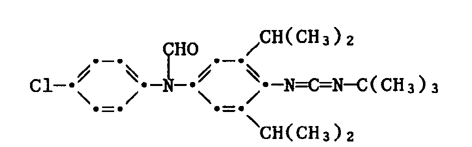

9. Schädlingsbekämpfungsmittel, welches als aktive Komponente mindestens eine Verbindung der Formel I 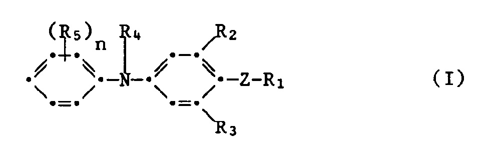 enthält, worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃ 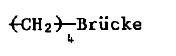 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen, oder eines ihrer Salze mit einer organischen oder anorganischen Säure zusammen mit geeigneten Trägern und/oder Zuschlagstoffen.

10. Schädlingsbekämpfungsmittel gemäss Anspruch 9, welches als aktive Komponente mindestens eine Verbindung der Formel enthält, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 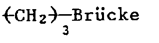 in 3,4-Stellung; n für 0, 1 oder 2; Z für -NH-CS-NH-, -N=C(SR₆)-NH- oder -N=C=N-; und R₆ für C₁-C₄-Alkyl stehen.

11. Schädlingsbekämpfungsmittel gemäss Anspruch 10, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -NH-CS-NH-stehen.

12. Schädlingsbekämpfungsmittel gemäss Anspruch 10, worin R₁ für C₁-C₅-Alkyl oder Cyclopentyl; R₂ und R₃je für C₁-C₄-Alkyl; R₄ für Wasserstoff, Methyl oder-CHO; R₅ für Methyl; n für 0 oder 1; Z für-N=C(SR₆)-NH-; und R₆ für C₁-C₄-Alkyl stehen.

13. Schädlingsbekämpfungsmittel gemäss Anspruch 10, worin R₁ für C₁-C₅-Alkyl oder Cyclopentyl; R₂ und R₃ je für C₁-C₄-Alkyl; R₄ für Wasserstoff, Methyl oder -CHO; R₅ für Methyl; n für 0 oder 1; und Z für -N=C=N-stehen.

14. Verwendung einer Verbindung der Formel I 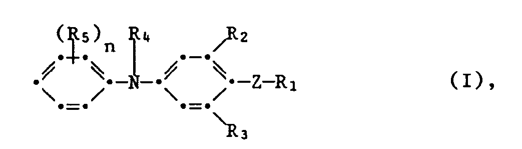 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃, 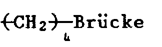 in 2,3- oder 3,4-Stellung
n für 0, 1, 2, 3 oder 4,
Z für -NH-CS-NH-, -N=C(SR₆)-NH- oder -N=C=N- und
R₆ für C₁-C₆-Alkyl oder Allyl stehen, oder eines ihrer Salze mit einer organischen oder anorganischen Säure zur Bekämpfung von Schädlingen an Tieren und Pflanzen.

15. Verwendung gemäss Anspruch 14 zur Bekämpfung von Insekten und Arachniden.

16. Verfahren zum Bedämpfen von Schädlingen an Tieren und Pflanzen, dadurch gekennzeichnet, dass man die Schädlinge in ihren verschiedenen Entwicklungsstadien mit einer Verbindung der Formel I 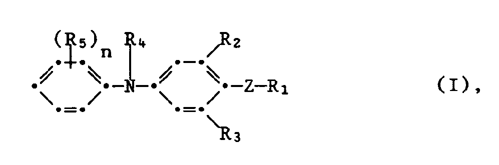 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ und R₃ je für C₁-C₅-Alkyl oder C₅-C₆-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy, ein- oder mehrfach durch Halogen substituiertes C₁-C₄-Alkoxy oder für eine (̵CH=CH)̵₂, (̵CH₂)̵₃ oder 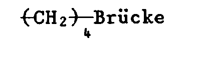 in 2,3- oder 3,4-Stellung;
n für 0, 1, 2, 3 oder 4,
Z für -NH-CS-NH-, -N=C(SR₆)-NH- oder -N=C=N- und
R₆ für C₁-C₆-Alkyl oder Allyl stehen, oder einem ihrer Salze mit einer organischen oder anorganischen Säure in Kontakt bringt.

17. Verfahren zur Herstellung einer Verbindung der Formel V 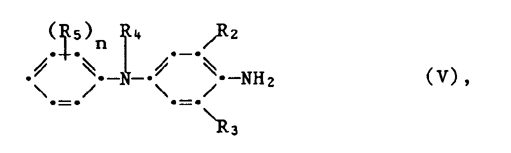 worin
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy oder für eine (̵CH=CH)̵₂, (̵CH₂)̵₃ 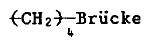 in 2,3- oder 3,4-Stellung und
n für 0, 1, 2, 3 oder 4 stehen, dadurch gekennzeichnet, dass man ein Anilin der Formel VI 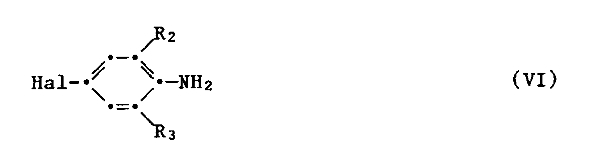 mit einem Formanilid der Formel VII 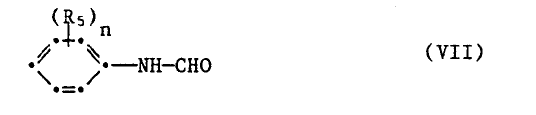 umsetzt, wobei R₂, R₃, R₅ und n die für Formel I angegebene Bedeutung haben und Hal für Halogen, insbesondere für Chlor oder Brom steht.

18. Verfahren gemäss Anspruch 17, worin R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂ oder 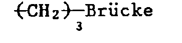 in 3,4-Stellung; und n für 0, 1 oder 2 stehen.

19. Verfahren gemäss Anspruch 18, worin R₂ und R₃ je für C₁-C₄-Alkyl; R₄ für Wasserstoff, Methyl oder -CHO; R₅ für Methyl; und n für 0 oder 1 stehen.

20. Verfahren gemäss Anspruch 17 zur Herstellung der Verbindungen der Formeln 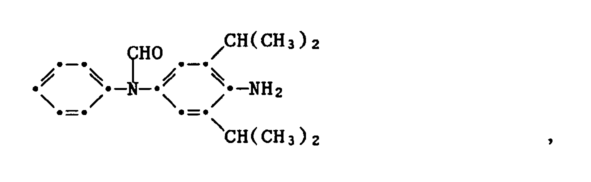 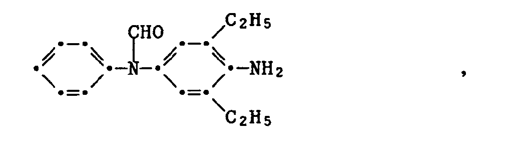 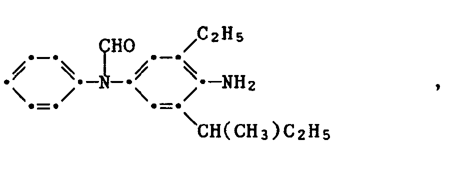 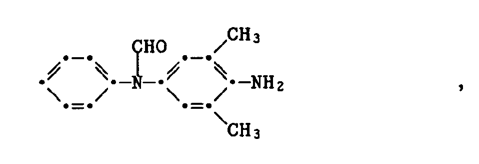 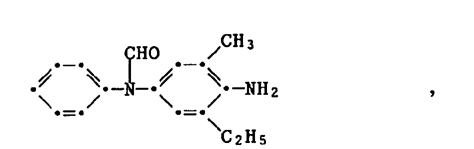 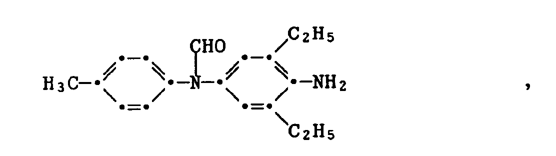 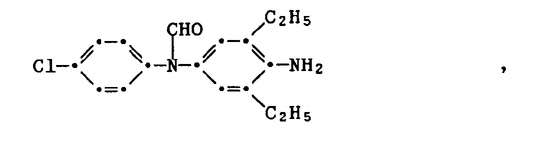 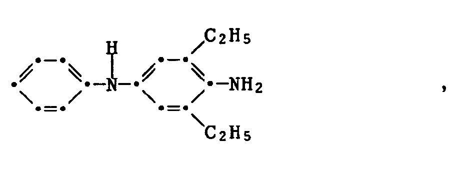 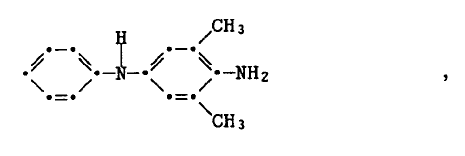 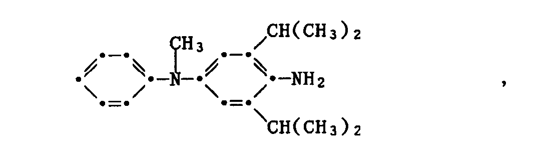 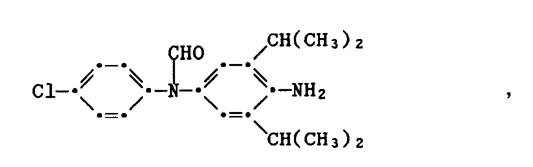 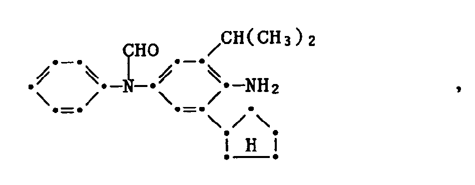 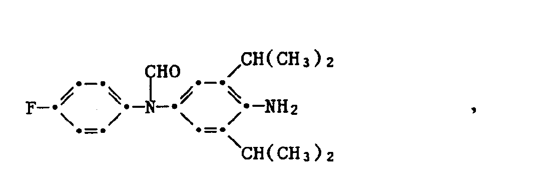 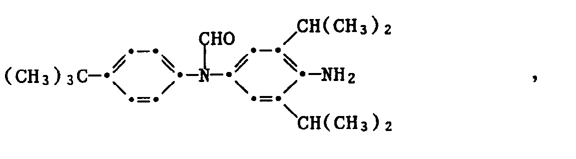 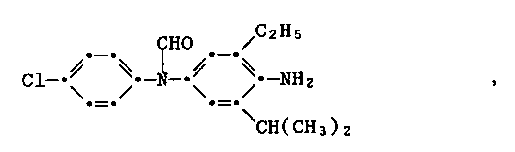 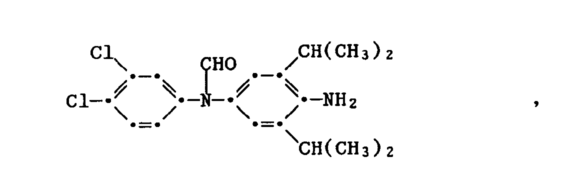 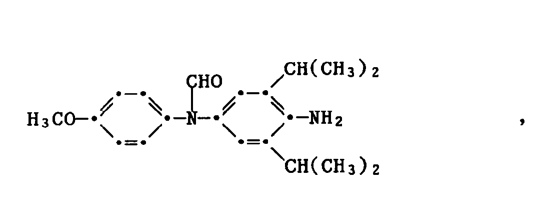 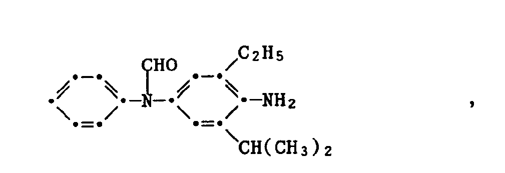 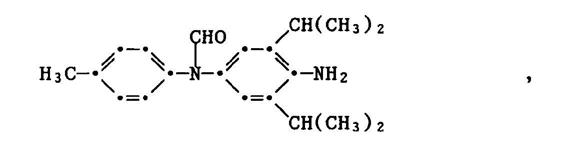 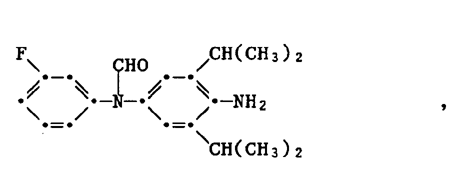 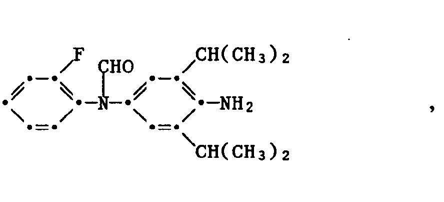 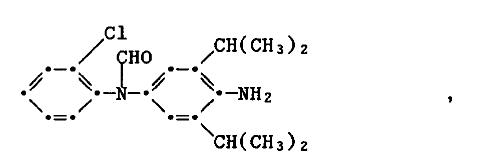 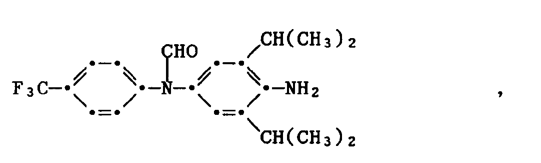 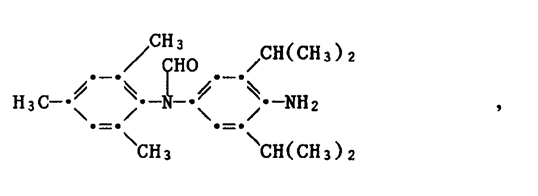 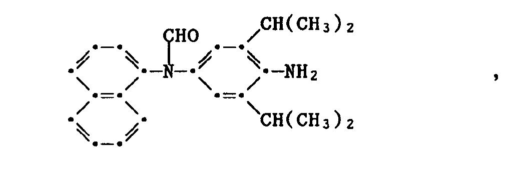 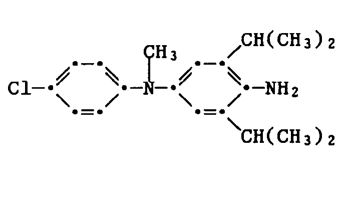 und,

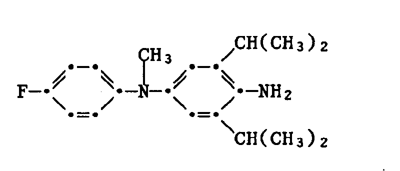

21. Verfahren zur Herstellung der Verbindungen der Formel II 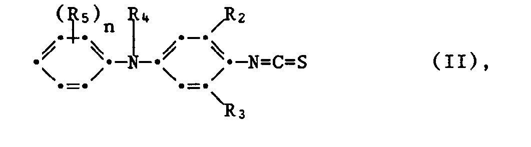 worin
R₂ und R₃ je für C₁-C₅-Alkyl oder C₅-Cₛ-Cycloalkyl;
R₄ für Wasserstoff, Methyl oder -CHO;
R₅ je für Halogen, C₁-C₄-Alkyl, ein- oder mehrfach durch Halogen substituiertes C₁-C₄-Alkyl, C₁-C₄-Alkoxy oder für eine (̵CH=CH)̵₂, (̵CH₂)̵₃ oder 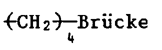 in 2,3- oder 3,4-Stellung und
n für 0, 1, 2, 3 oder 4 stehen, dadurch gekennzeichnet, dass man ein Aminodiphenylamin der Formel 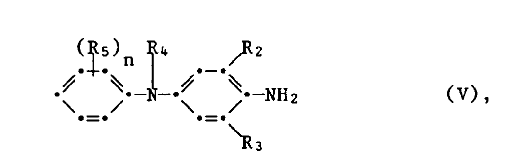 mit Thiophosgen umsetzt, wobei R₂, R₃, R₄, R₅ und n die für Formel angegebene Bedeutung haben.

22. Verfahren gemäss Anspruch 21, worin R₂ für C₁-C₅-Alkyl; R₃ für C₁-C₅-Alkyl oder C₅-C₆-Cycloalkyl; R₄ für Wasserstoff, Methyl oder -CHO; R₅ je für Halogen, C₁-C₃-Alkyl oder für eine (̵CH=CH)̵₂, 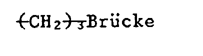 in 3,4-Stellung; und n für 0, 1 oder 2 stehen.

23. Verfahren gemäss Anspruch 22, worin R₂ und R₃ je für C₁-C₄-Alkyl; R₄ für Wasserstoff, Methyl oder -CHO; R₅ für Methyl; und n für 0 oder 1 stehen.

24. Verfahren gemäss Anspruch 21 zur Herstellung der Verbindungen der Formeln 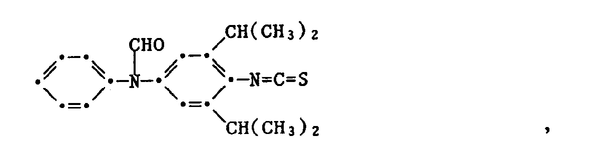 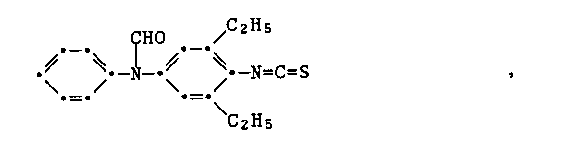 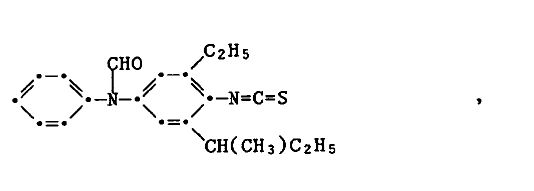 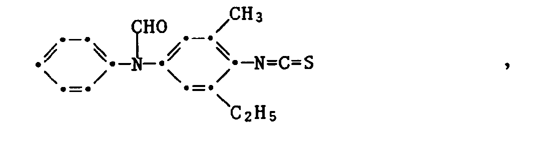 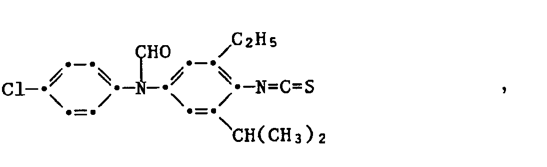 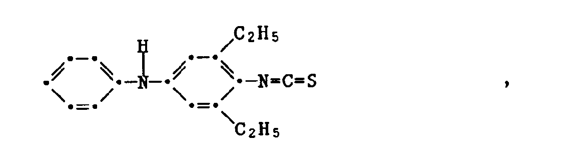 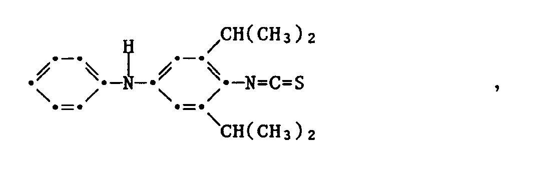 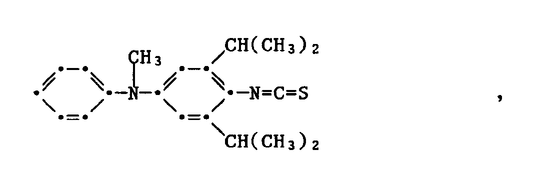 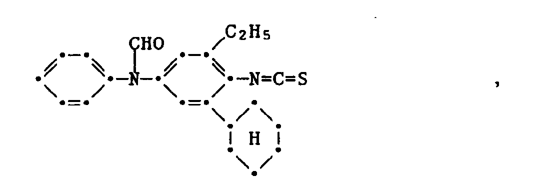 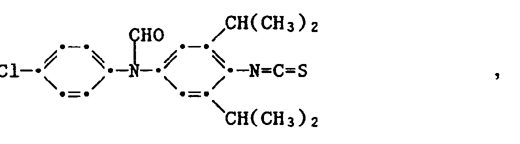 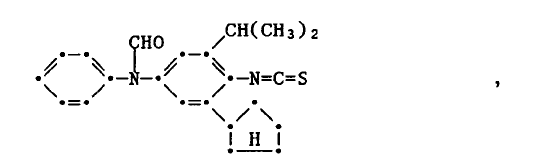 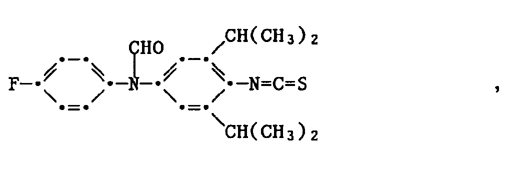 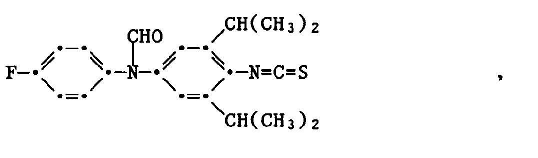 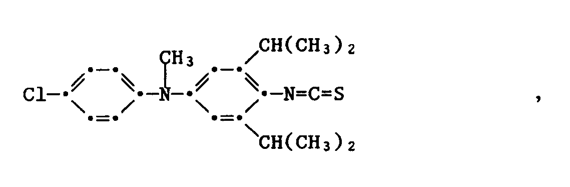 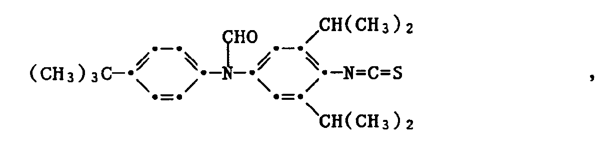 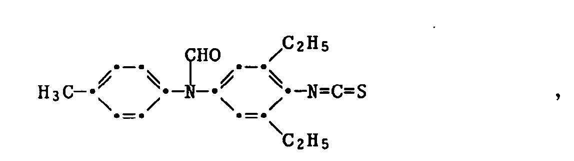 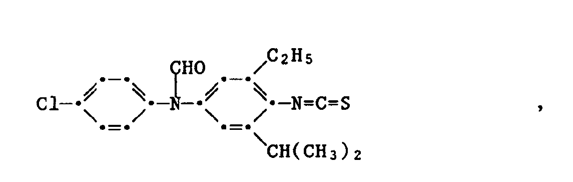 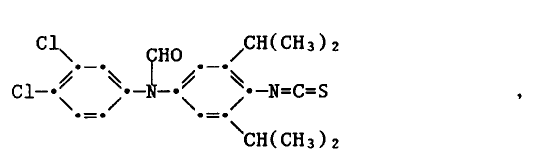 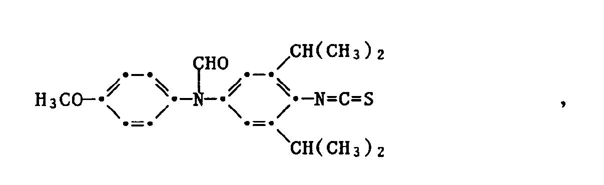 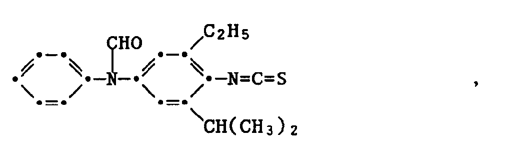 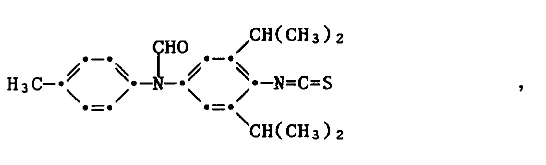 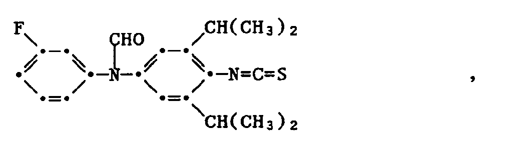 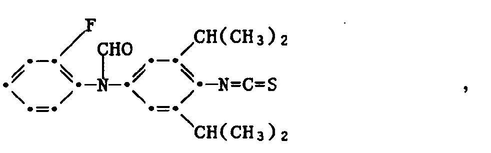 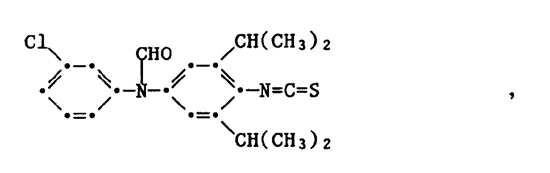 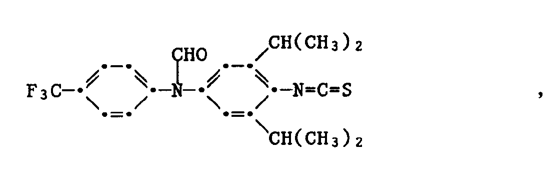 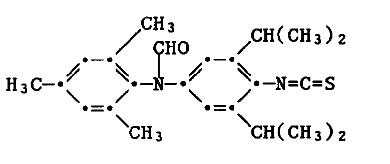 und,

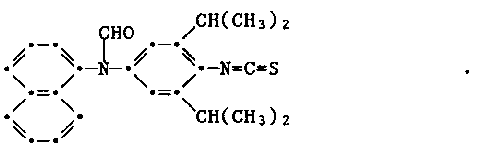

## Claims (Claims for the following Contracting State(s) : AT, BE, CH, DE, FR, GB, IT, LI, NL)

1. A compound of formula I 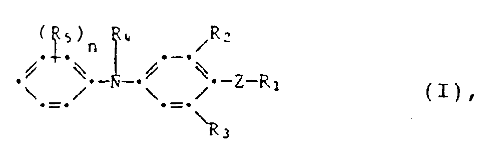 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈Cycloalkyl substituted by one or more C₁-C₃alkyl groups; or C₃-C₈Cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C-₄alkoxy substituted by one or more halogen atoms; or a (̵CH=CH)̵₂, (̵CH₂)̵₃or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or a salt thereof with an organic or inorganic acid.

2. A compound of formula I according to claim 1, wherein R₁ is C₁-C₈alkyl, C₁-C₈alkyl substituted by one or more halogen atoms and/or C₁-C₅alkoxy groups, or is C₃-C₈-cycloalkyl; R₂ is C₁-C₅alkyl; R₃ is C₁-C₅alkyl or C₅-Cₛcycloalkyl; R₄ is hydrogen, methyl or-CHO; R₅ is halogen, C₁-C₃alkyl or a (̵CH=CH)̵₂ or (̵CH₂)̵₃ bridge in the 3,4-position; n is 0, 1 or 2; Z is -NH-CS-NH-, -N=C(SR₆)-NH- or -N=C=N-; and R₆ is C₁-C₄alkyl.

3. A compound of formula I according to claim 2, wherein R₁ is C₁-C₅alkyl or cyclopentyl; R₂ and R₃ are each C₁-C₄alkyl; R₄ is hydrogen, methyl or-CHO; R5 is methyl; n is 0 or 1; and Z is -NH-CS-NH-.

4. A compound of formula I according to claim 2, wherein R₁ is C₁-C₅alkyl or cyclopentyl; R₂ and R₃ are each C₁-C₄alkyl; R₄ is hydrogen, methyl or-CHO; R₅ is methyl; n is 0 or 1; Z is -N=C(SR₆)-NH-; and R₆ is C₁-C₄alkyl.

5. A compound of formula I according to claim 2, wherein R₁ is C₁-C₅alkyl or cyclopentyl; R₂ and R₃ are each C₁-C₄alkyl; R₄ is hydrogen, methyl or -CHO; R₅ is methyl; n is 0 or 1; and Z is -N=C=N-.

6. A compound according to any one of claims 1 to 3 of formula

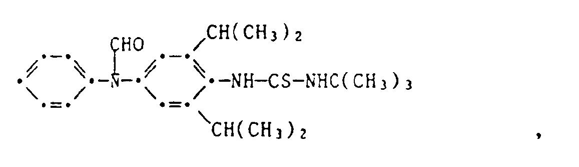

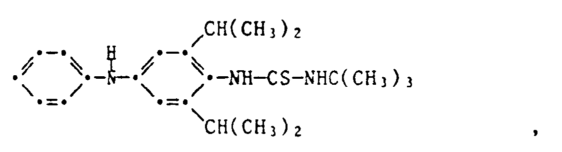

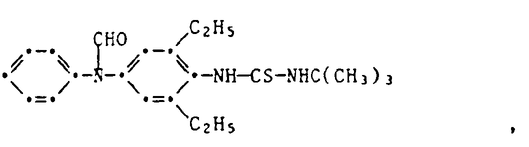

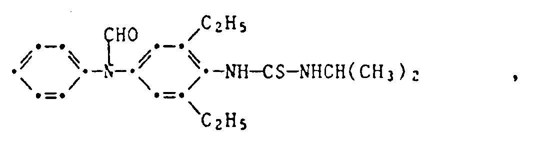

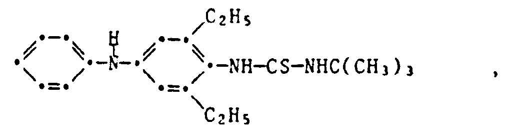

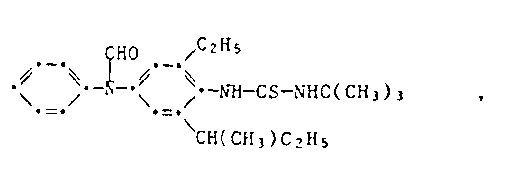

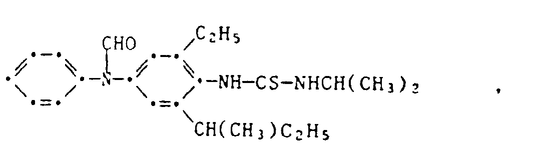

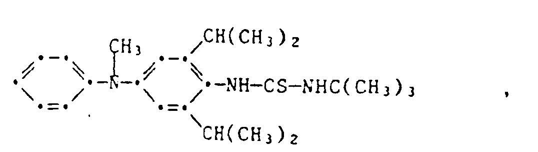

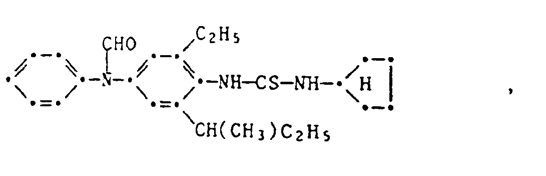

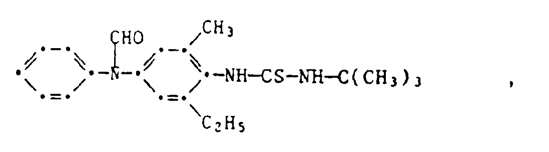

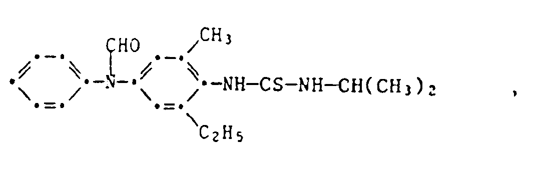

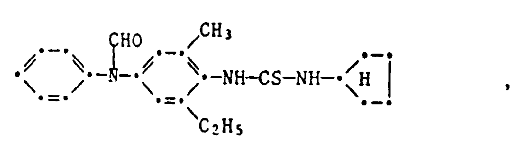

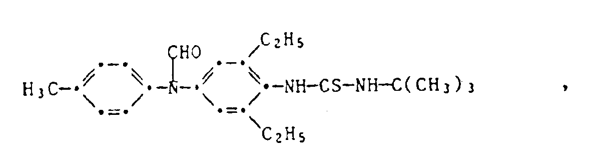

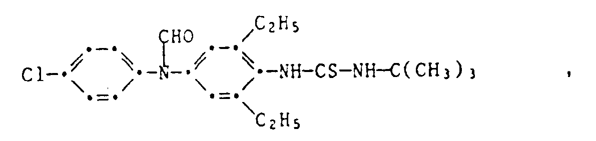

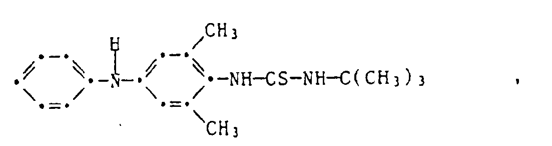

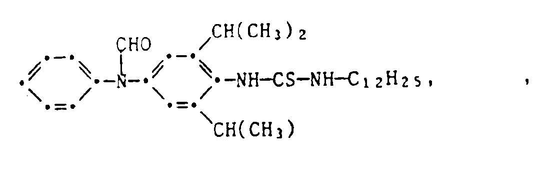

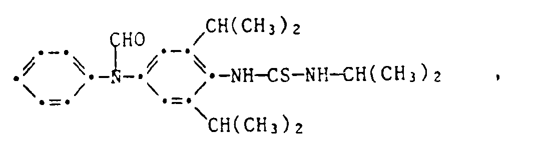

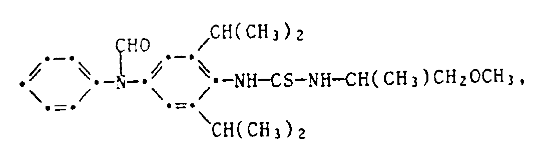

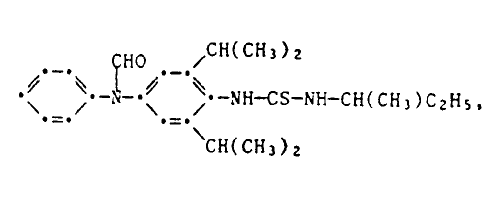

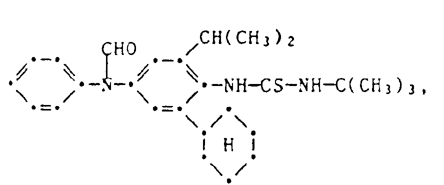

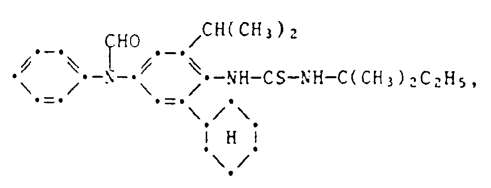

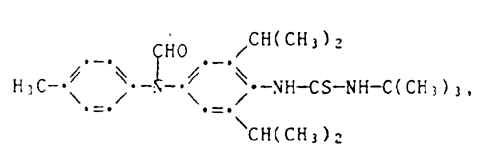

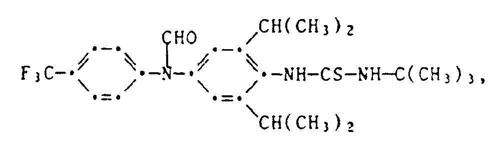

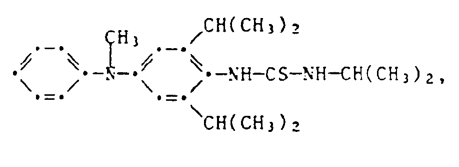

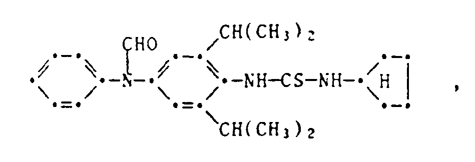

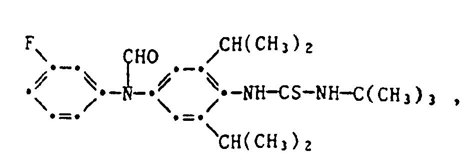

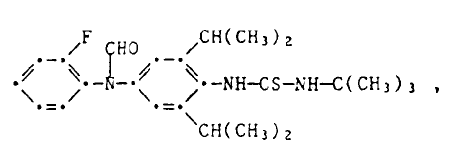

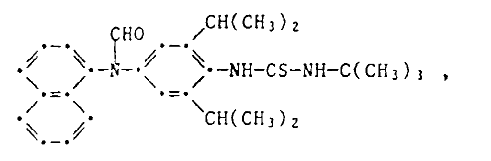

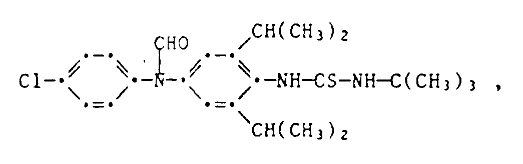

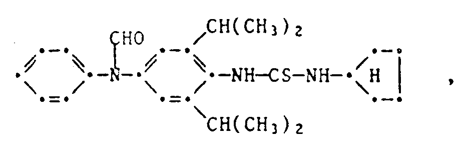

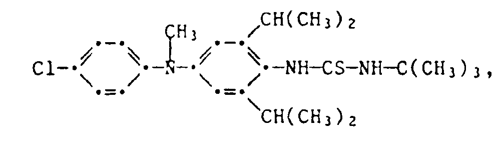

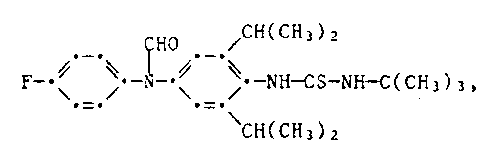

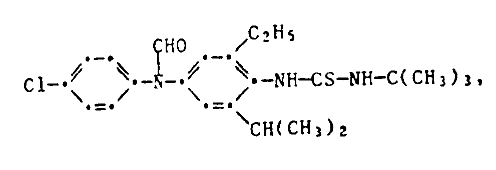

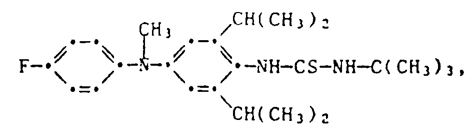

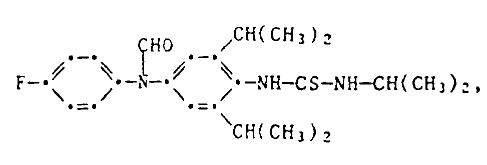

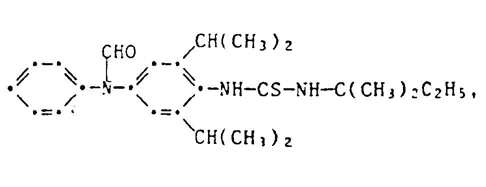

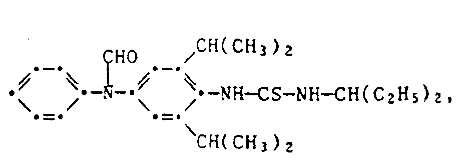

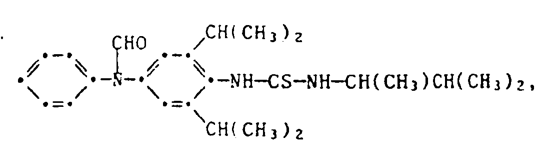

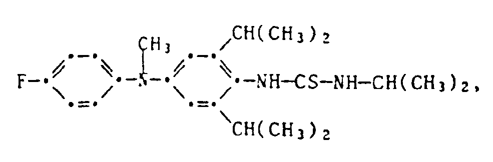

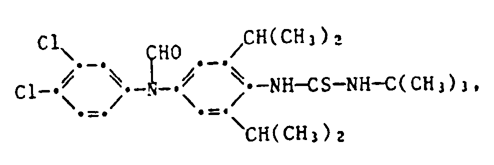

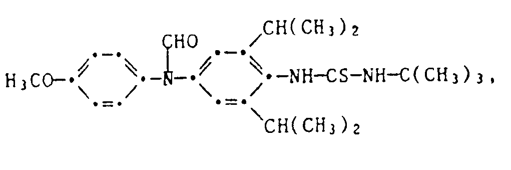

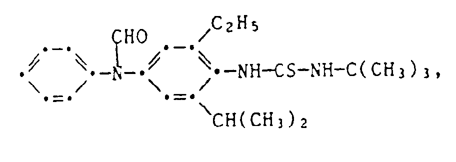

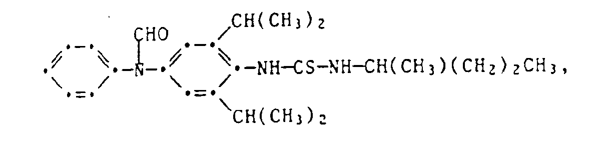

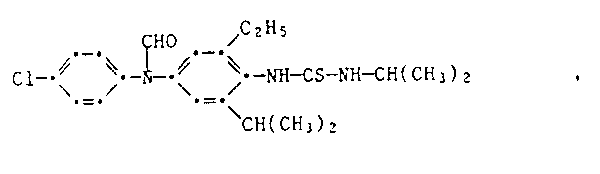

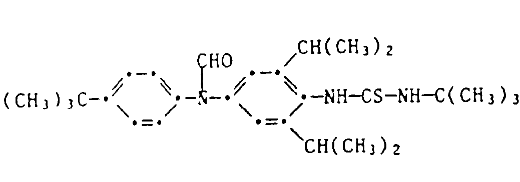

or

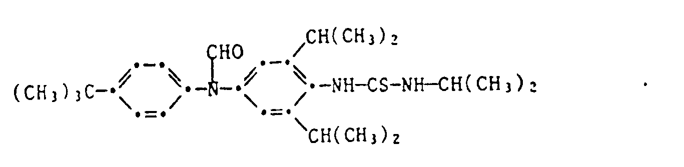

7. A compound according to any one of claims 1, 2 or 4 of formula 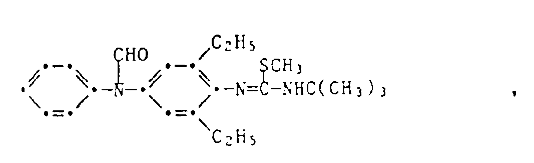 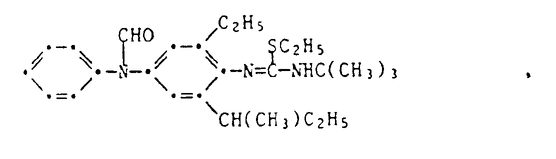 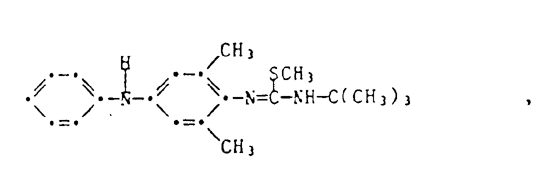 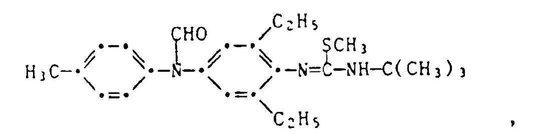 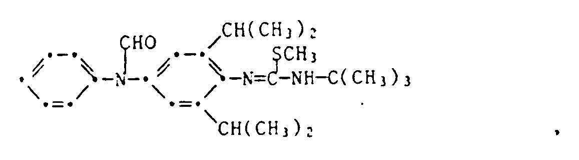 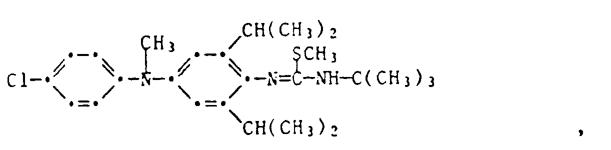 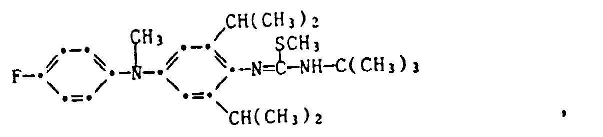 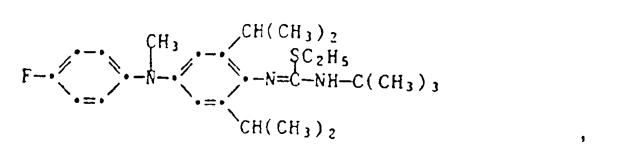 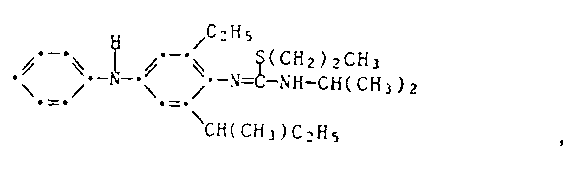 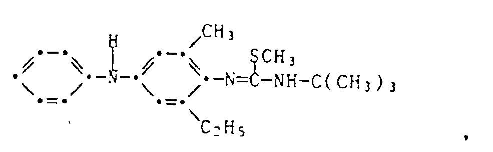 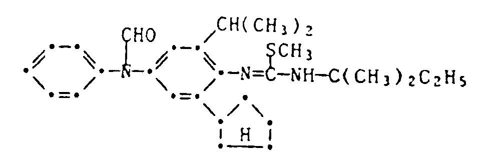 or

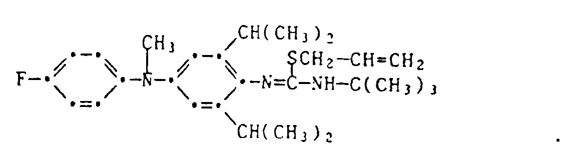

8. A compound according to any one of claims 1, 2 or 5 of formula 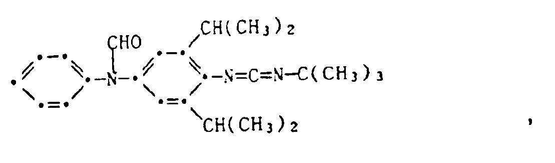 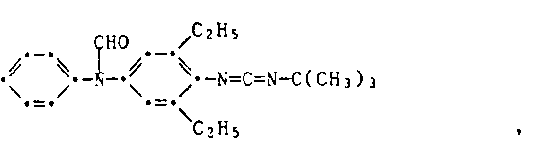 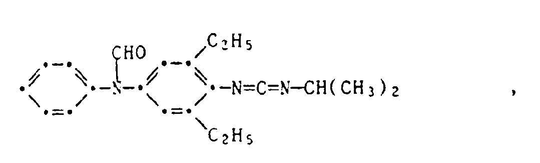 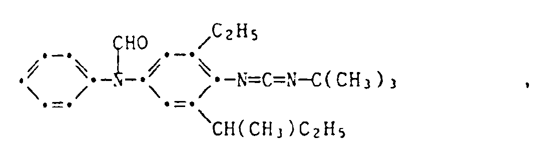 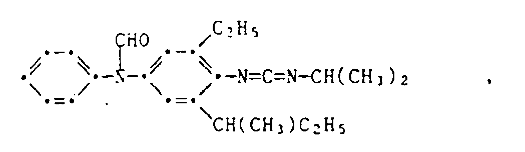 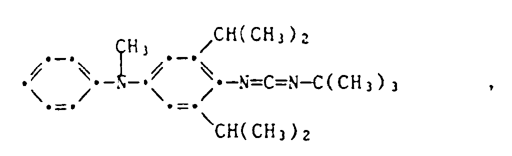 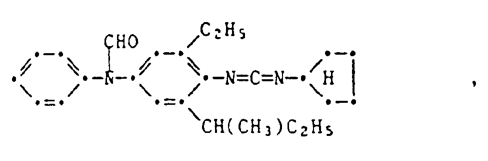 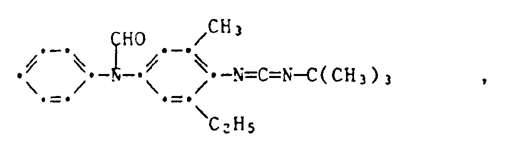 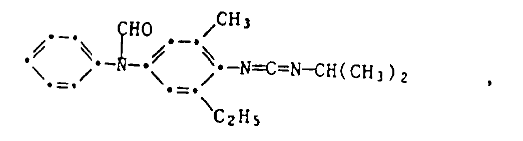 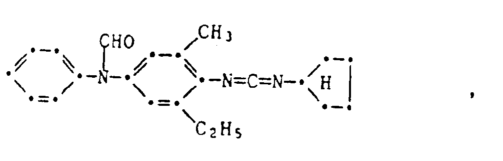 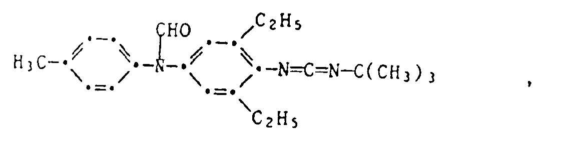 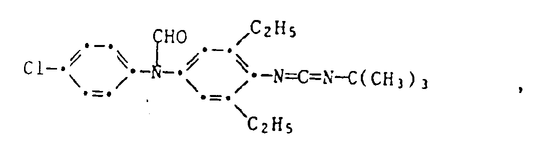 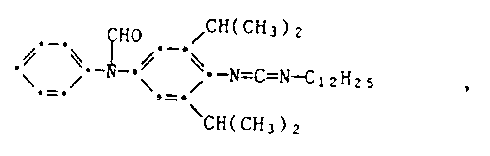 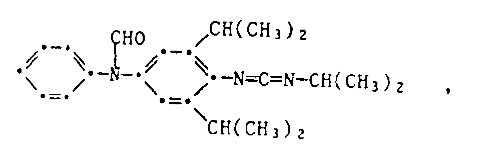 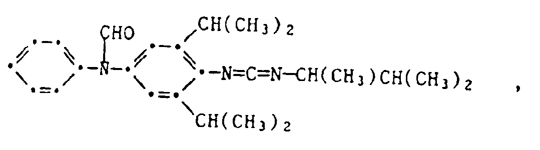 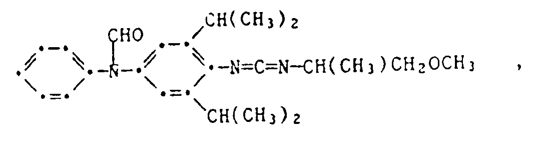 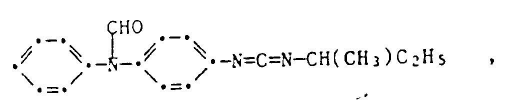 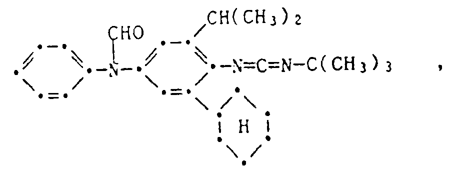 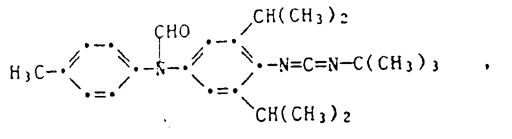 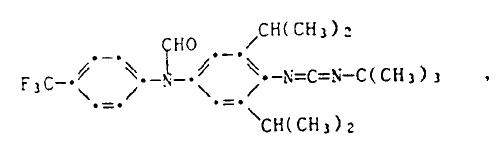 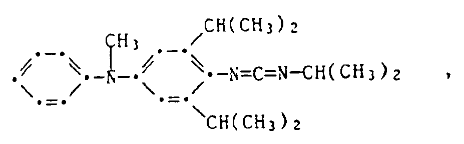 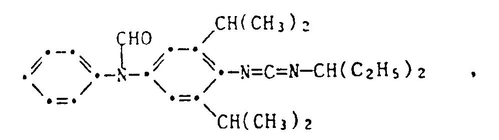 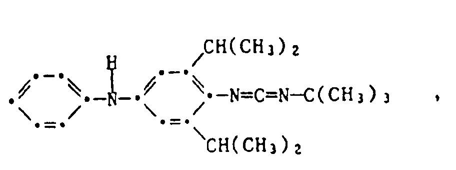 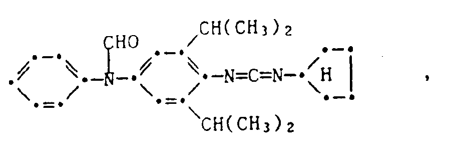 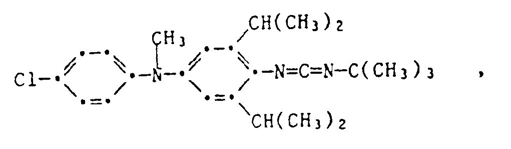 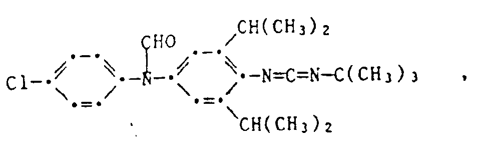 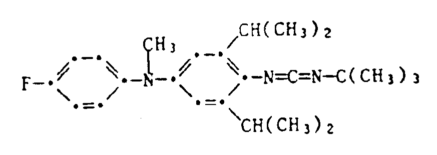 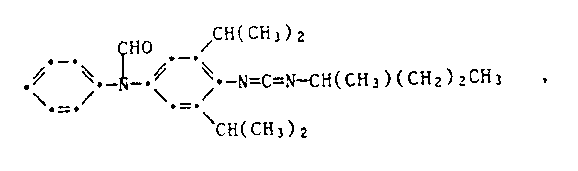 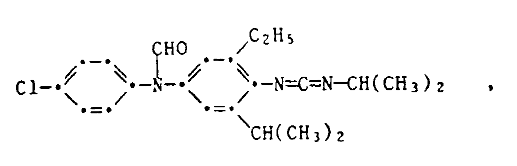 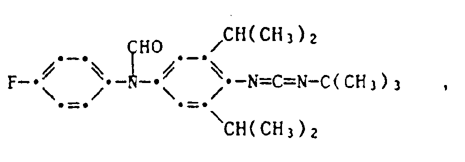 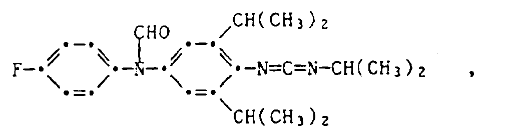 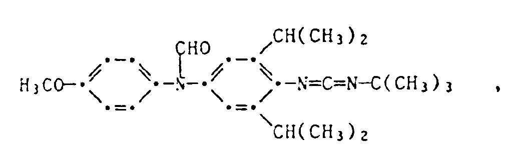 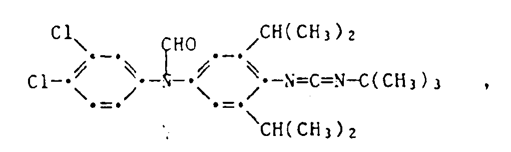 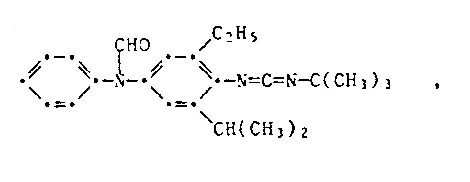 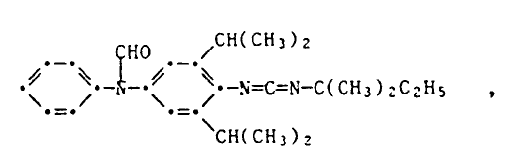 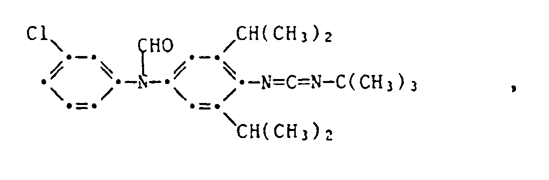 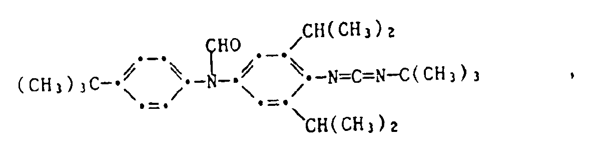 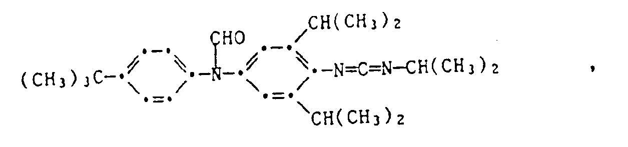 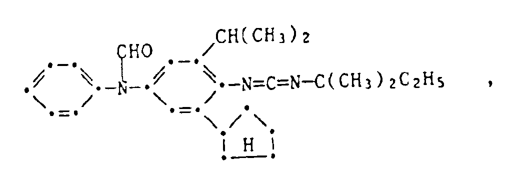 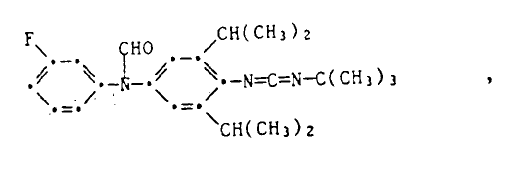 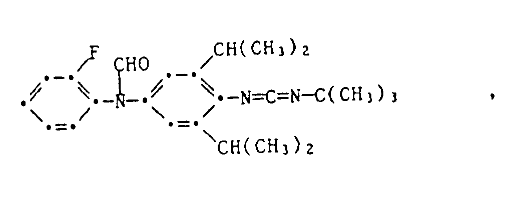 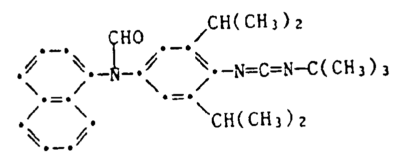 or

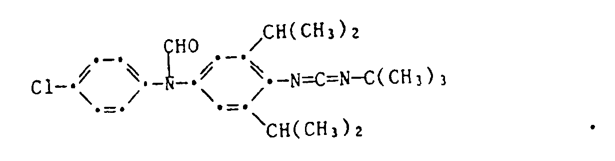

9. A process for the preparation of a compound of formula I 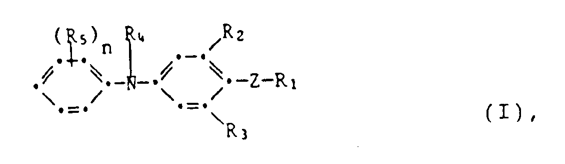 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4, Z is -NH-CS-NH-, -N=C(SR₆)-NH- or -N=C=N-, and
R₆ is C₁-C₆-alkyl or allyl, which process comprises
A) reacting an isothiocyanate of formula II 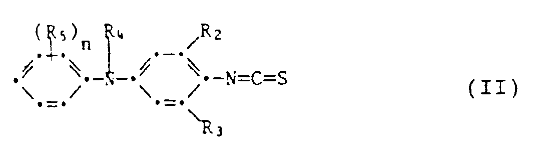 with an amine of formula III 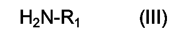 in an organic solvent or diluent at normal pressure and at a temperature of from 0 to +150_{°}C to give the thiourea and, if desired,
B) reacting the resultant thiourea with a compound of formula IV 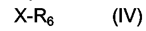 in an inert organic solvent under slightly elevated or normal pressure and at a temperature of from +10 to 250°C to give the isothiourea, or
C) converting the resultant thiourea into the carbodiimide by removal of hydrogen sulfide in an aprotic organic solvent or diluent under normal pressure and at a temperature of from 0 to +150_{°}C, in which formulae above R₁, R₂, R₃, R₄, R₅, R₆ and n have the given meanings and X is a leaving group.

10. A pesticidal composition comprising, as active component, at least one compound of formula I 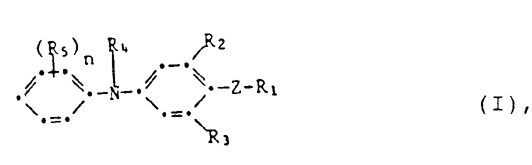 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy, C₁-C₄ alkoxy substituted by one or more halogen atoms; or a (̵CH=CH)̵, (̵CH₂)̵₃,or (̵CH₂)̵₄, bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or a salt thereof with an organic or inorganic acid, together with suitable carriers and/or adjuvants.

11. A pesticidal composition according to claim 10 comprising, as active component, at least one compound of formula I wherein R₁ is C₁-C₈alkyl; C₁-C₈alkyl substituted by one or more halogen atoms and/or C₁-C₅alkoxy groups; or C₃-C₈cycloalkyl; R₂ is C₁-C₅alkyl; R₃ is C₁-C₅alkyl or C₅-C₆cycloalkyl; R₄ is hydrogen, methyl or-CHO; R₅ is halogen, C₁-C₃alkyl or a (̵CH=CH)̵₂, or (̵CH₂)̵₃ bridge in the 3,4-position; n is 0, 1 or 2; Z is -NH-CS-NH-, -N=C(SR₆)-NH- or-N=C=N-; and R₆ is C₁-C₄-alkyl.

12. A pesticidal composition according to claim 11, wherein R₁ is C₁-C₅alkyl or cyclopentyl; R₂ and R₃ are each C₁-C₄alkyl; R₄ is hydrogen, methyl or-CHO; R₅ is methyl; n is 0 or 1; and Z is -NH-CS-NH-.

13. A pesticidal composition according to claim 11, wherein R₁ is C₁-C₅alkyl or cyclopentyl; R₂ and R₃ are each C₁-C₄alkyl; R₄ is hydrogen, methyl or-CHO; R₅ is methyl; n is 0 or 1; Z is -N=C(SR₆)-NH-; and R₆ is C₁-C₄alkyl.

14. A pesticidal composition according to claim 11, wherein R₁ is C₁-C₅alkyl or cyclopentyl; R₂ and R₃ are each C₁-C₄alkyl; R₄ is hydrogen, methyl or -CHO; R₅ is methyl; n is 0 or 1; and Z is -N=C=N-.

15. The use of a compound of formula I 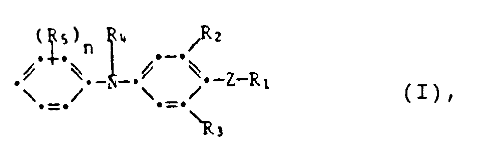 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl, C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or a salt thereof with an organic or inorganic acid, for controlling pests of animals and plants.

16. The use according to claim 15 for controlling insects and arachnids.

17. A method of controlling pests of animals and plants, which comprises contacting said pests in their different development stages with a compound of formula I 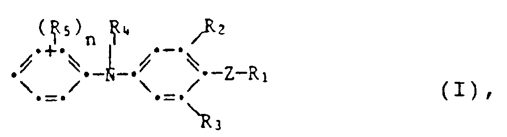 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈Cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃, or (̵CH₂)̵₄, bridge in the 2,3-or 3,4- position;
n is 0, 1, 2, 3 or 4,
Z is -NH-CS-NH-, -N=C(SR₆)-NH- or -N=C=N-, and
R₆ is C₁-C₆alkyl or allyl, or with a salt thereof with an organic or inorganic acid.

18. A compound of formula V 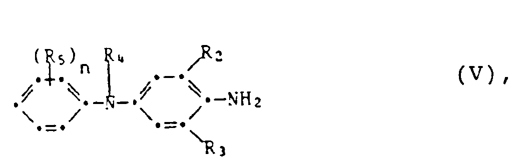 wherein
R₂ and R₃ are each C₁-C₅alkyl or C₅-Cₛcycloalkyl;
R₄ is hydrogen, methyl or -CHO;
R₅ is halogen; C₁-C₄-alkyl; C₁-C₄alkyl substituted by one or more halogen atoms; C₁-C₄alkoxy; or a (̵CH=CH)̵₂, (̵CH₂)̵₃, or (̵CH₂)̵₄, bridge in the 2,3- or 3,4-position; and
n is 0, 1, 2, 3 or 4.

19. A compound of formula V according to claim 18, wherein R₂ is C₁-C₅alkyl; R₃ is C₁-C₄alkyl or C₅-Cₛcycloalkyl; R₄ is hydrogen, methyl or-CHO; R₅ is halogen, C₁-C₃alkyl or a (̵CH=CH)̵₂, or (̵CH₂)̵₃, bridge in the 3,4- position; and n is 0, 1 or 2.

20. A compound offormula V according to claim 19, wherein R₂ and R₃ are each C₁-C₄alkyl; R₄ is hydrogen, methyl or -CHO; R₅ is methyl; and n is 0 or 1.

21. A compound according to claim 18 of formula

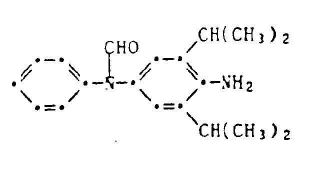

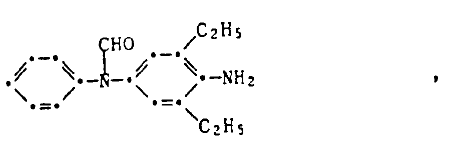

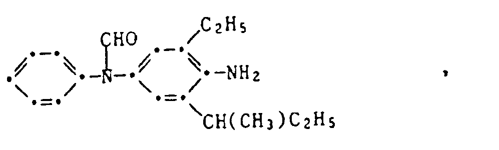

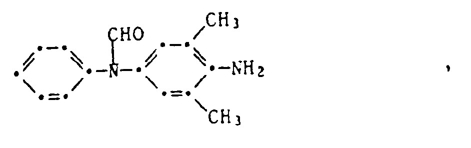

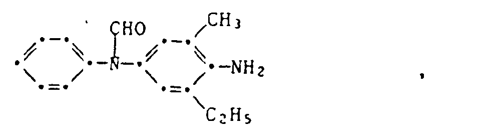

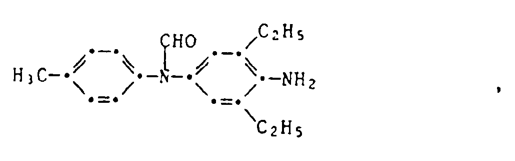

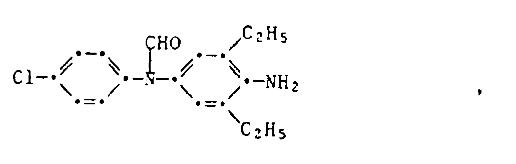

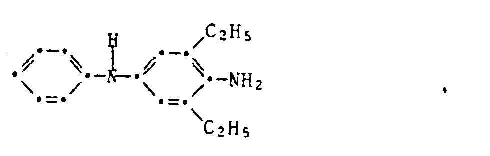

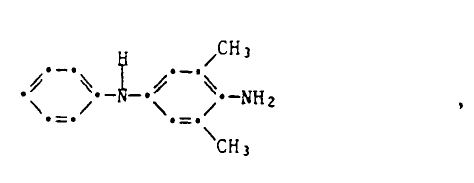

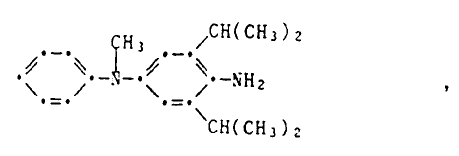

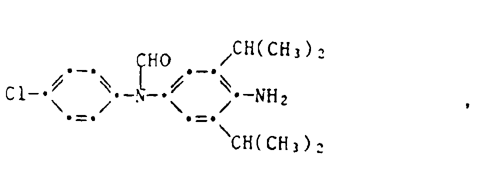

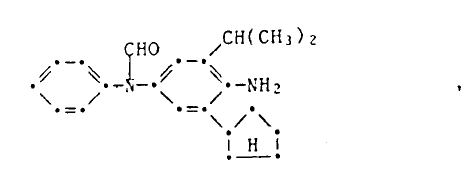

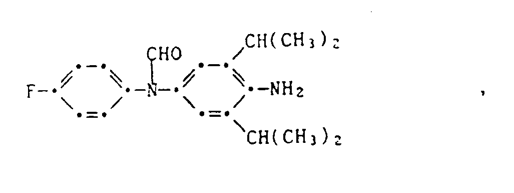

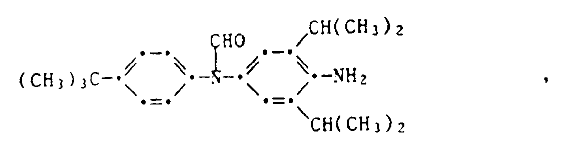

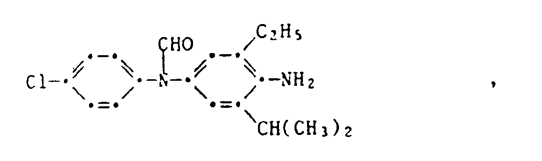

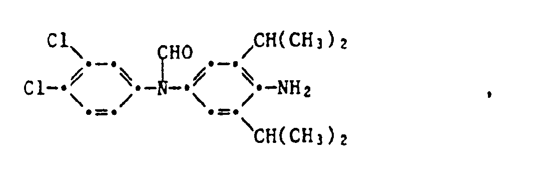

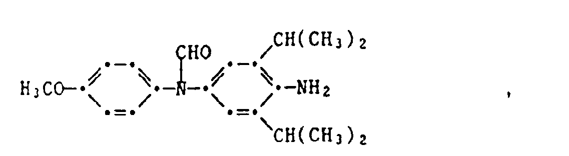

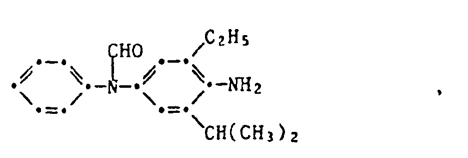

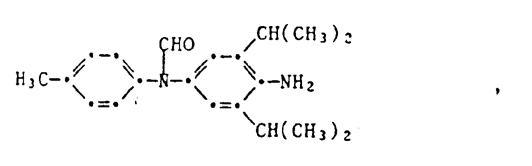

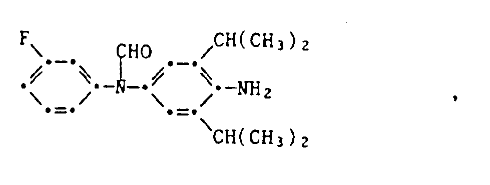

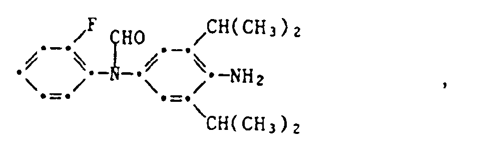

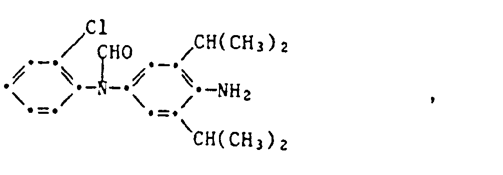

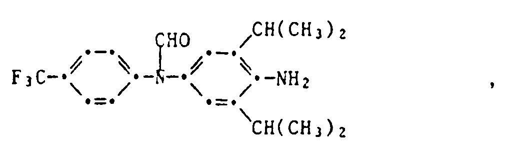

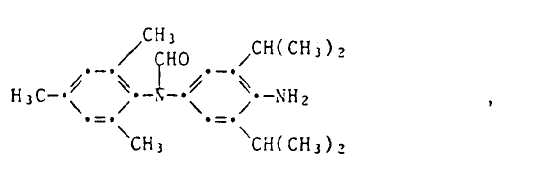

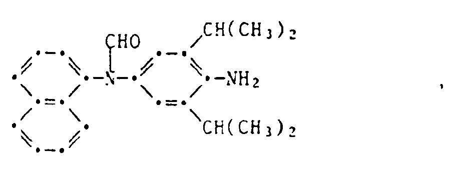

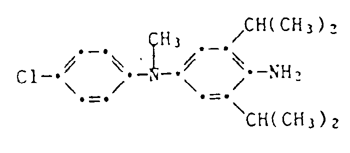

or

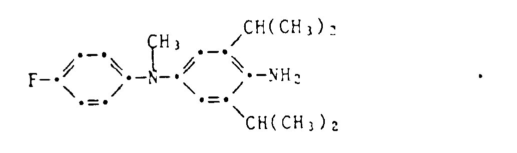

22. A compound of formula II 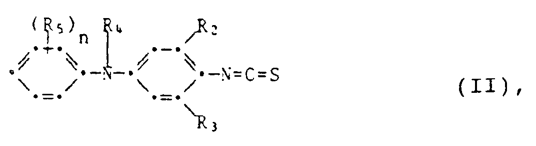 wherein
R₂ and R₃ are each C₁-C₅alkyl or C₅-Cₛcycloalkyl;
R₄ is hydrogen, methyl or -CHO;
R₅ is halogen; C₁-C₄-alkyl; C₁-C₄alkyl substituted by one or more halogen atoms; C₁-C₄alkoxy; or a (̵CH=CH)̵₂, (̵CH₂)̵₃ or (̵CH₂)̵₄ bridge in the 2,3- or 3,4-position; and
n is 0, 1, 2, 3 or 4.

23. A compound of formula II according to claim 22, wherein R₂ is C₁-C₅alkyl; R₃ is C₁-C₅alkyl or C₅-Cₛcycloalkyl; R₄ is hydrogen, methyl or-CHO; R₅ is halogen, C₁-C₃alkyl or a (̵CH=CH)̵₂, or (̵CH₂)̵₃, bridge in the 3,4- position; and n is 0, 1 or 2.

24. A compound offormula II according to claim 23, wherein R₂ and R₃ are each C₁-C₄alkyl; R₄ is hydrogen, methyl or -CHO; R₅ is methyl; and n is 0 or 1.

25. A compound according to claim 22 of formula

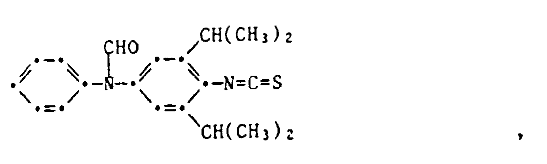

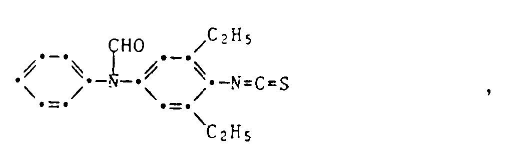

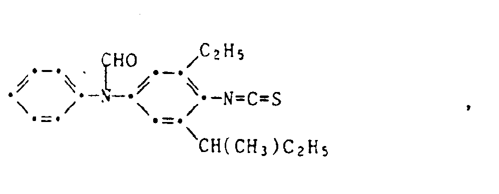

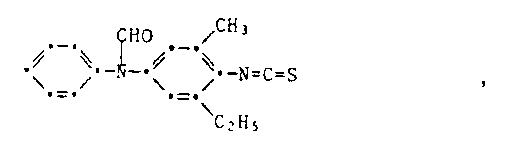

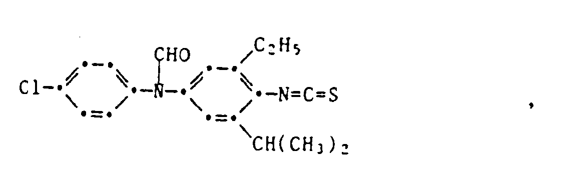

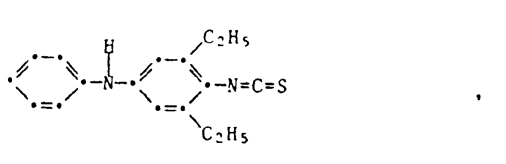

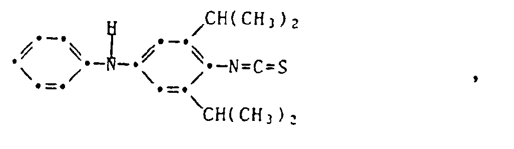

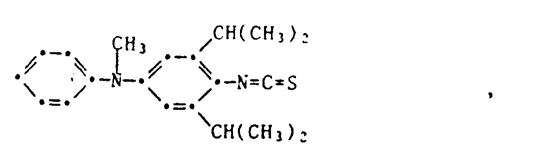

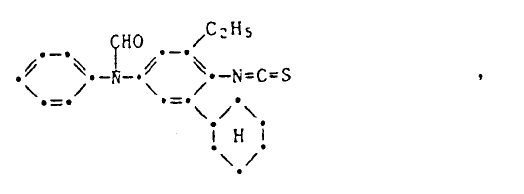

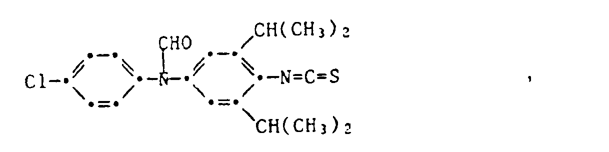

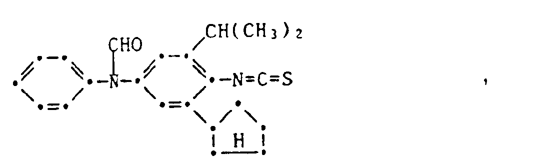

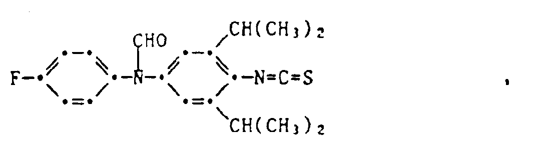

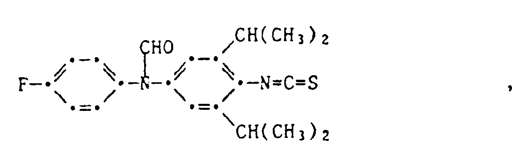

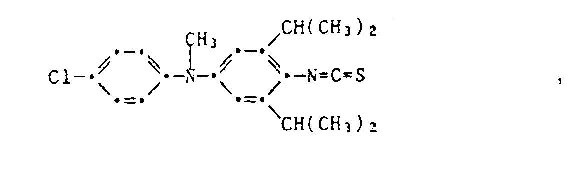

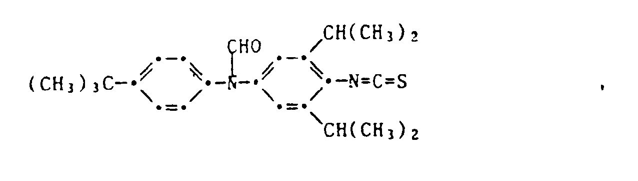

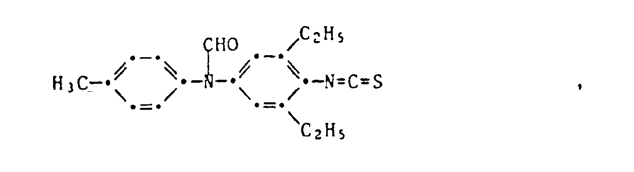

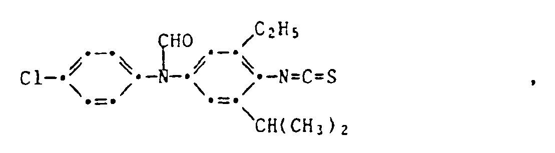

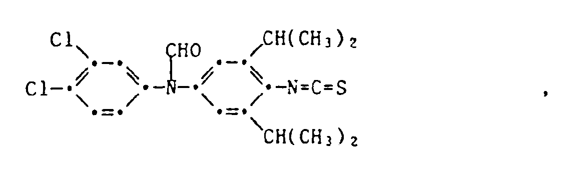

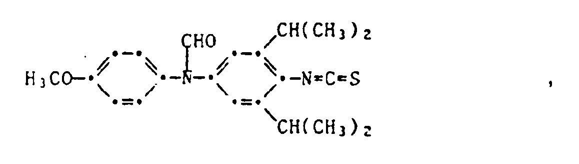

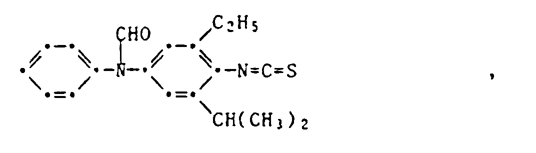

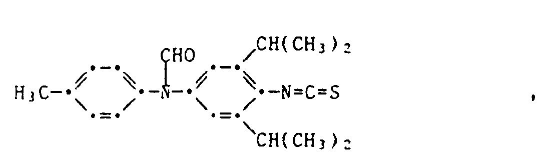

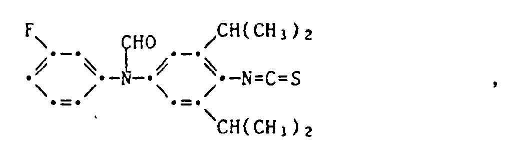

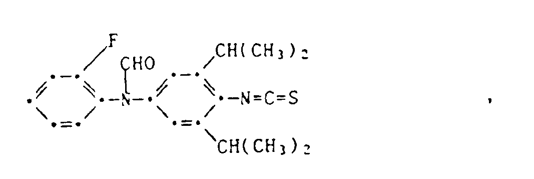

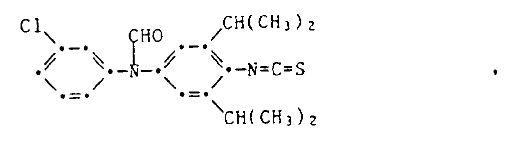

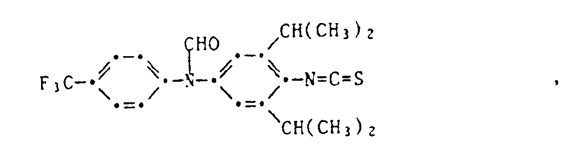

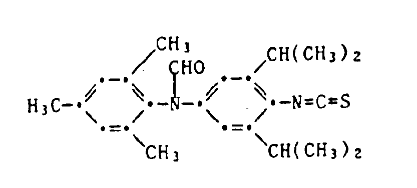

or

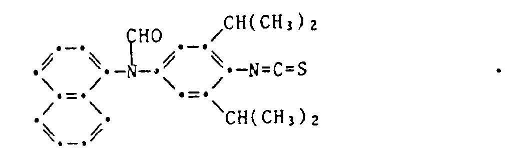

## Claims (Claims for the following Contracting State(s) : ES)

1. A process for the preparation of a compound of formula I 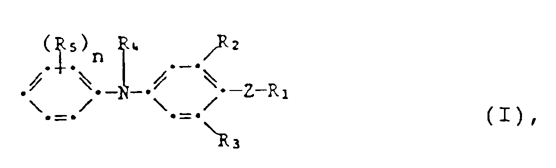 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄a-1koxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, which process comprises
A) reacting an isothiocyanate of formula II 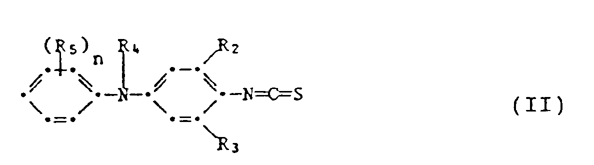 with an amine of formula III 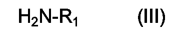 in an organic solvent or diluent at normal pressure and at a temperature of from 0 to +150_{°}C to give the thiourea and, if desired,
B) reacting the resultant thiourea with a compound of formula IV 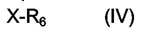 in an inert organic solvent under slightly elevated or normal pressure and at a temperature of from +10 to 250°C to give the isothiourea, or
C) converting the resultant thiourea into the carbodiimide by removal of hydrogen sulfide in an aprotic organic solvent or diluent under normal pressure and at a temperature of from 0 to +150_{°}C, in which formulae above R₁, R₂, R₃, R₄, R₅, R₆ and n have the given meanings and X is a leaving group.

2. A process according to claim 1, wherein R₁ is C₁-C₈-alkyl, C₁-C₈alkyl substituted by one or more halogen atoms and/or C₁-C₅alkoxy groups, or is C₃-C₈cycloalkyl; R₂ is C₁-C₅alkyl; R₃ is C₁-C₅alkyl or C₅-Cₛcycloalkyl; R₄ is hydrogen, methyl or-CHO; R₅ is halogen, C₁-C₃alkyl or a (̵CH=CH)̵₂ or (̵CH₂)̵₃, bridge in the 3,4-position; n is 0, 1 or 2; Z is -NH-CS-NH-, -N=C(SR₆)-NH- or -N=C=N-; and R₆ is C₁-C₄alkyl.

3. A process according to claim 2, wherein R₁ is C₁-C₅-alkyl or cyclopentyl; R₂ and R₃ are each C₁-C₄alkyl; R₄ is hydrogen, methyl or-CHO; R₅ is methyl; n is 0 or 1; and Z is -NH-CS-NH-.

4. A process according to claim 2, wherein R₁ is C₁-C₅-alkyl or cyclopentyl; R₂ and R₃ are each C₁-C₄alkyl; R₄ is hydrogen, methyl or-CHO; R₅ is methyl; n is 0 or 1; Z is -N=C(SR₆)-NH-; and R₆ is C₁-C₄alkyl.

5. A process according to claim 2, wherein R₁ is C₁-C₅-alkyl or cyclopentyl; R₂ and R₃ are each C₁-C₄alkyl; R₄ is hydrogen, methyl or-CHO; R₅ is methyl; n is 0 or 1; and Z is -N=C=N-.

6. A process according to any one of claims 1 to 3 for the preparation of a compound of formula

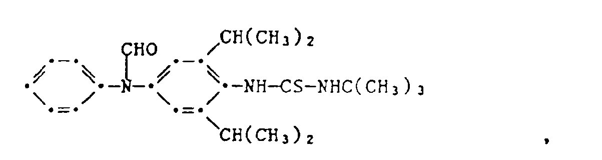

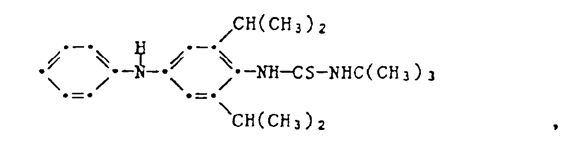

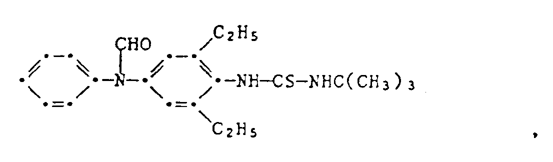

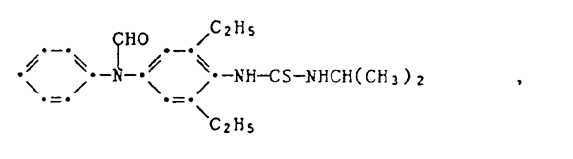

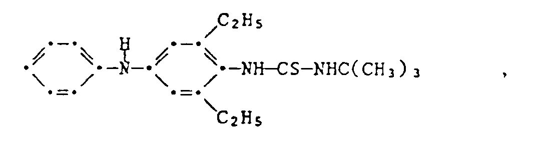

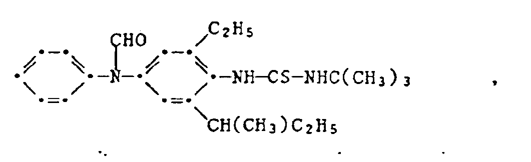

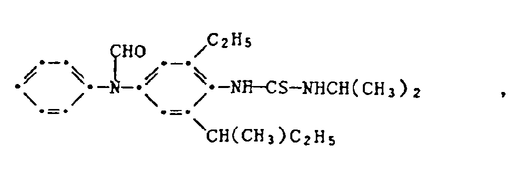

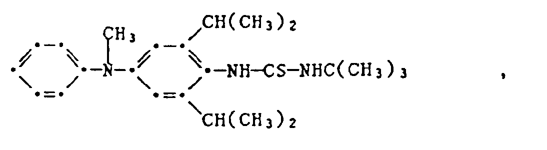

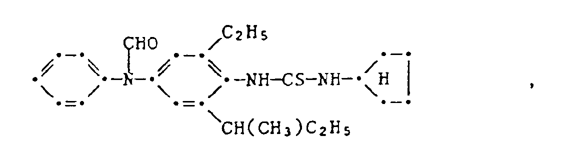

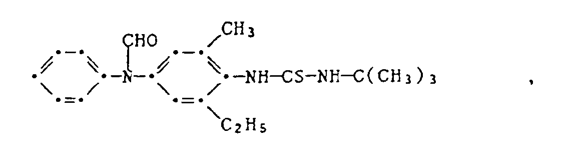

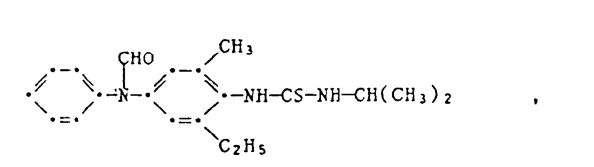

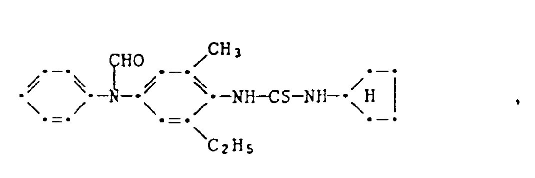

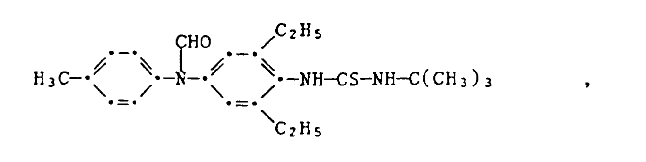

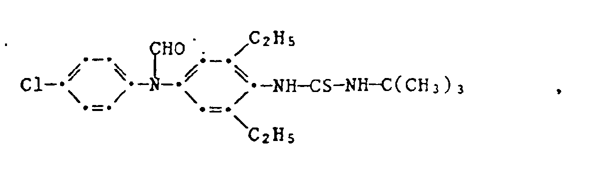

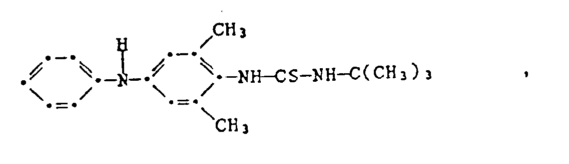

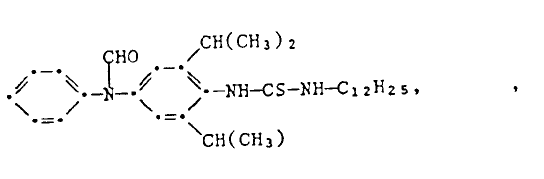

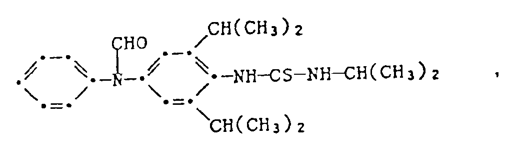

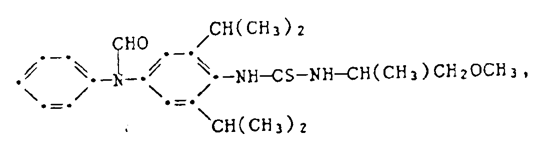

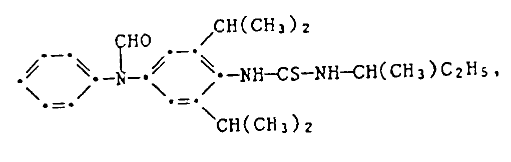

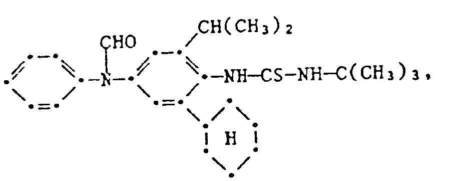

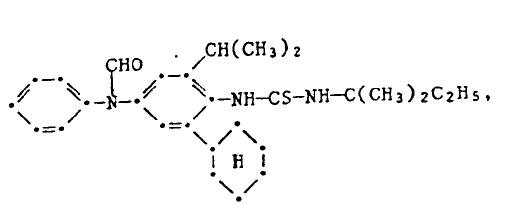

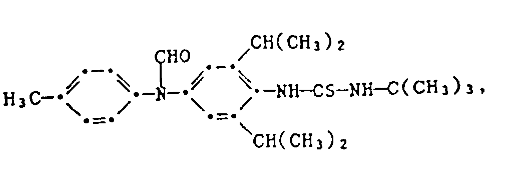

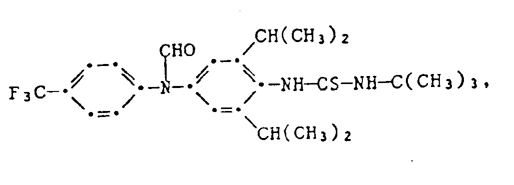

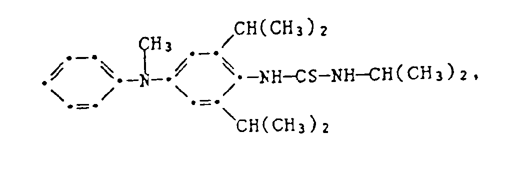

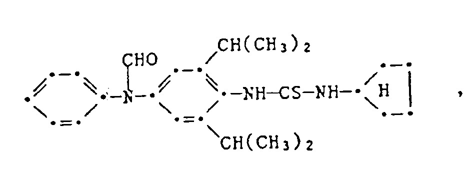

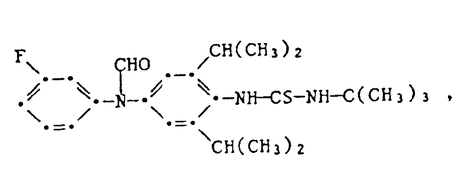

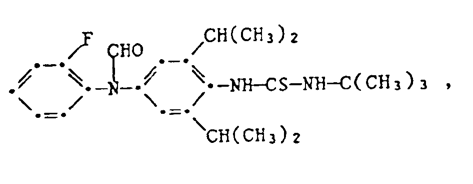

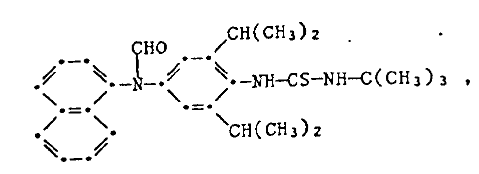

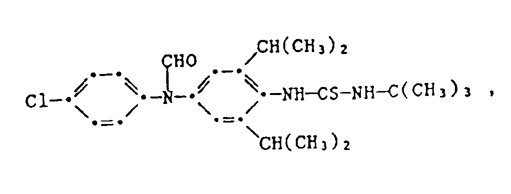

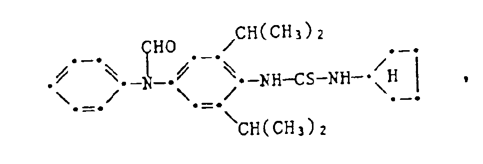

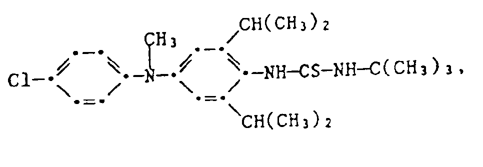

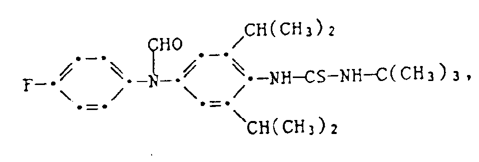

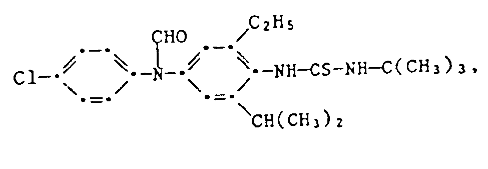

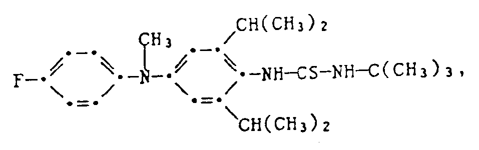

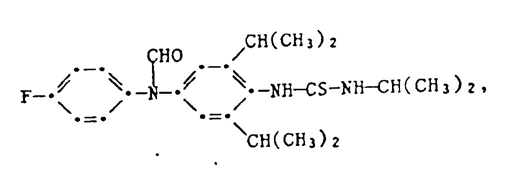

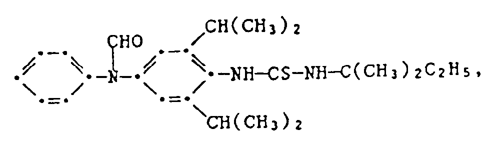

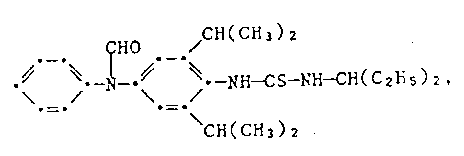

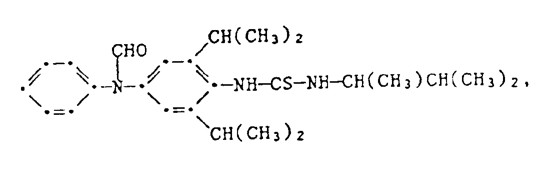

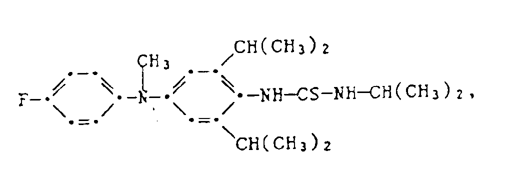

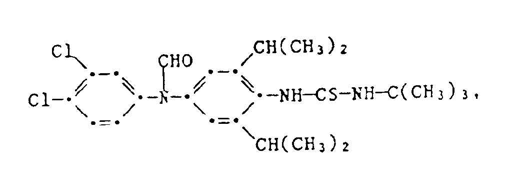

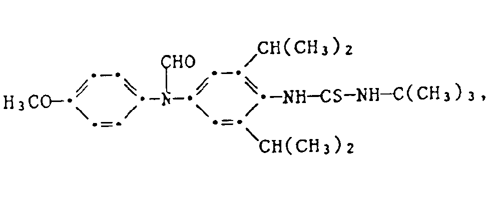

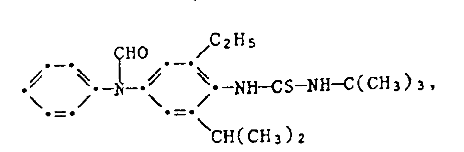

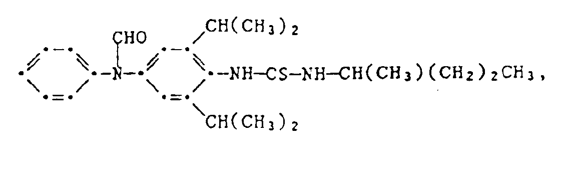

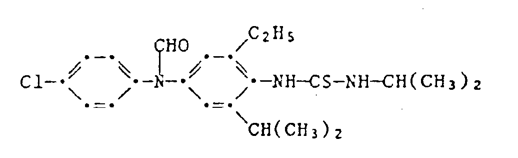

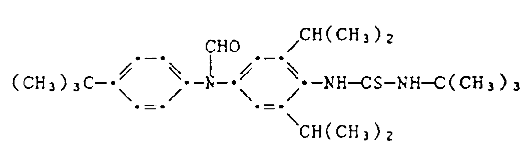

or

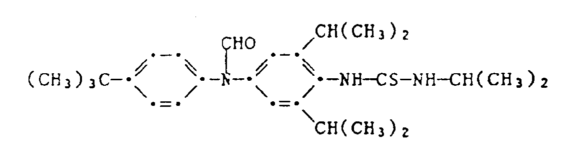

7. A process according to any one of claims 1, 2 or 4 for the preparation of a compound of formula 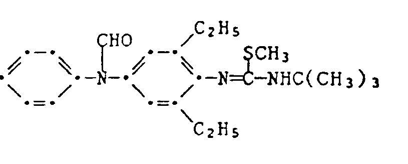 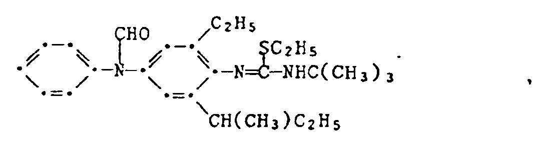 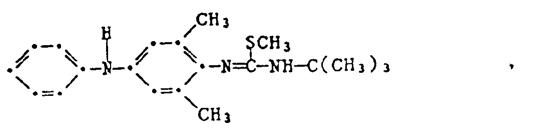 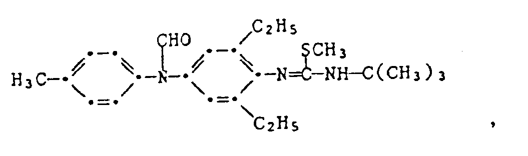 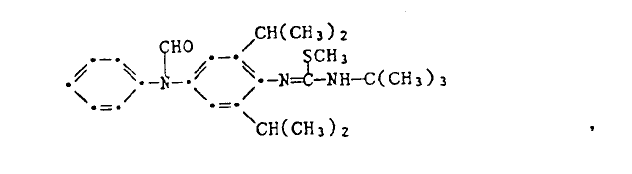 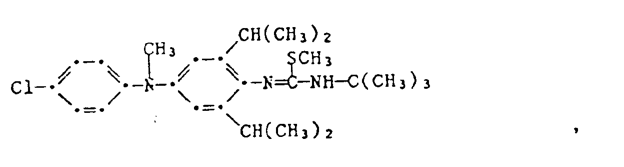 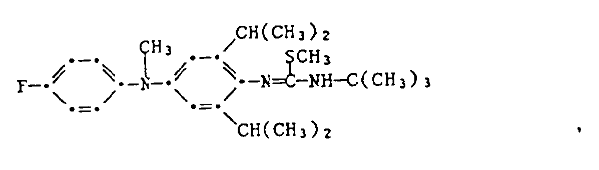 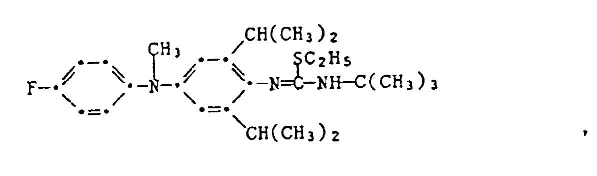 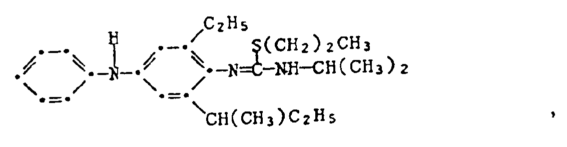 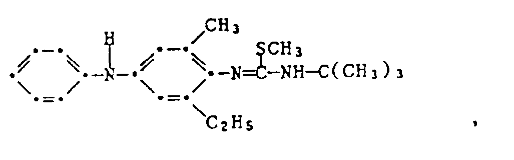 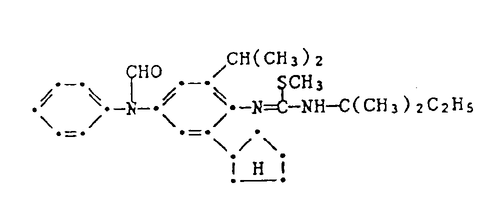 or

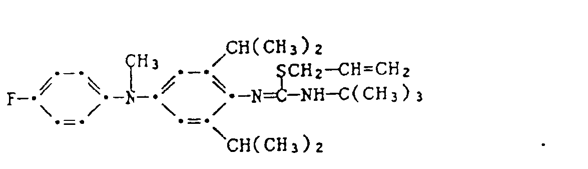

8. A process according to any one of claims 1, 2 or 5 for the preparation of a compound of formula 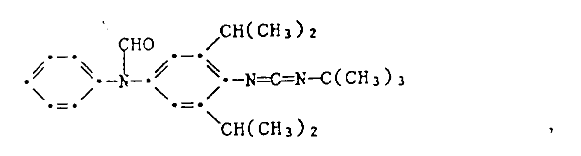 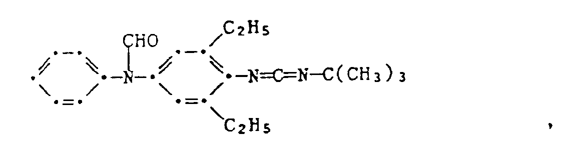 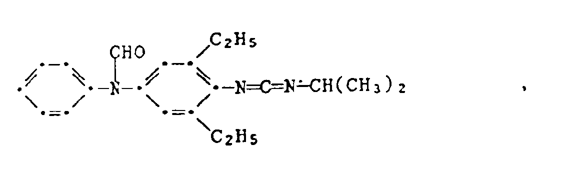 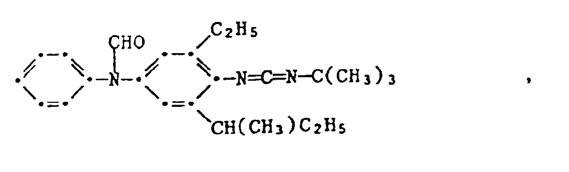 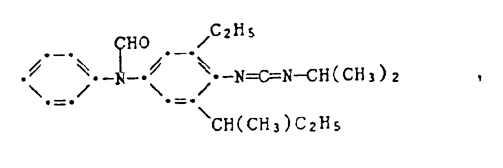 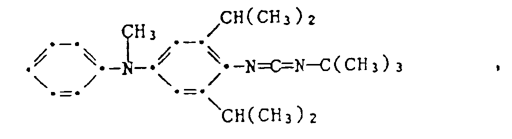 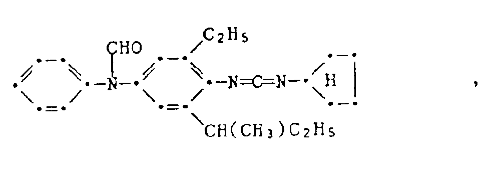 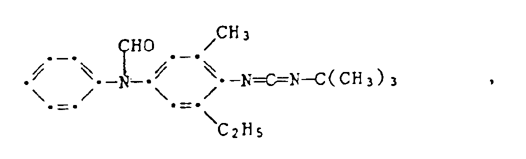 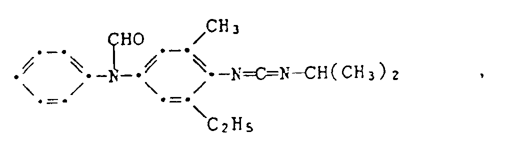 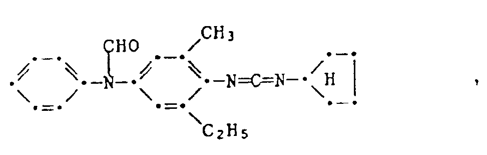 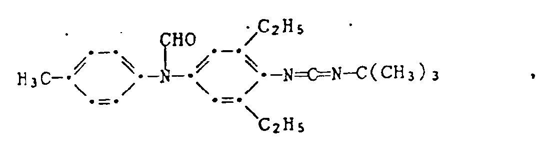 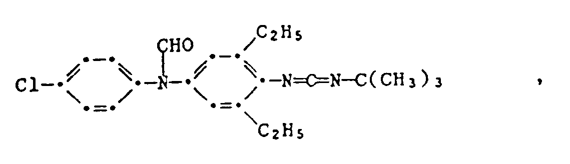 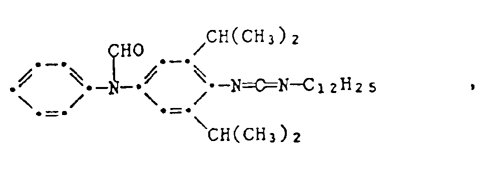 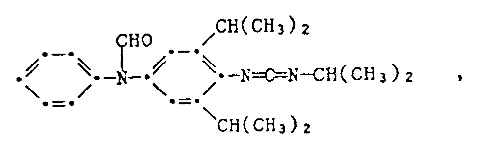 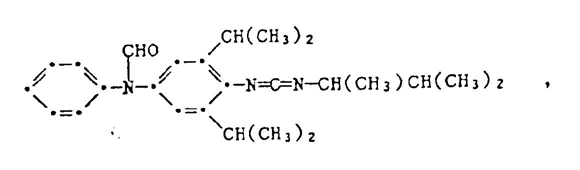 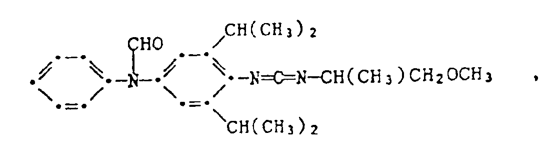 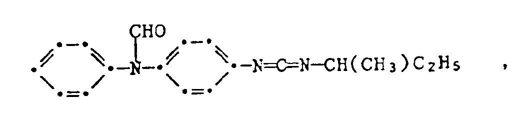 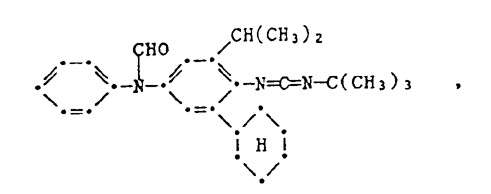 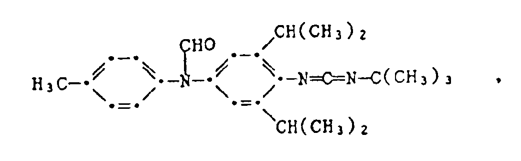 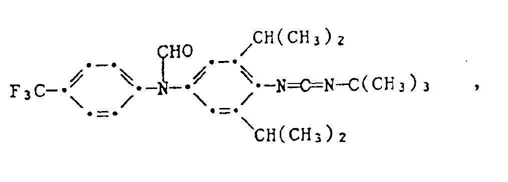 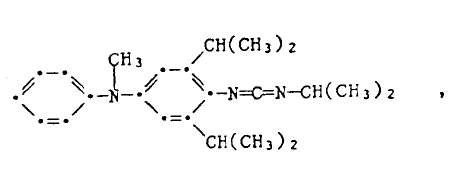 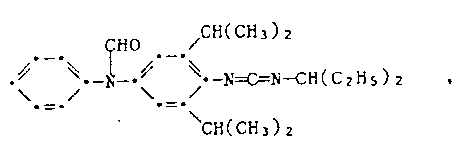 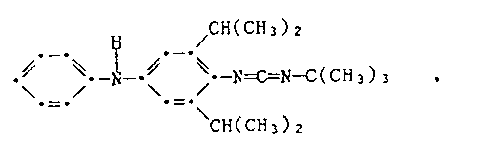 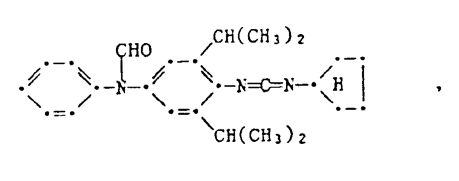 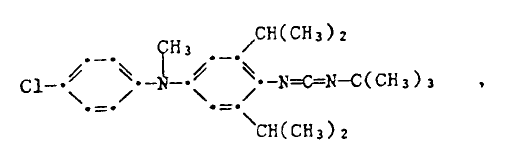 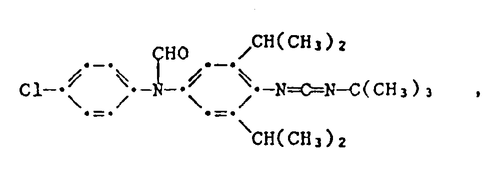 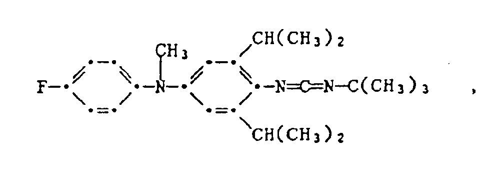 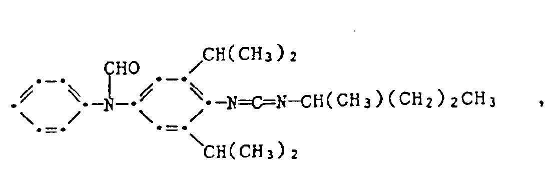 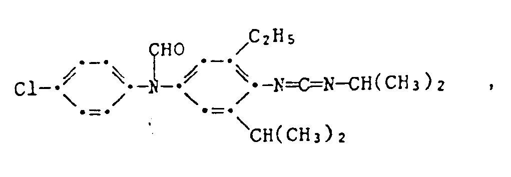 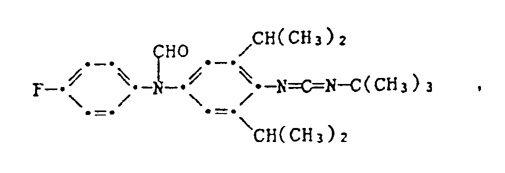 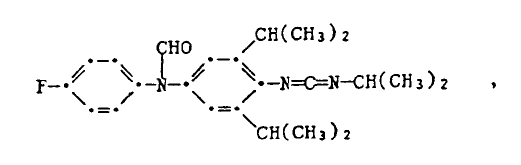 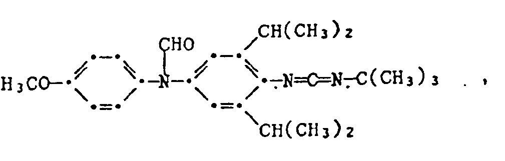 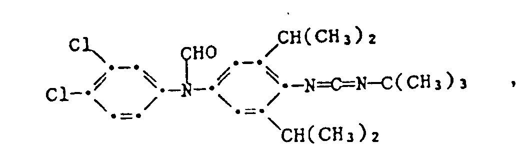 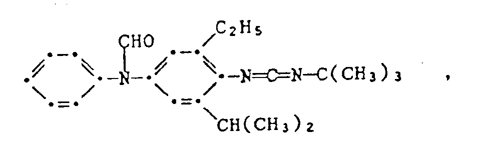 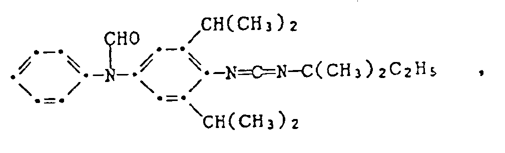 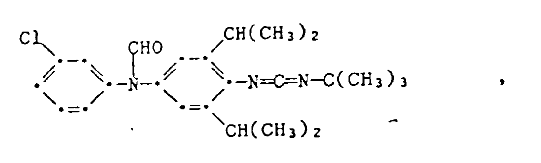 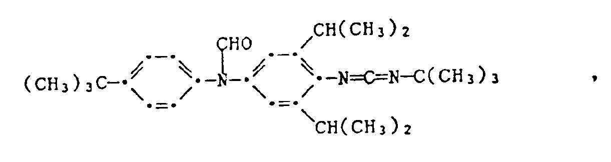 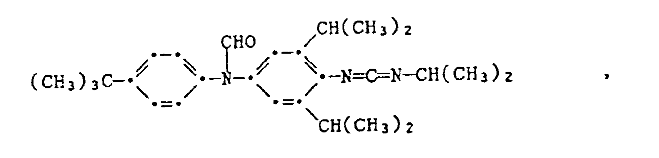 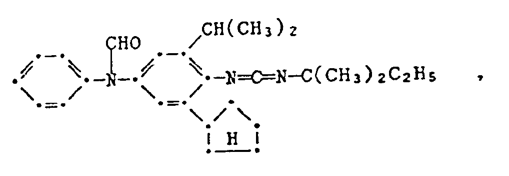 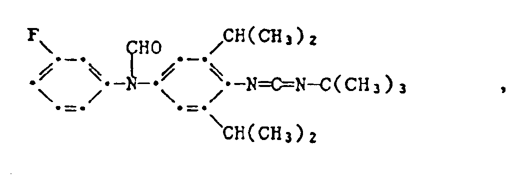 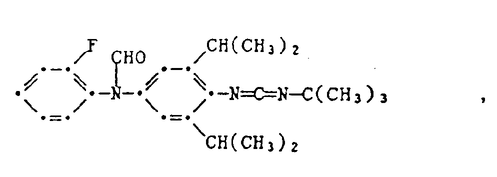 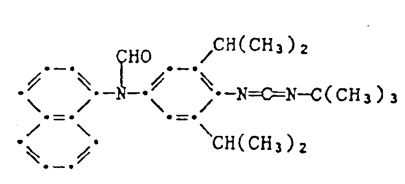 or

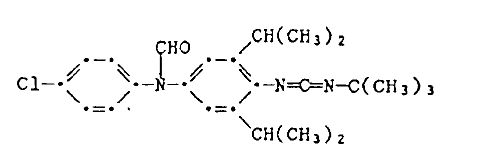

9. A pesticidal composition comprising, as active component, at least one compound of formula I 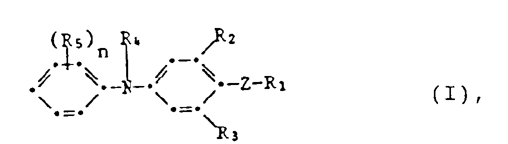 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or a salt thereof with an organic or inorganic acid, together with suitable carriers and/or adjuvants.

10. A pesticidal composition according to claim 9 comprising, as active component, at least one compound of formula I wherein R₁ is C₁-C₈alkyl; C₁-C₈alkyl substituted by one or more halogen atoms and/or C₁-C₅alkoxy groups; or C₃-C₈cycloalkyl; R₂ is C₁-C₅alkyl; R₃ is C₁-C₅alkyl or C₅-C₆cycloalkyl; R₄ is hydrogen, methyl or-CHO; R₅ is halogen, C₁-C₃alkyl or a (̵CH=CH)̵₂ or (̵CH₂)̵₃ bridge in the 3,4-position; n is 0, 1 or 2; Z is -NH-CS-NH-, -N=C(SR₆)-NH- or-N=C=N-; and R₆ is C₁-C₄-alkyl.

11. A pesticidal composition according to claim 10, wherein R₁ is C₁-C₅ₐₗkyl or cyclopentyl; R₂ and R₃3 are each C₁-C₄alkyl; R₄ is hydrogen, methyl or -CHO; R₅ is methyl; n is 0 or 1; and Z is -NH-CS-NH-.

12. A pesticidal composition according to claim 10, wherein R₁ is C₁-C₅alkyl or cyclopentyl; R₂ and R₃ are each C₁-C₄alkyl; R₄ is hydrogen, methyl or-CHO; R₅ is methyl; n is 0 or 1; Z is -N=C(SR₆)-NH-; and R₆ is C₁-C₄alkyl.

13. A pesticidal composition according to claim 10, wherein R₁ is C₁-C₅alkyl or cyclopentyl; R₂ and R₃ are each C₁-C₄alkyl; R₄ is hydrogen, methyl or -CHO; R₅ is methyl; n is 0 or 1; and Z is -N=C=N-.

14. The use of a compound of formula I 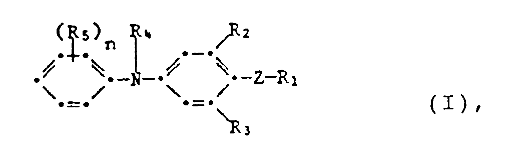 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈Cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or a salt thereof with an organic or inorganic acid, for controlling pests of animals and plants.

15. The use according to claim 14 for controlling insects and arachnids.

16. A method of controlling pests of animals and plants, which comprises contacting said pests in their different development stages with a compound of formula I 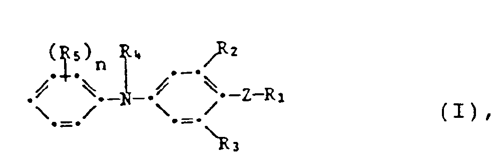 wherein
R₁ is C₁-C₁₂alkyl; C₁-C₁₂alkyl substituted by one or more halogen atoms and/or C₁-C₆alkoxy groups; C₃-C₈-cycloalkyl; C₃-C₈cycloalkyl substituted by one or more C₁-C₃alkyl groups; or C₃-C₈cycloalkyl-C₁-C₄alkyl;
R₂ and R₃ are each C₁-C₅alkyl or C₅-Cₛcycloalkyl;
R₄ is hydrogen, methyl or -CHO;
R₅ is halogen; C₁-C₄alkyl; C₁-C₄-alkyl substituted by one or more halogen atoms; C₁-C₄-alkoxy; C₁-C₄ alkoxy substituted by one or more halogen atoms; or a (̵CH=CH)̵₂, (̵CH₂)̵₃ or (̵CH₂)̵₄ bridge in the 2,3- or 3,4- position;
n is 0, 1, 2, 3 or 4,
Z is -NH-CS-NH-, -N=C(SR₆)-NH- or -N=C=N-, and
R₆ is C₁-C₆-alkyl or allyl, or with a salt thereof with an organic or inorganic acid.

17. A process for the preparation of a compound of formula V 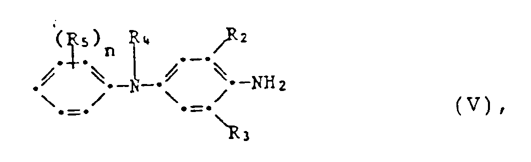 wherein
R₂ and R₃ are each C₁-C₅alkyl or C₅-Cₛcycloalkyl;
R₄ is hydrogen, methyl or -CHO;
R₅ is halogen; C₁-C₄-alkyl; C₁-C₄alkyl substituted by one or more halogen atoms; C₁-C₄alkoxy; or a (̵CH=CH)̵₂, (̵CH₂)̵₃ or (̵CH₂)̵₄ bridge in the 2,3- or 3,4-position; and
n is 0, 1, 2, 3 or 4, which comprises reacting an aniline of formula VI 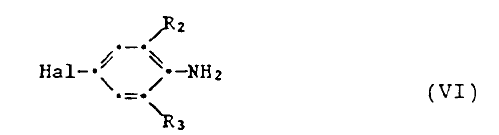 with a formanilide of formula VII 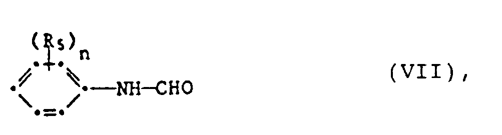 in which formulae R₂, R₃, R₅ and n are as defined forformula I and Hal is halogen, especially chlorine or bromine.

18. A process according to claim 17, wherein R₂ is C₁-C₅alkyl; R₃ is C₁-C₅alkyl or C₅-C₆cycloalkyl; R₄ is hydrogen, methyl or -CHO; R₅ is halogen, C₁-C₃alkyl or a (̵CH=CH)̵₂ or (̵CH₂)̵₃ bridge in the 3,4-position; and n is 0, 1 or 2.

19. A process according to claim 18, wherein R₂ and R₃ are each C₁-C₄alkyl, R₄ is hydrogen, methyl or -CHO, R₅ is methyl, and n is 0 or 1.

20. A process according to claim 17 for the preparation of a compound of formula 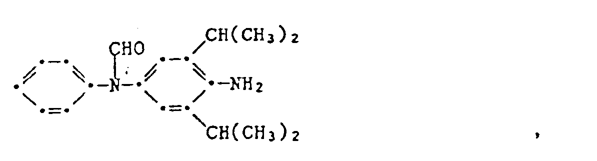 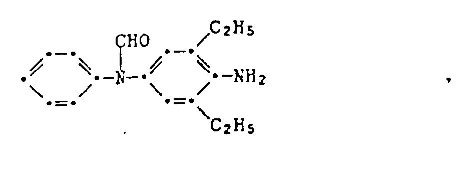 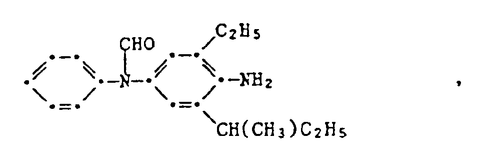 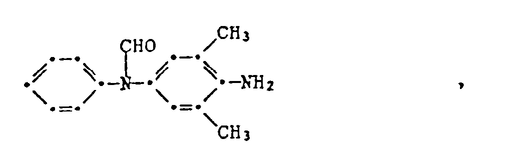 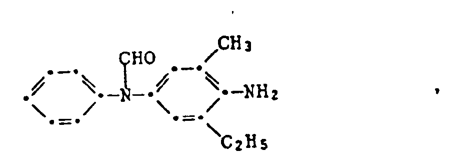 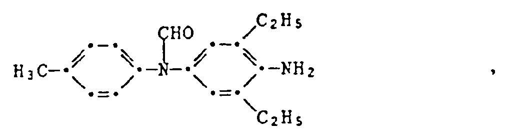 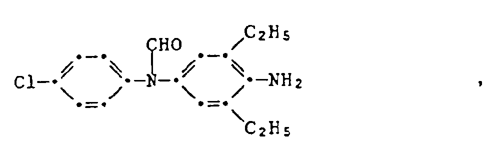 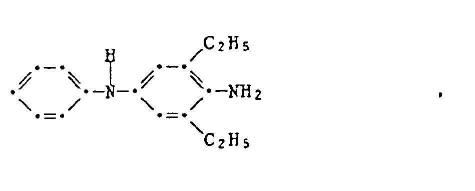 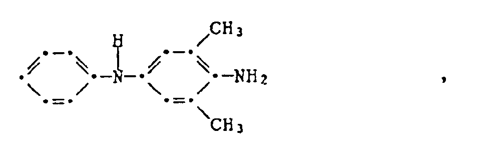 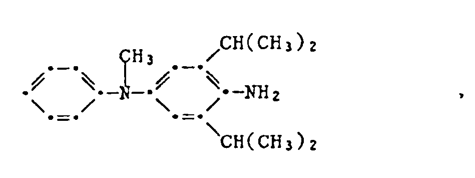 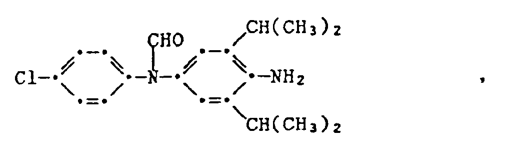 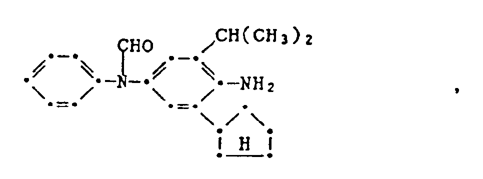 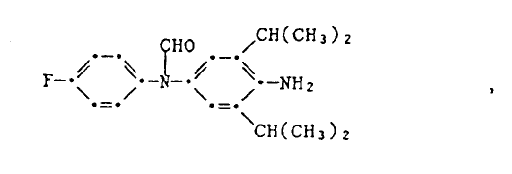 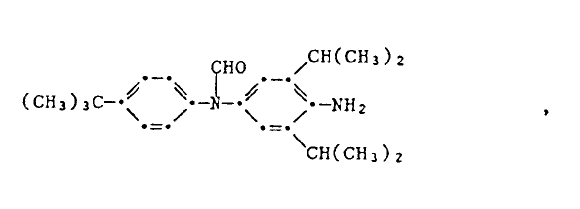 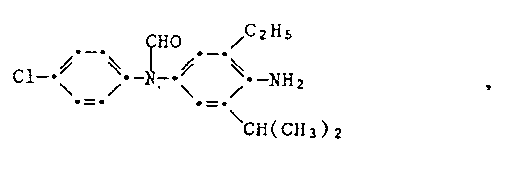 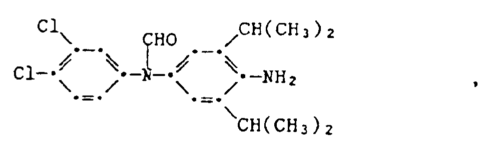 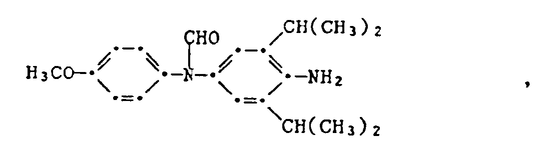 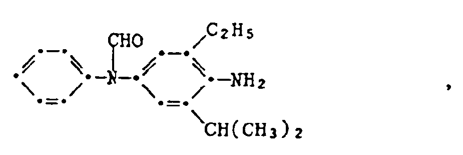 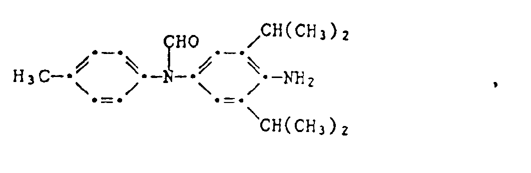 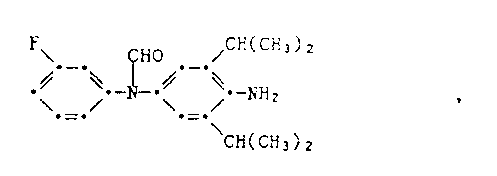 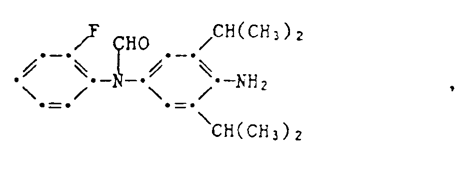 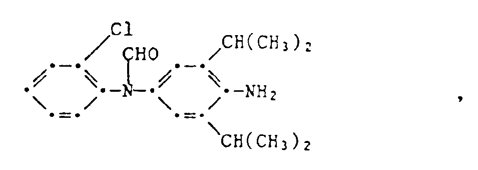 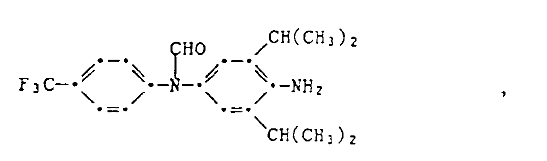 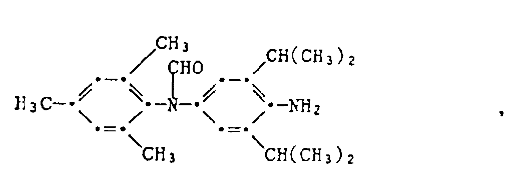 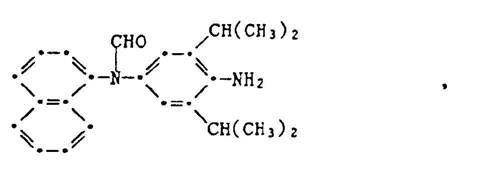 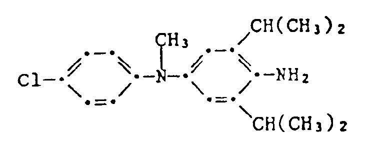 or

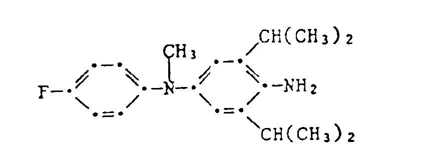

21. A process for the preparation of a compound of formula II 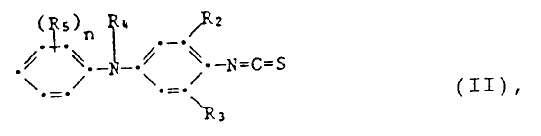 wherein
R₂ and R₃ are each C₁-C₅alkyl or C₅-Cₛcycloalkyl;
R₄ is hydrogen, methyl or -CHO;
R₅ is halogen; C₁-C₄-alkyl; C₁-C₄alkyl substituted by one or more halogen atoms; C₁-C₄alkoxy; or a (̵CH=CH)̵₂, (̵CH₂)̵₃ or (̵CH₂)̵₄ bridge in the 2,3- or 3,4-position; and
n is 0, 1, 2, 3 or 4, which comprises reacting an aminodiphenylamine of formula 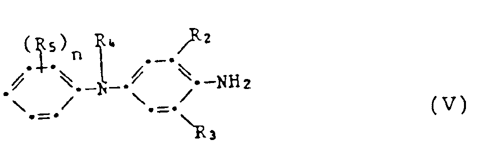 with thiophosgene, in which formula R₂, R₃, R₄, R₅ and n are as defined for formula I.

22. A process according to claim 21, wherein R₂ is C₁-C₅alkyl; R₃ is C₁-C₅alkyl or C₅-C₆cycloalkyl; R₄ is hydrogen, methyl or -CHO; R₅ is halogen, C₁-C₃alkyl or a (̵CH=CH)̵₂ or (̵CH₂)̵₃ bridge in the 3,4-position; and n is 0, 1 or 2.

23. A process according to claim 22, wherein R₂ and R₃ are each C₁-C₄alkyl; R₄ is hydrogen, methyl or -CHO; R₅ is methyl; and n is 0 or 1.

24. A process according to claim 21 for the preparation of a compound of formula 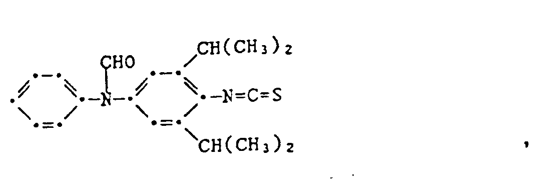 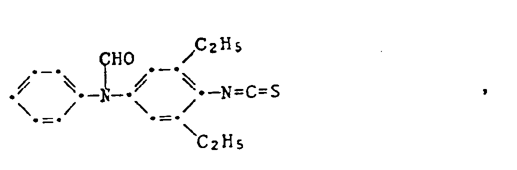 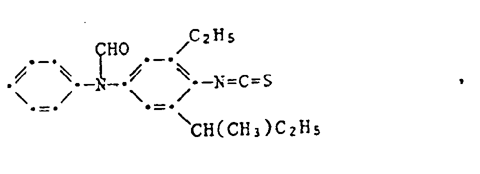 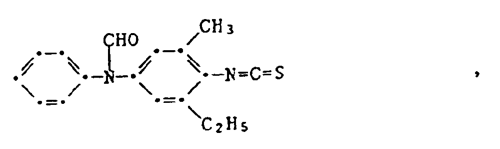 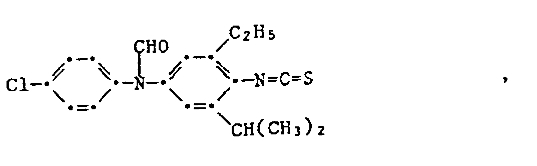 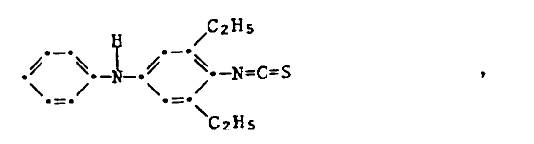 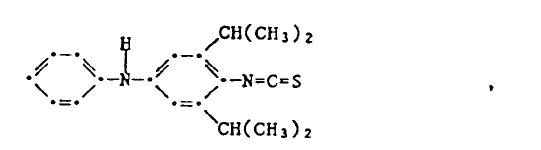 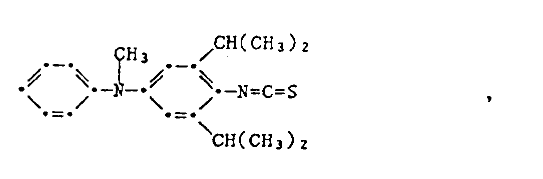 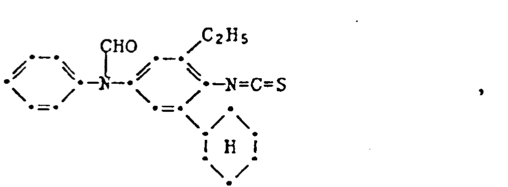 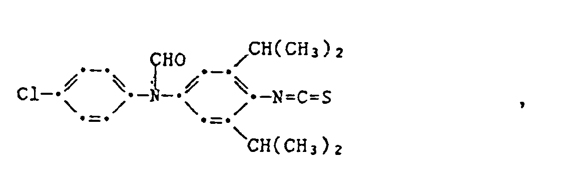 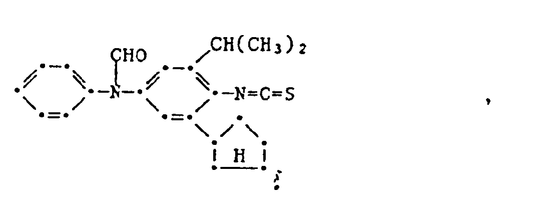 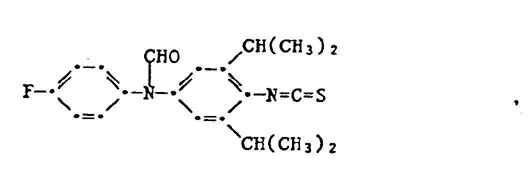 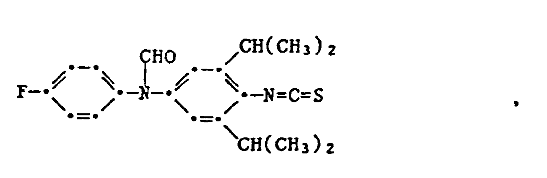 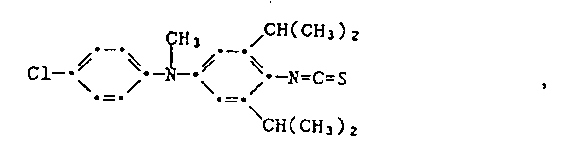 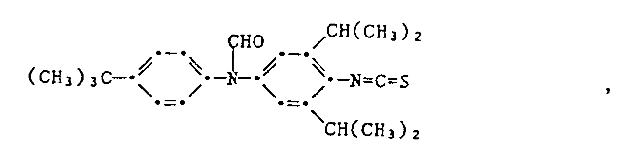 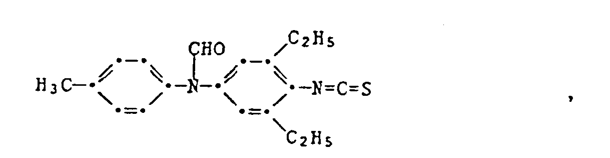 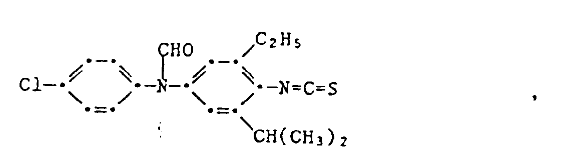 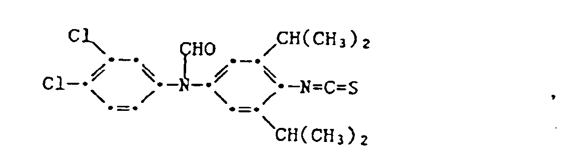 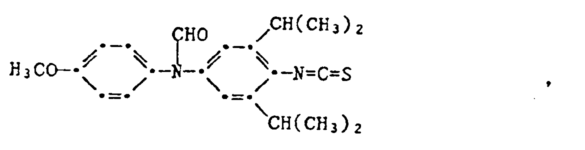 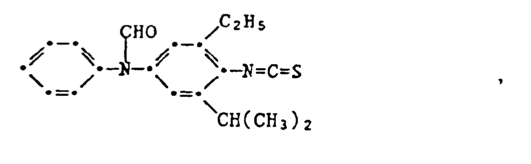 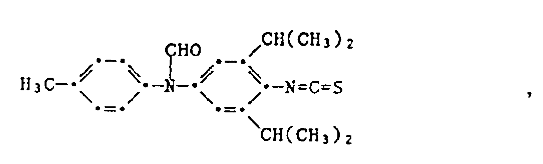 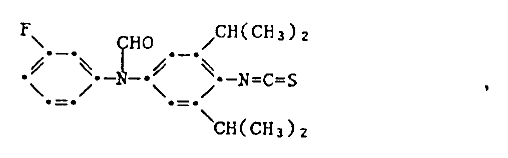 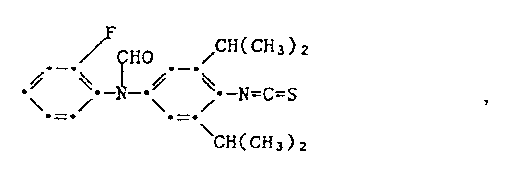 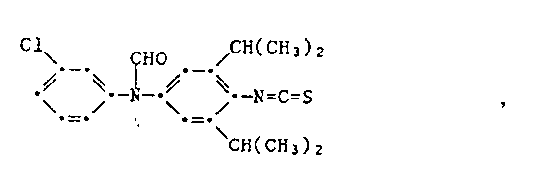 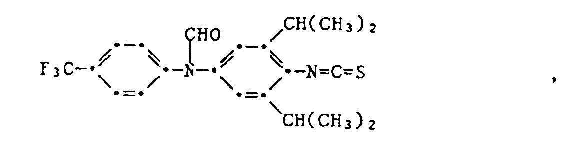 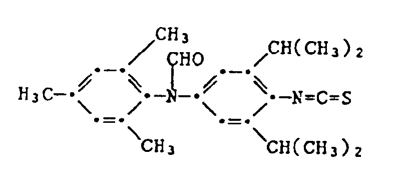 or

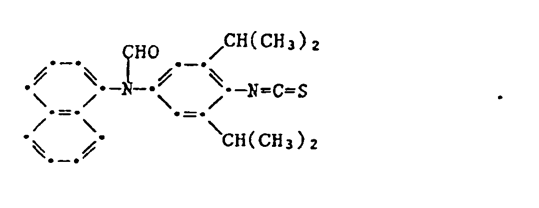

## Revendications (Revendications pour l'(les) Etat(s) contractant(s) suivant(s) : AT, BE, CH, DE, FR, GB,GR, IT, LI, NL)

1. Composés de formule I 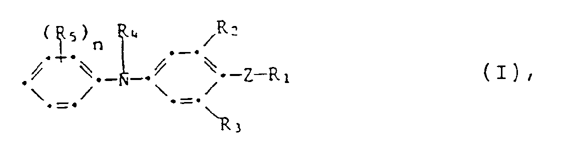 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C₁-C₄ ;
R₂ et R₃ représentent chacun un groupe alkyle en C1-C5 ou cycloalkyle en C5-C6 ;
R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, et leurs sels d'acides organiques ou minéraux.

2. Composés de formule 1 de la revendication 1, dans laquelle R₁ représente un groupe alkyle en C1-C8, un groupe alkyle en C1-C8 portant un ou plusieurs substituants halogéno et/ou alcoxy en C1-C5, ou un groupe cycloalkyle en C3-C8 ; R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4 ; n est égal à 0, 1 ou 2 ; Z représente -NH-CS-NH-, -N=C(SR₆)-NH- ou -N=C=N- ; et R₆ représente un groupe alkyle en C1-C4.

3. Composés de formule 1 de la revendication 2, dans laquelle R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -NH-CS-NH-.

4. Composés de formule I de la revendication 2, dans laquelle R₁ représente un groupe alkyle en C1-C5 ou cyclopentyle ; R₂ et R₃ représentent chacun un groupe alkyle en C₁-C₄ ; R₄ représente l'hydrogène, un groupe méthyle ou -CHO ; R₅ représente un groupe méthyle ; n est égal à 0 ou 1 ; Z représente -N=C(SRₛ)-NH- ; et R₆ représente un groupe alkyle en C1-C4.

5. Composés de formule I de la revendication 2, dans laquelle R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -N=C=N-.

6. Les composés selon l'une des revendications 1 à 3, de formules

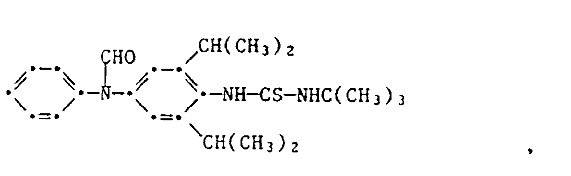

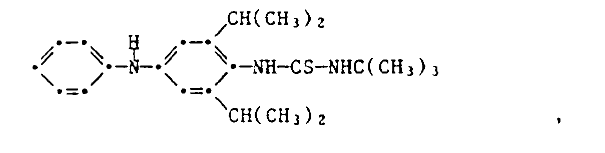

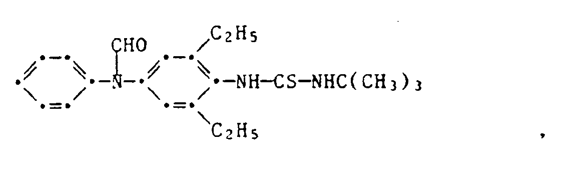

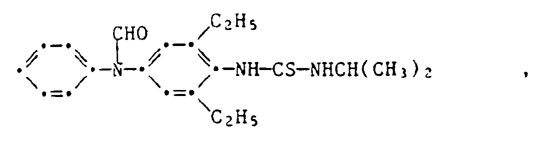

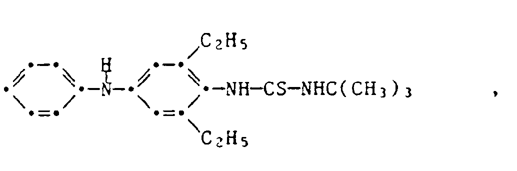

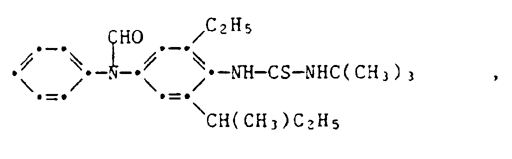

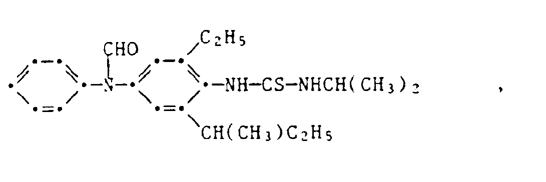

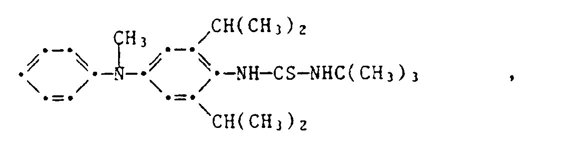

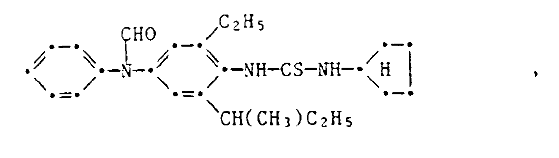

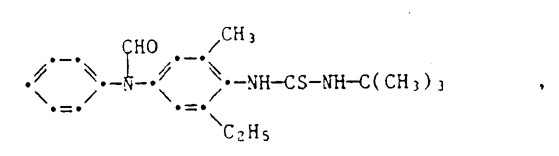

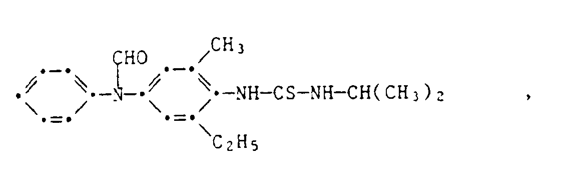

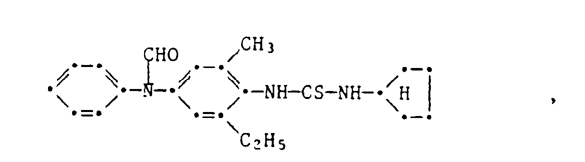

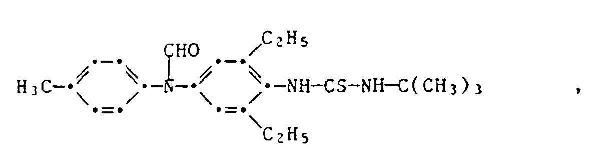

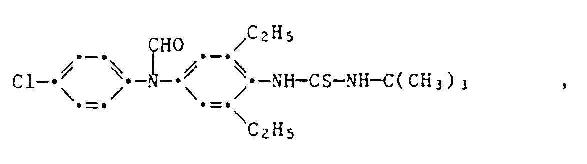

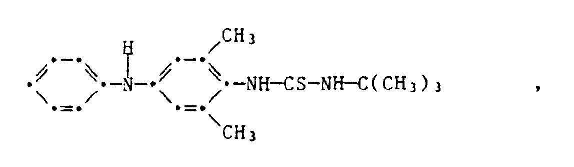

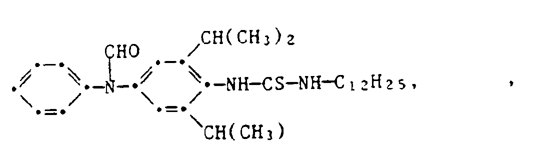

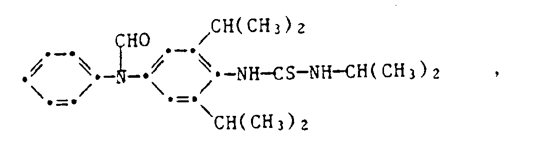

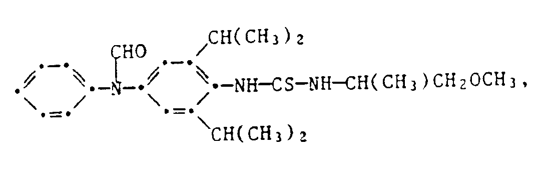

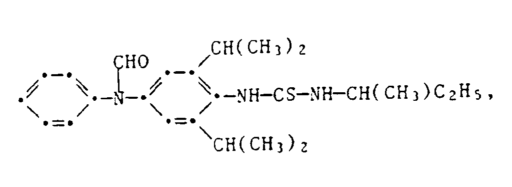

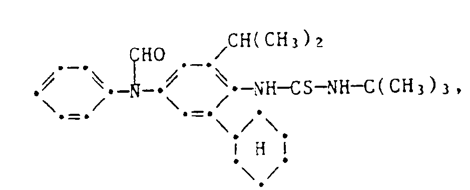

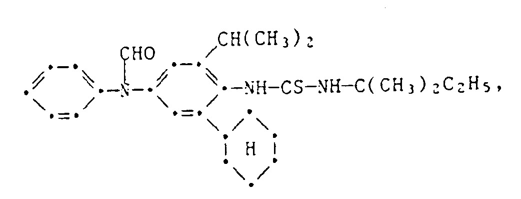

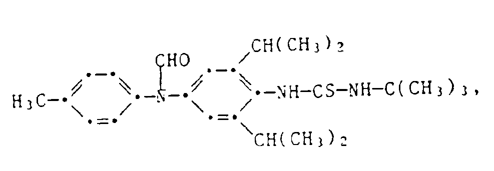

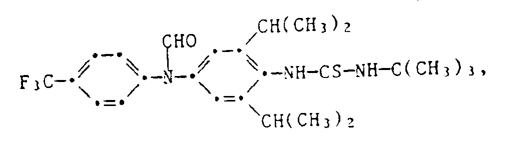

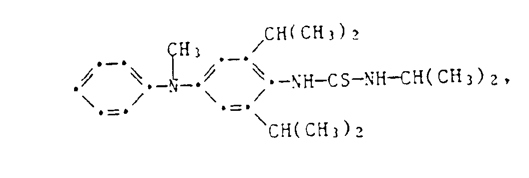

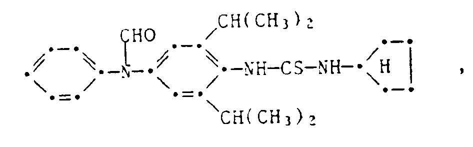

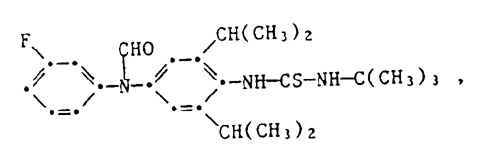

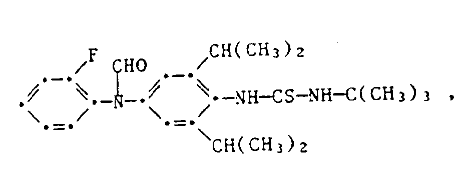

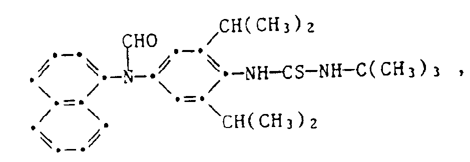

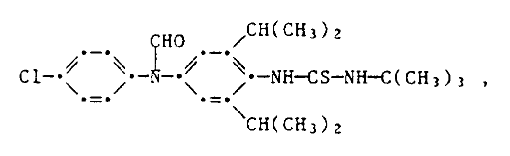

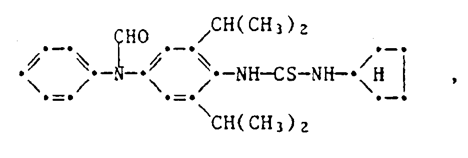

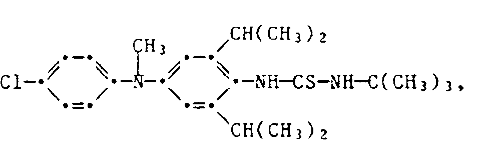

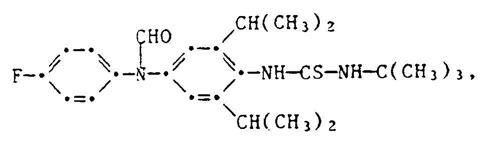

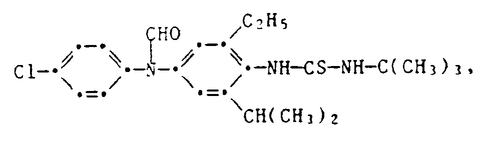

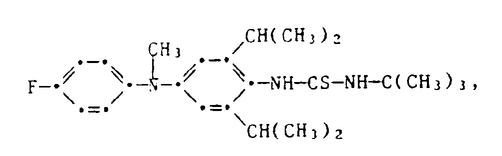

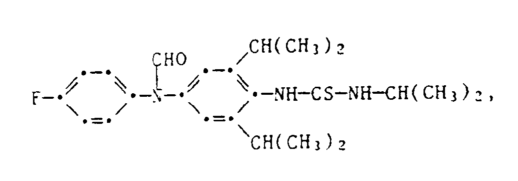

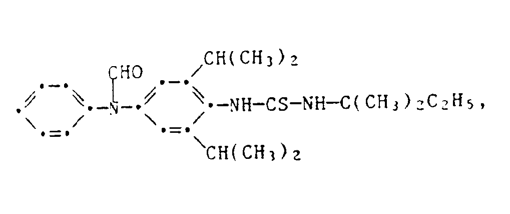

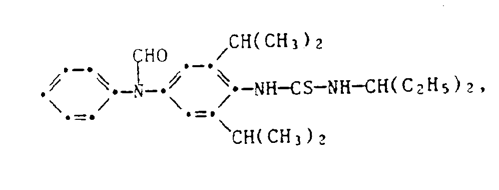

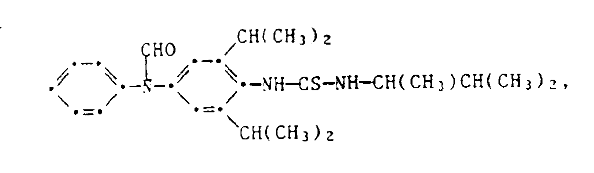

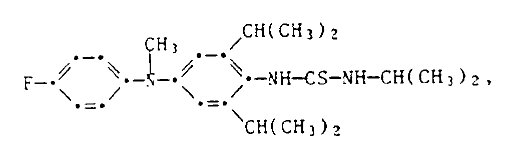

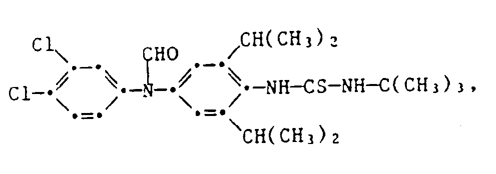

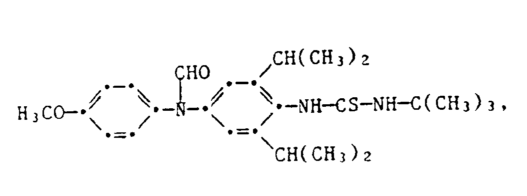

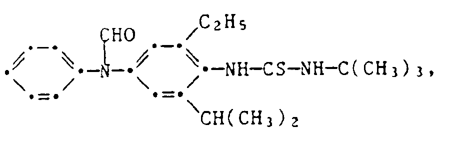

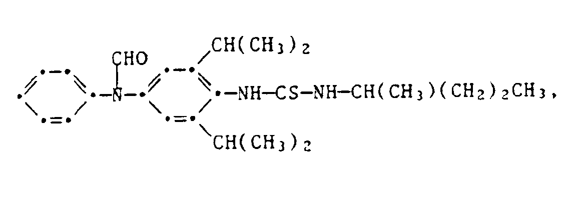

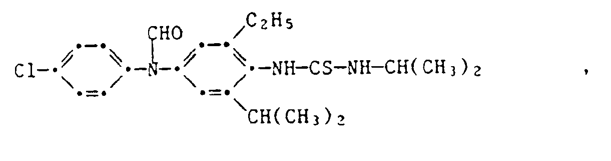

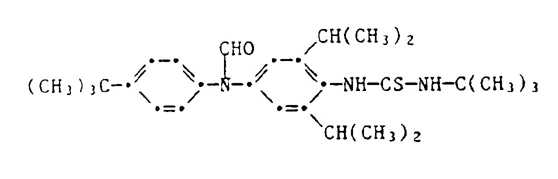

et

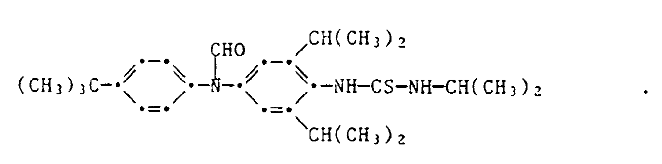

7. Les composés selon l'une des revendications 1, 2 ou 4, de formules 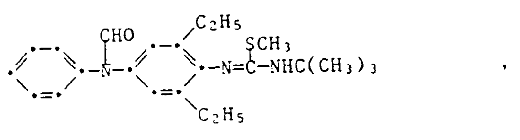 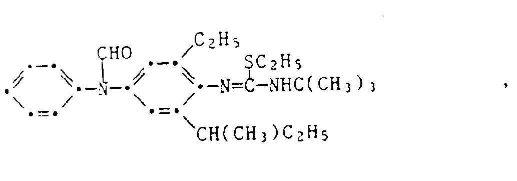 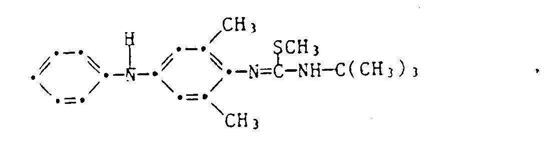 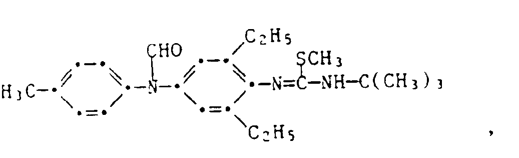 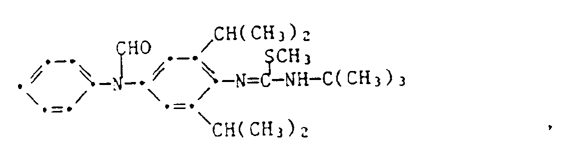 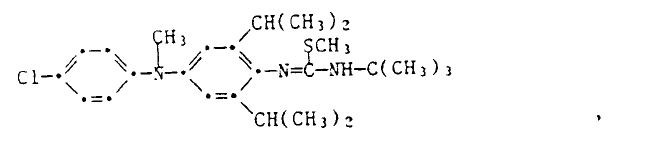 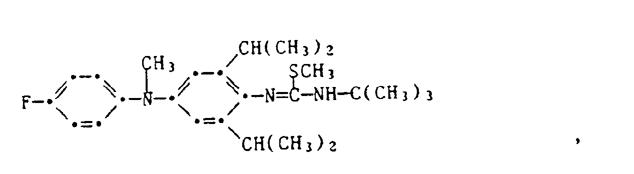 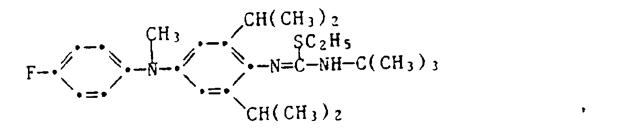 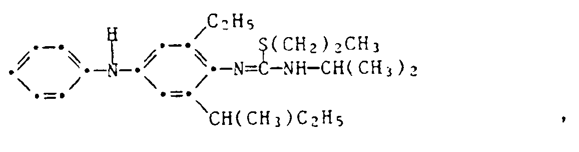 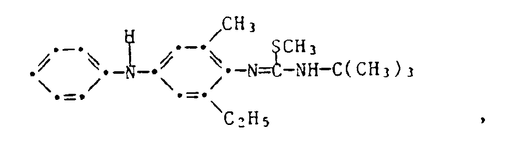 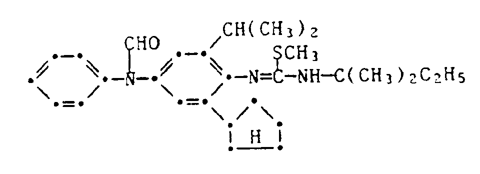 et

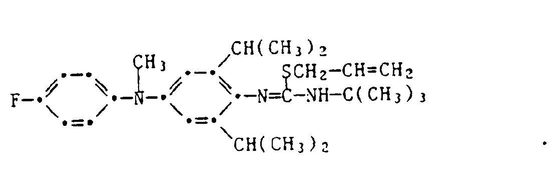

8. Les composés selon l'une des revendications 1, 2 ou 5, de formules 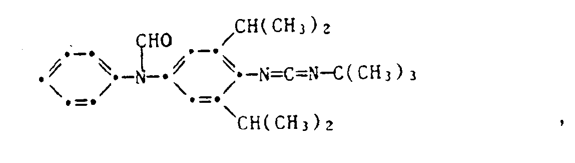 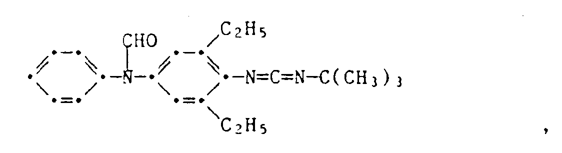 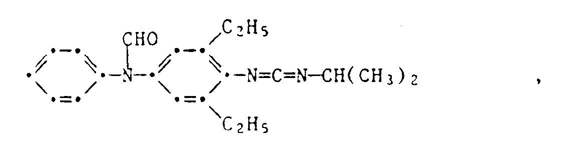 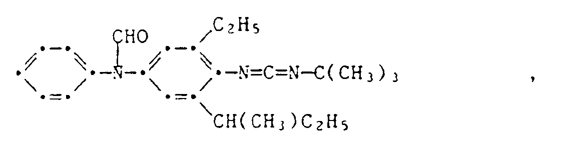 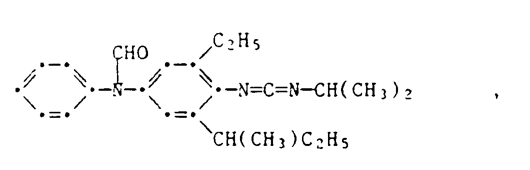 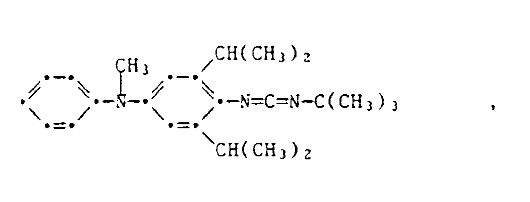 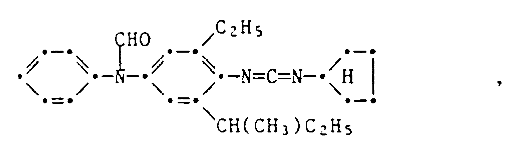 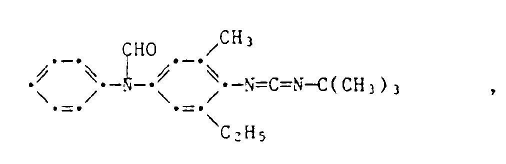 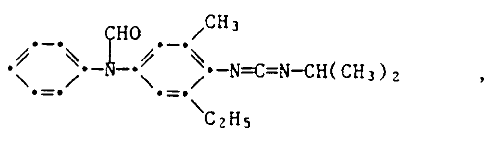 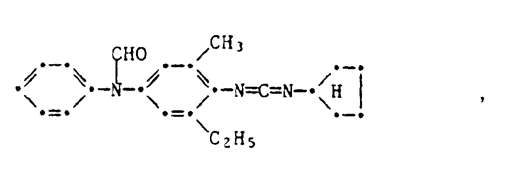 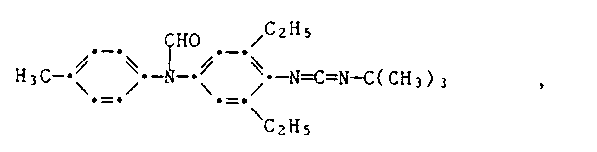 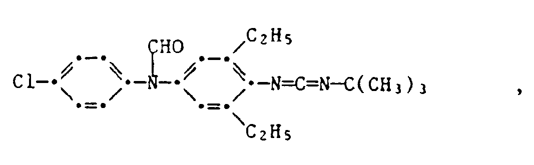 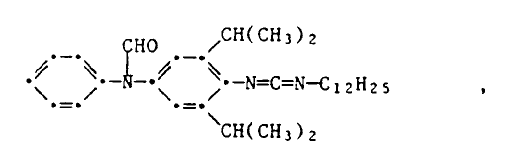 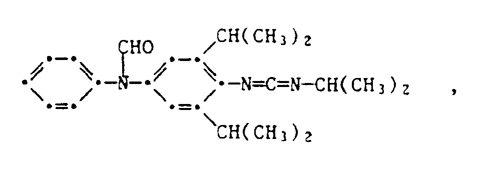 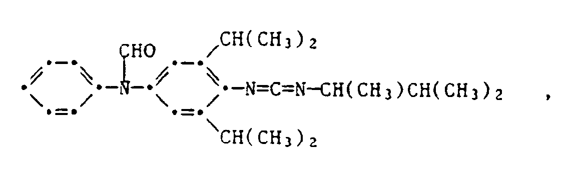 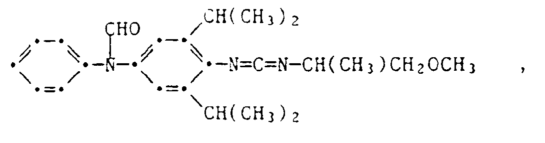 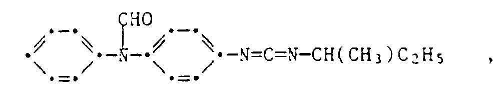 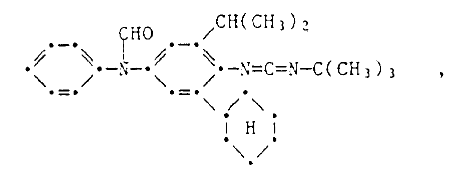 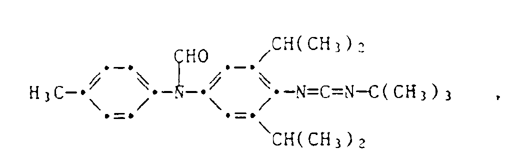 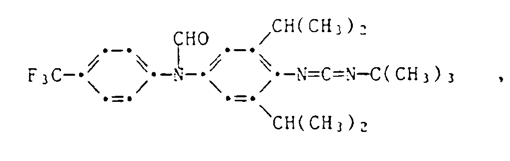 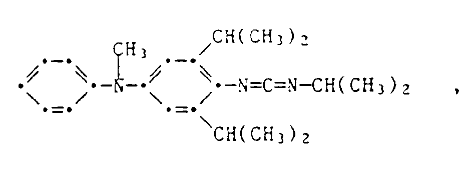 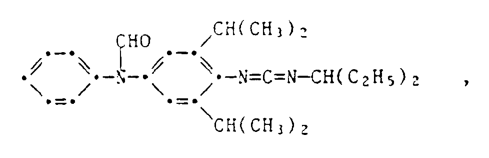 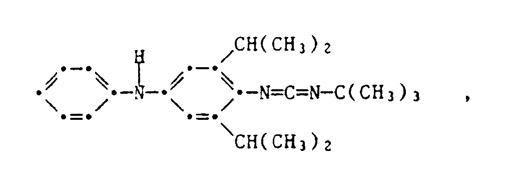 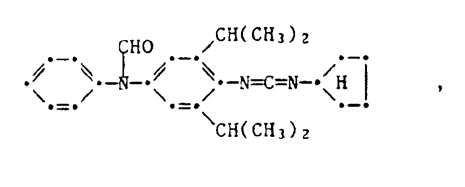 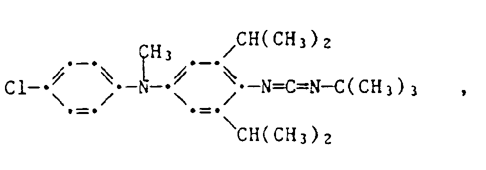 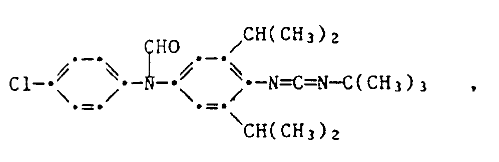 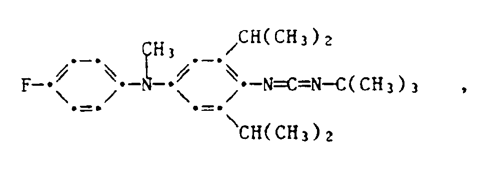 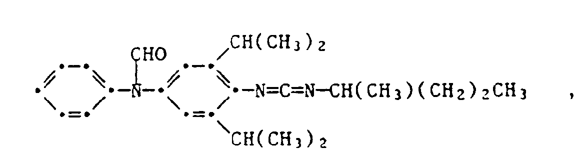 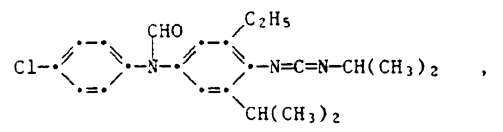 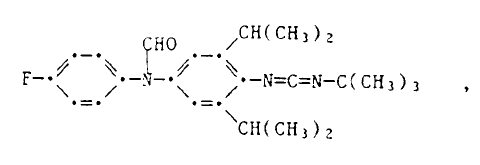 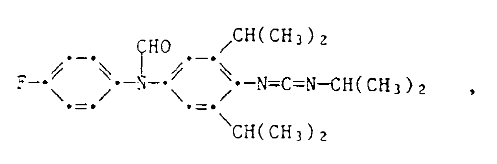 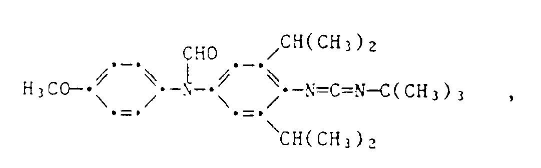 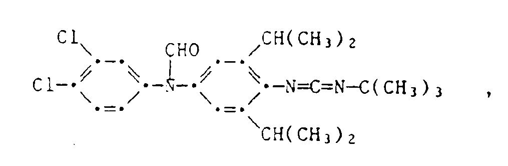 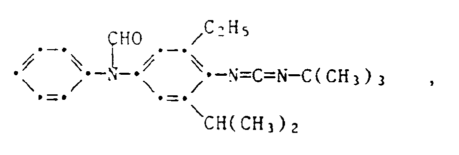 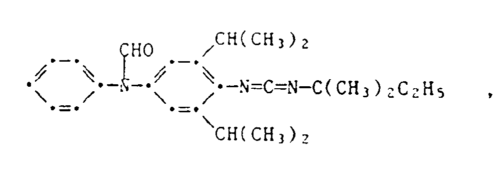 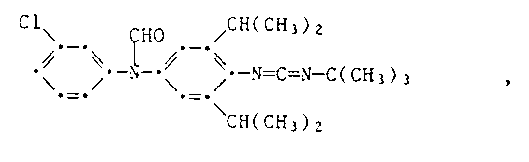 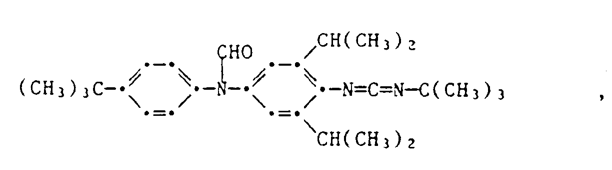 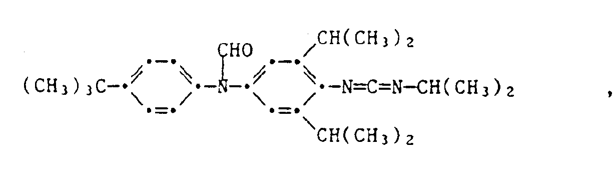 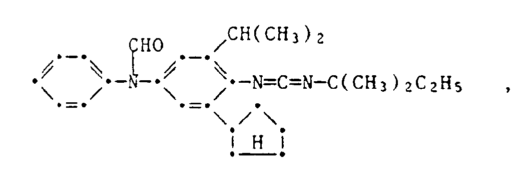 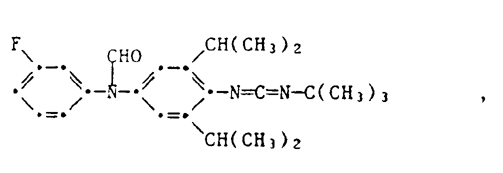 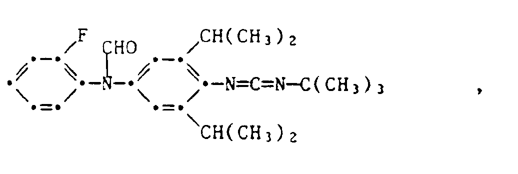 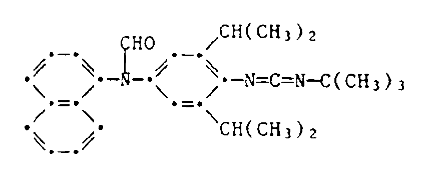 et

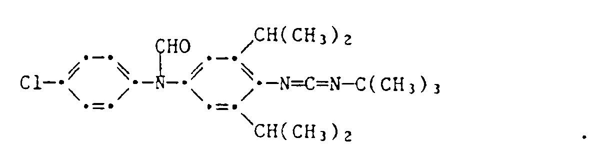

9. Procédé de préparation d'un composé de formule 1 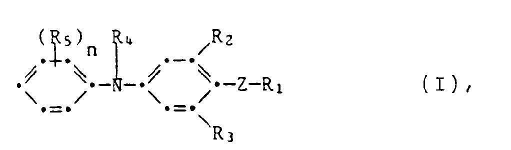 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH )̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, caractérisé en ce que
A) on fait réagir un isothiocyanate de formule II 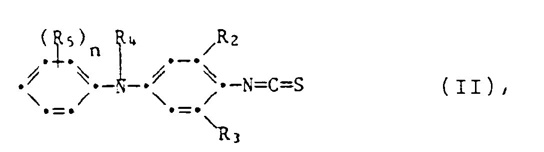 avec une amine de formule III H₂N-R₁ (III) dans un solvant ou diluant organique à pression normale et à une température de 0 à +150_{°}C, la réaction donnant une thiourée, et le cas échéant
B) on fait réagir cette thiourée avec un composé de formule IV X-Rₛ (IV) dans un solvant organique inerte à pression normale ou légèrement supérieure à la normale et à une température de +10 à +250_{°}C, la réaction donnant l'isothiourée, ou bien
C) on convertit la thiourée, par scission de sulfure d'hydrogène, dans un solvant ou diluant organique apro- tonique, à pression normale et à une température de 0 à +150°C, en le carbodiimide, R₁, R₂, R₃, R₄, R₅, R₆ et n ayant les significations indiquées ci-dessus et X représentant un substituant éliminable.

10. Produit pesticide contenant au moins un composant actif consistant en un composé de formule 1 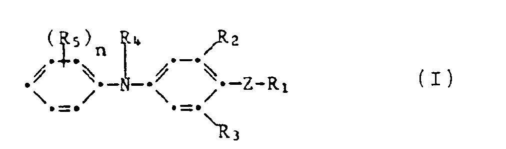 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄, en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou en l'un de ses sels d'acide organique ou minéral, avec des véhicules et/ou additifs appropriés.

11. Produit pesticide selon revendication 10, contenant au moins un composant actif consistant en un composé de formule I dans laquelle R₁ représente un groupe alkyle en C1-C8, un groupe alkyle en C1-C8 portant un ou plusieurs substituants halogéno et/ou alcoxy en C1-C5, ou un groupe cycloalkyle en C3-C8 ; R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH )̵₂ ou (̵CH₂)̵₃ en position 3,4; n est égal à 0, 1 ou 2 ; Z représente -NH-CS-NH-, -N=C(SR₆)-NH- ou -N=C=N- ; et R₆ représente un groupe alkyle en C1-C4.

12. Produit pesticide selon revendication 11, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -NH-CS-NH-.

13. Produit pesticide selon revendication 11, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; Z représente -N=C(SR₆)-NH- ; et R₆ représente un groupe alkyle en C1-C4.

14. Produit pesticide selon revendication 11, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -N=C=N-.

15. Utilisation d'un composé de formule 1 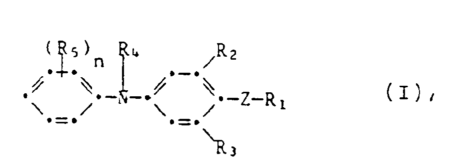 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou l'un de ses sels d'acide organique ou minéral, pour la lutte contre les parasites des animaux et des végétaux.

16. Utilisation selon la revendication 15 pour la lutte contre les insectes et les arachnides.

17. Procédé pour combattre les parasites des animaux et des végétaux, caractérisé en ce que l'on met les parasites, dans leurs divers stades de développement, en contact avec un composé de formule 1 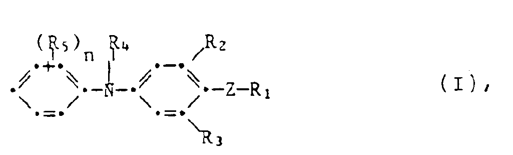 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou l'un de ses sels d'acide organique ou minéral.

18. Composé de formule V 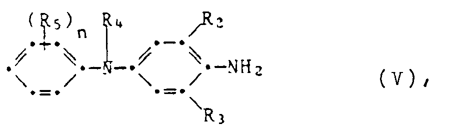 dans laquelle
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4 ou un pont (̵CH=CH)̵ ₂, (̵CH₂ )̵₃ ou (̵CH₂)̵₄ en position 2,3 ou 3,4 et
n est égal à 0, 1, 2, 3 ou 4.

19. Composés de formule V de la revendication 18 dans laquelle R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4 ; et n est égal à 0, 1 ou 2.

20. Composés de formule V de la revendication 19 dans laquelle R₂ et R₃ représentent chacun un groupe alkyle en C1-C4; R₄ représente l'hydrogène, un groupe méthyle ou -CHO ; R₅ représente un groupe méthyle ; et n est égal à 0 ou 1.

21. Les composés selon revendication 18, de formules

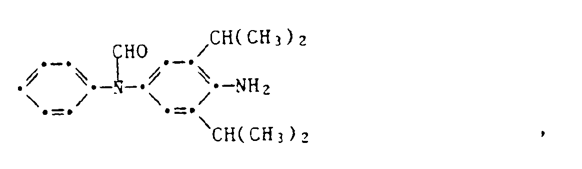

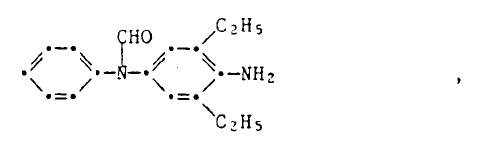

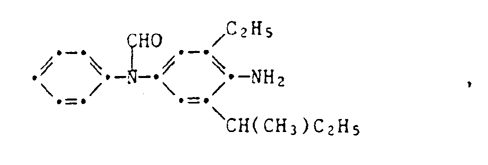

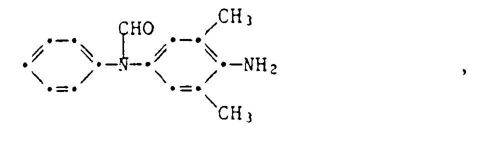

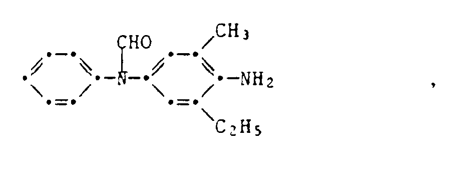

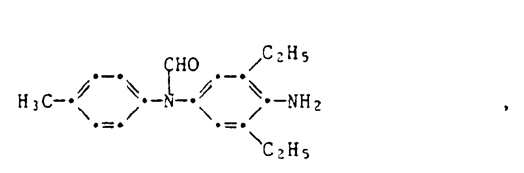

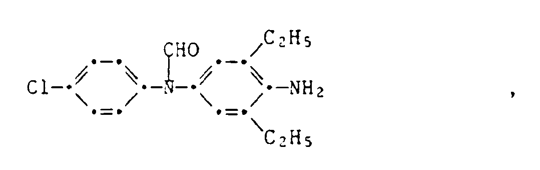

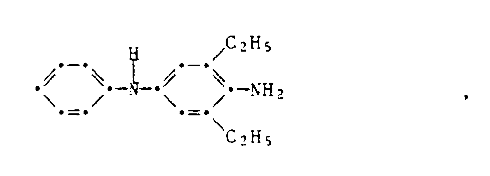

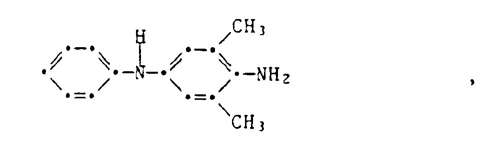

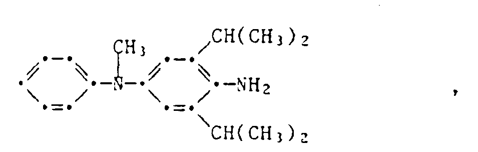

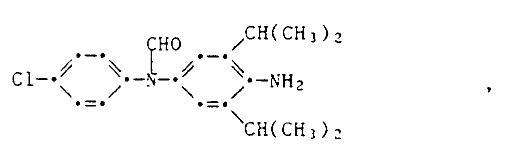

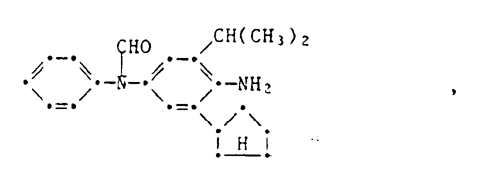

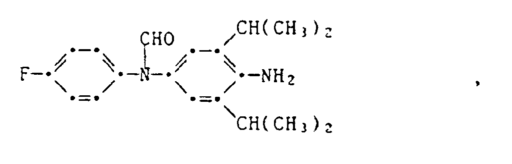

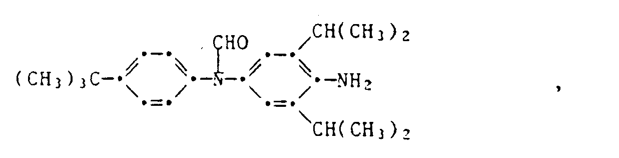

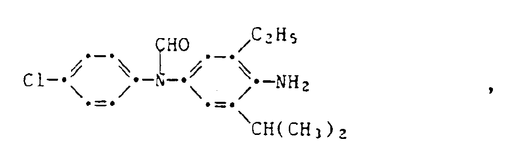

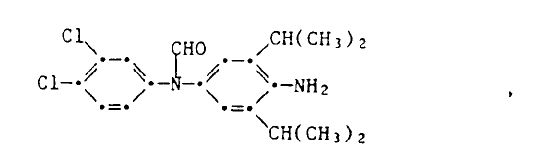

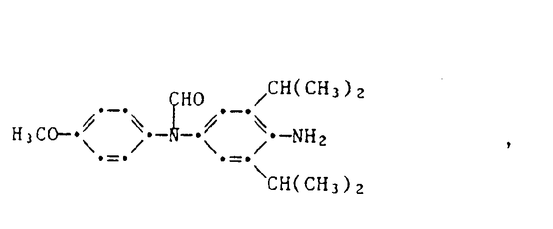

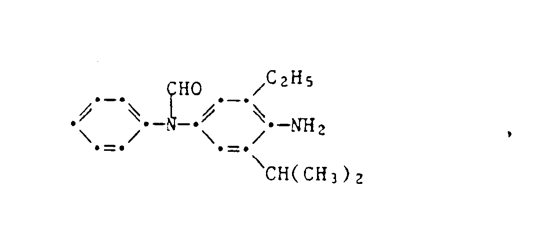

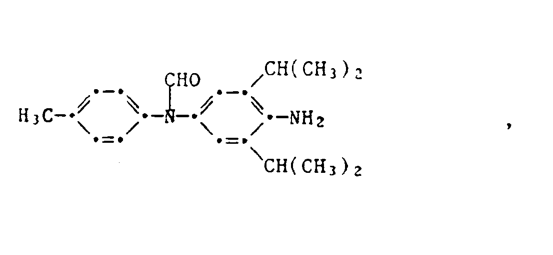

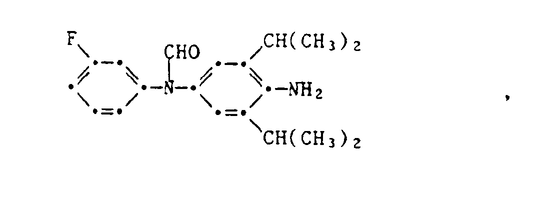

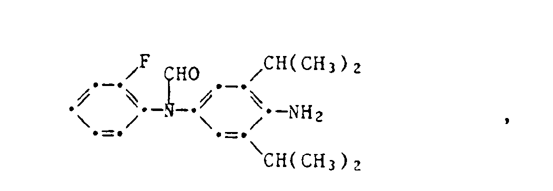

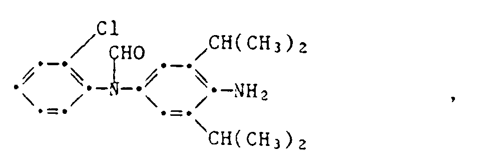

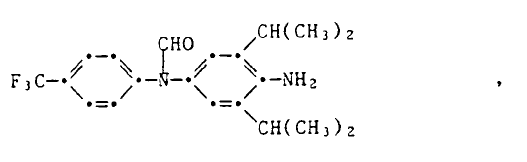

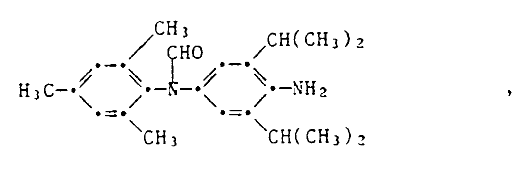

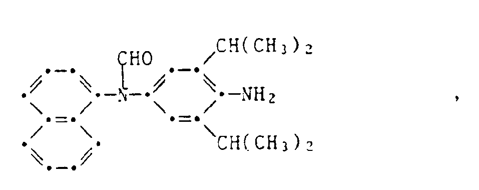

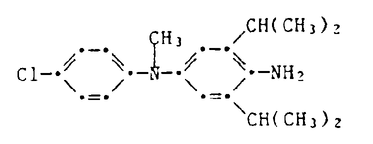

et

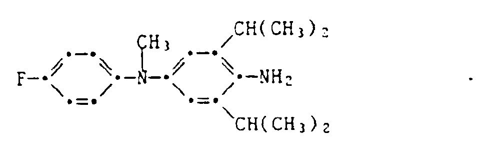

22. Composés de formule II 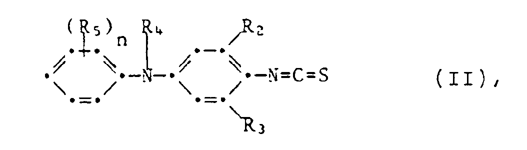 dans laquelle
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4 ou un pont (̵CH=CH)̵₂, (̵CH₂)̵₃ ou (̵CH₂)̵₄ en position 2,3 ou 3,4 et
n est égal à 0, 1, 2, 3 ou 4.

23. Composés de formule Il de la revendication 22 dans laquelle R₂ représente un groupe alkyle en C1-C5 ; R₃ représente un groupe alkyle en C1-C5 ou cycloalkyle en C5-C6 ; R₆ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4 ; et n est égal à 0, 1 ou 2.

24. Composés de formule II de la revendication 23, dans laquelle R₂ et R₃ représentent chacun un groupe alkyle en C1-C4 ; R₄ représente l'hydrogène, un groupe méthyle ou -CHO ; R₅ représente un groupe méthyle ; et n est égal à 0 ou 1.

25. Les composés selon revendication 22, de formules

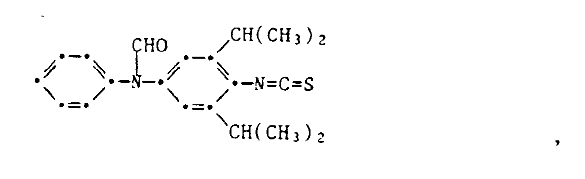

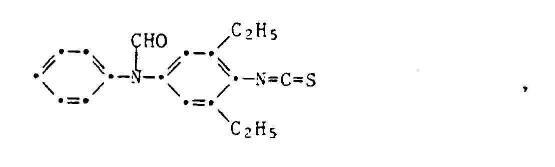

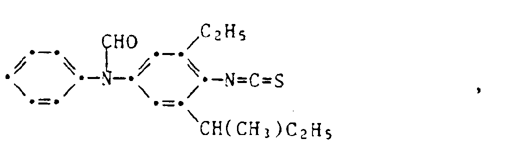

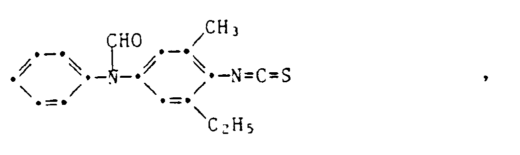

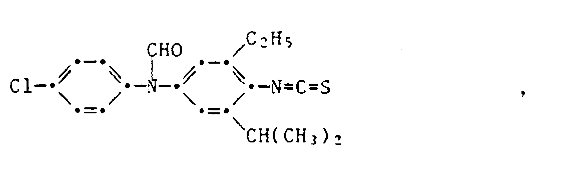

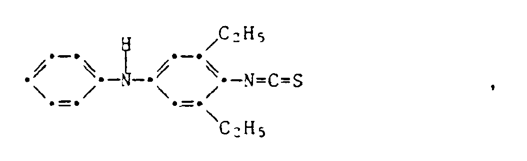

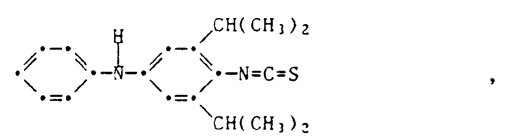

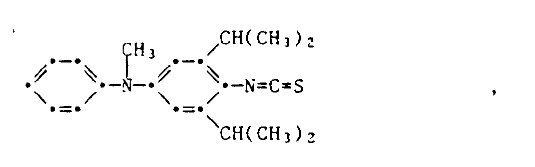

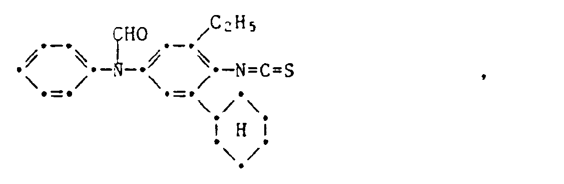

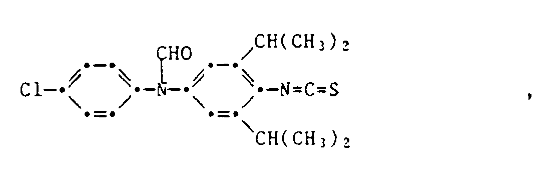

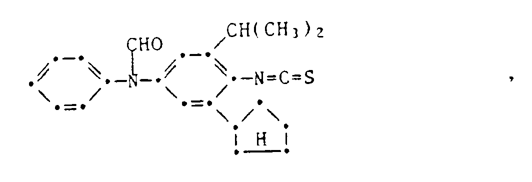

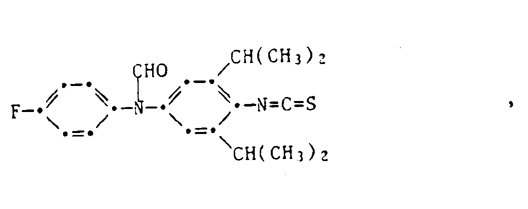

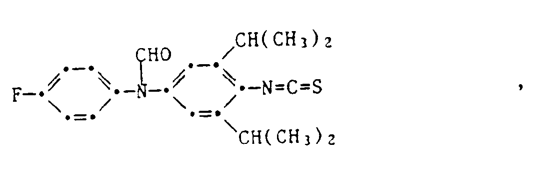

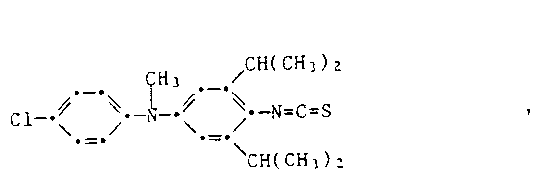

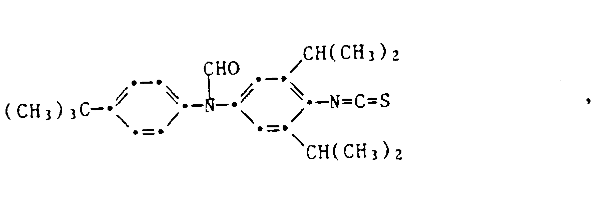

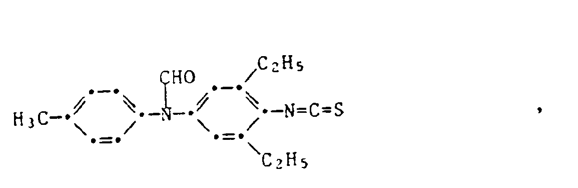

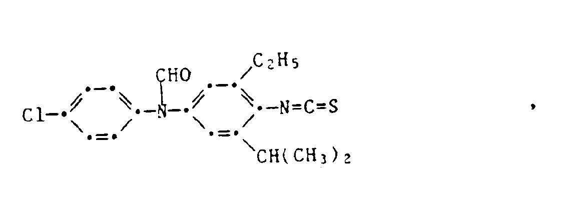

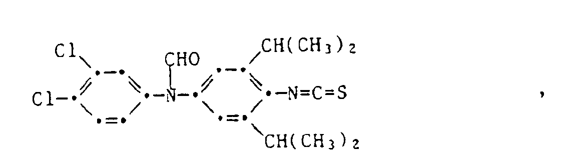

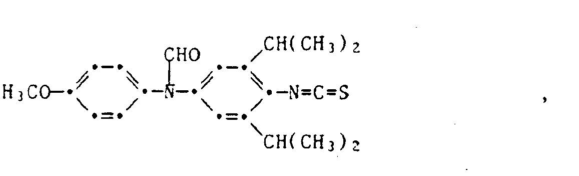

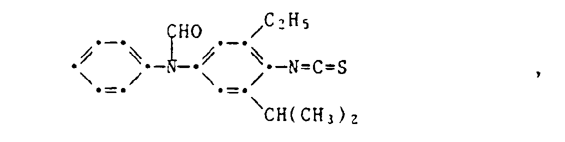

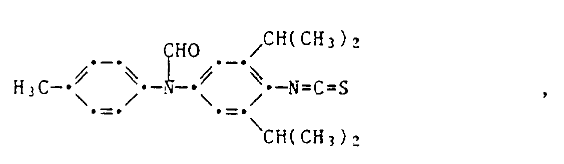

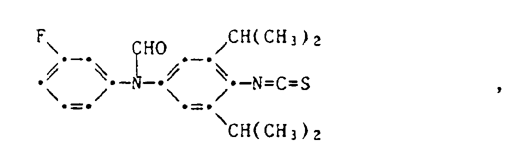

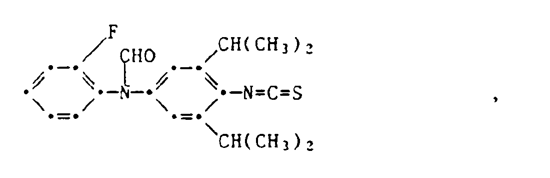

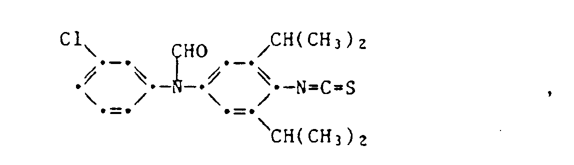

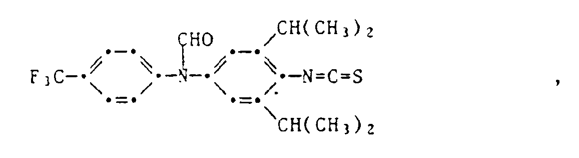

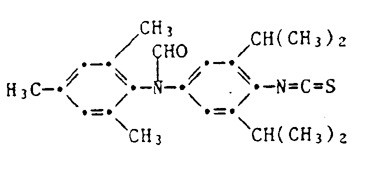

et

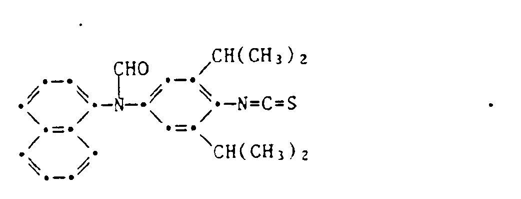

## Revendications (Revendications pour l'(les) Etat(s) contractant(s) suivant(s) : ES)

1. Procédé de préparation d'un composé de formule 1 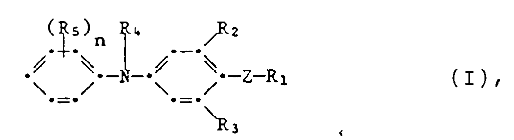 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4,
Z représente -NH-CS-NH-, -N=C(SR₆)-NH- ou -N=C=N- et R₆ représente un groupe alkyle en C1-C6 ou allyle, caractérisé en ce que
A) on fait réagir un isothiocyanate de formule II 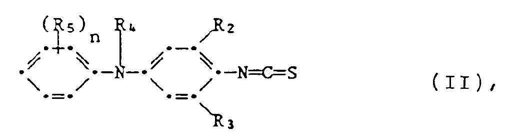 avec une amine de formule III 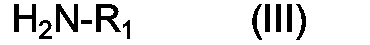 dans un solvant ou diluant organique à pression normale et à une température de 0 à +150_{°}C, la réaction donnant une thiourée, et le cas échéant
B) on fait réagir cette thiourée avec un composé de formule IV 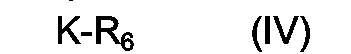 dans un solvant organique inerte à pression normale ou légèrement supérieure à la normale et à une température de +10 à +250_{°}C, la réaction donnant l'isothiourée, ou bien
C) on convertit la thiourée, par scission de sulfure d'hydrogène, dans un solvant ou diluant organique apro- tonique, à pression normale et à une température de 0 à +150°C, en le carbodiimide, R₁, R₂, R₃, R₄, R₅, R₆ et n ayant les significations indiquées ci-dessus et K représentant un substituant éliminable.

2. Procédé selon la revendication 1, dans lequel R₁ représente un groupe alkyle en C1-C8, un groupe alkyle en C1-C8 portant un ou plusieurs substituants halogéno et/ou alcoxy en C1-C5, ou un groupe cycloalkyle en C3-C8 ; R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH )̵₂ ou (̵CH₂)̵₃ en position 3,4 ; n est égal à 0, 1 ou 2 ; Z représente -NH-CS-NH-, -N=C(SR₆)-NH- ou -N=C=N- ; et R₆ représente un groupe alkyle en C1-C4.

3. Procédé selon la revendication 2, dans lequel R₁ représente un groupe alkyle en C1-C5 ou cyclopentyle; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -NH-CS-NH-.

4. Procédé selon la revendication 2, dans lequel R₁ représente un groupe alkyle en C1-C5 ou cyclopentyle; R₂ et R₃ représentent chacun un groupe alkyle en C1-C4 ; R₄ représente l'hydrogène, un groupe méthyle ou -CHO ; R₅ représente un groupe méthyle , n est égal à 0 ou 1 ; Z représente -N=C(SRₛ)-NH- ; et R₆ représente un groupe alkyle en C1-C4.

5. Procédé selon la revendication 2, dans lequel R₁ représente un groupe alkyle en C1-C5 ou cyclopentyle; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -N=C=N-.

6. Procédé selon l'une des revendications 1 à 3, pour la préparation des composés de formules 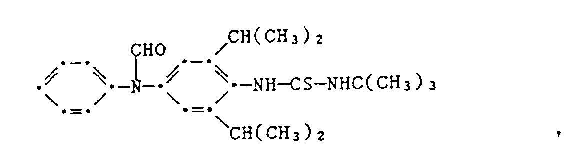 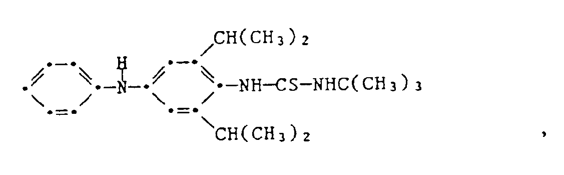 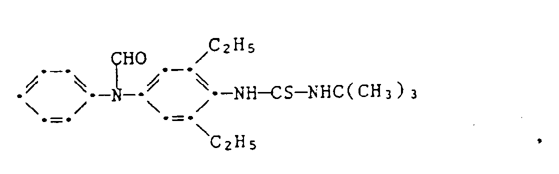 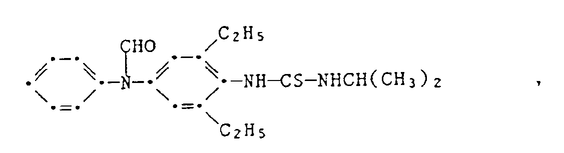 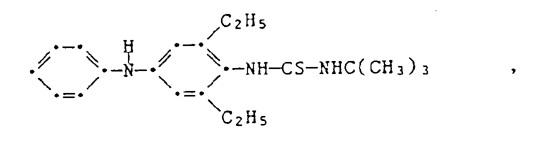 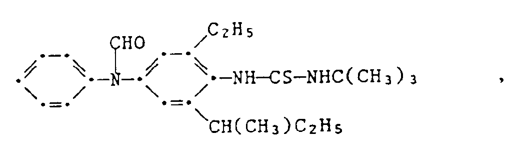 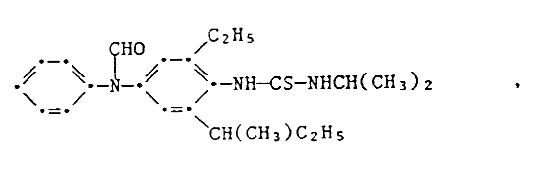 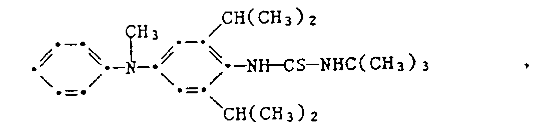 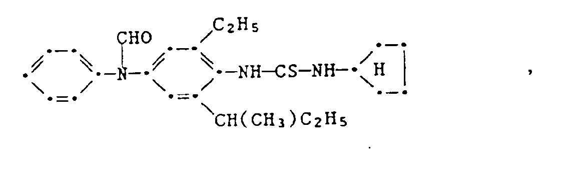 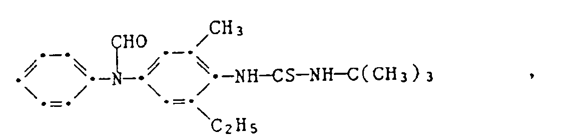 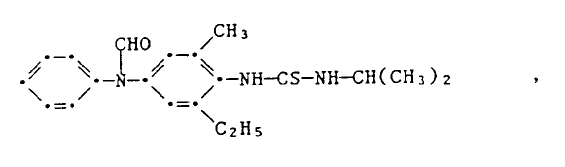 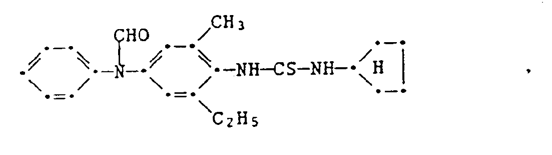 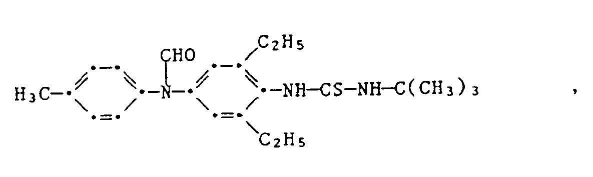 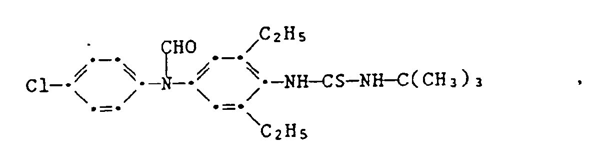 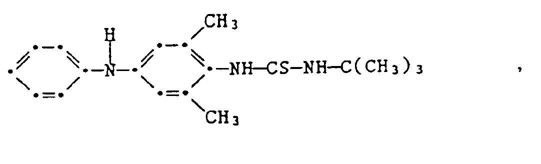 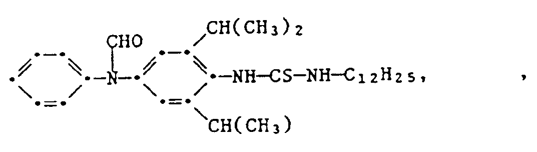 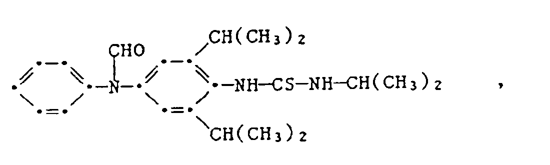 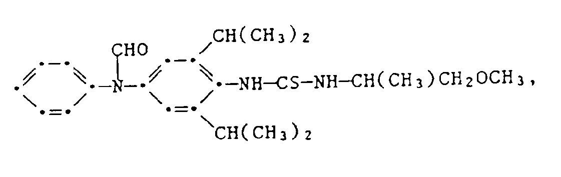 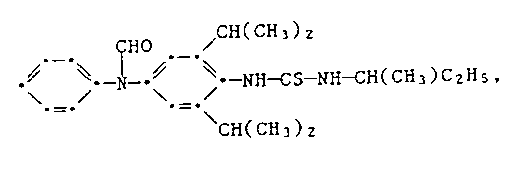 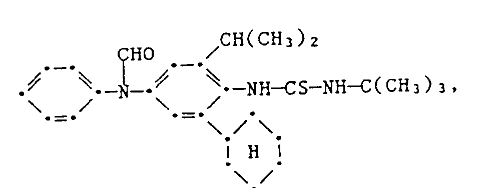 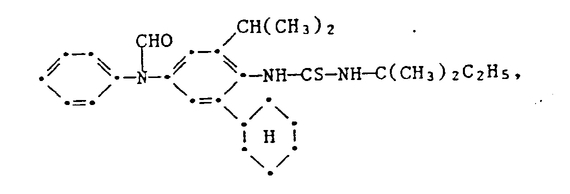 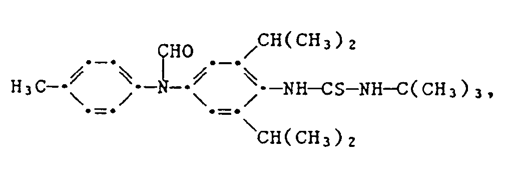 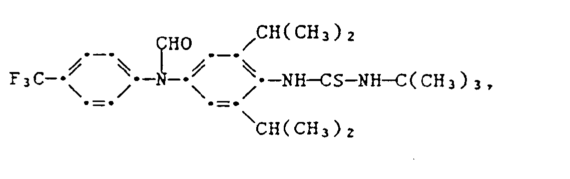 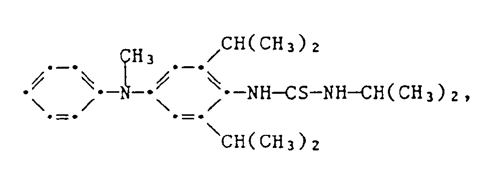 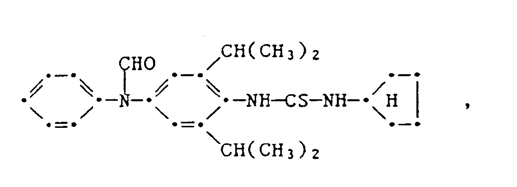 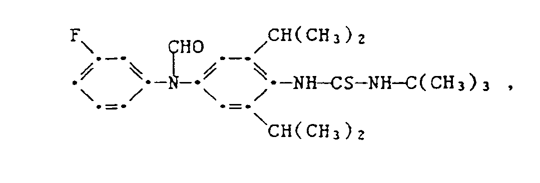 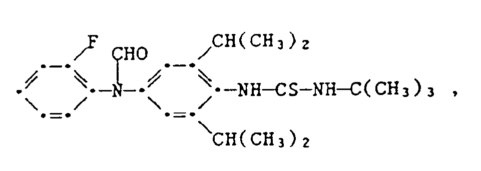 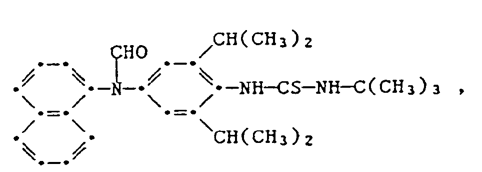 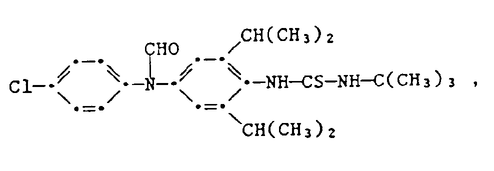 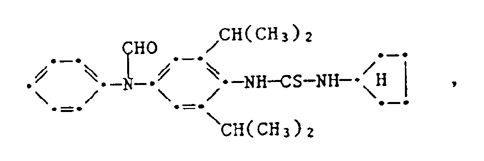 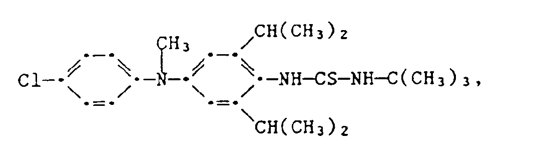 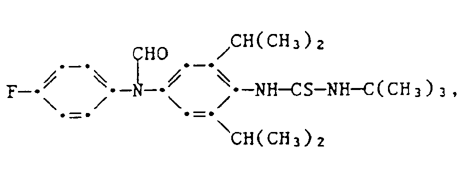 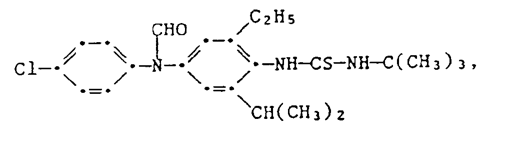 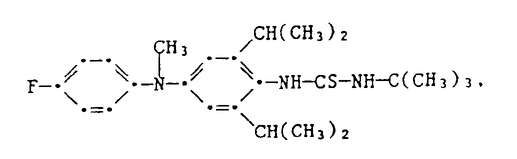 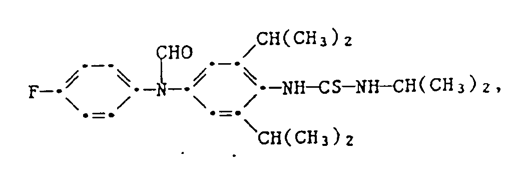 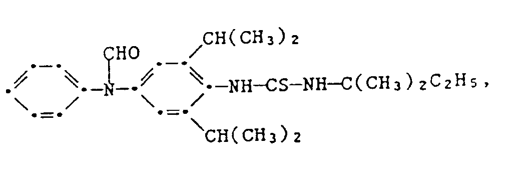 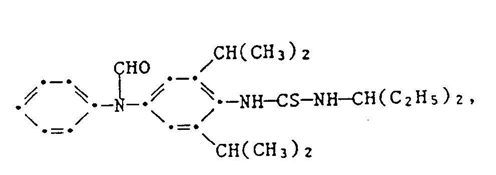 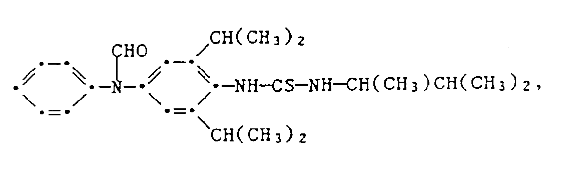 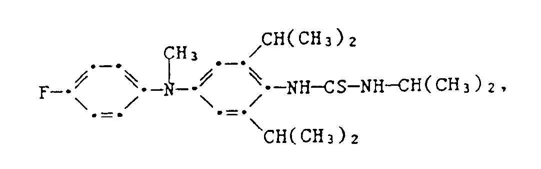 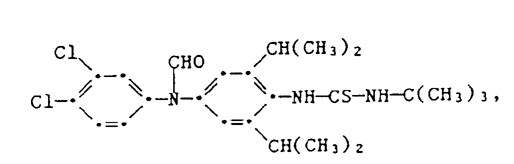 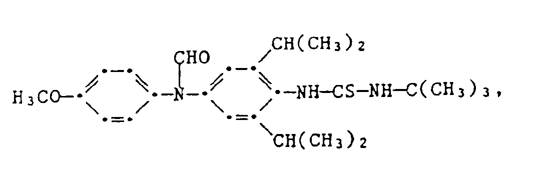 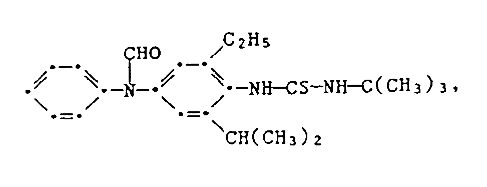 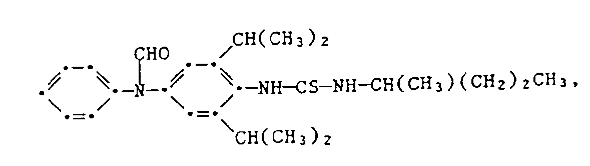 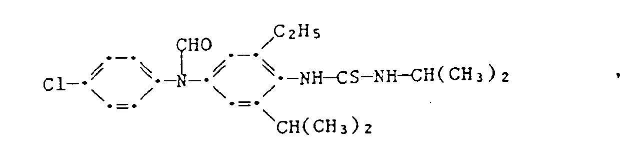 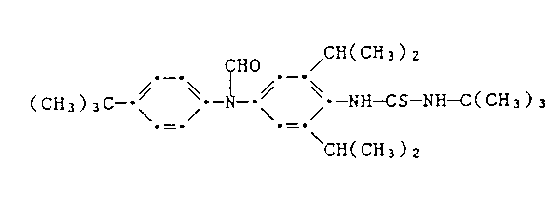 et

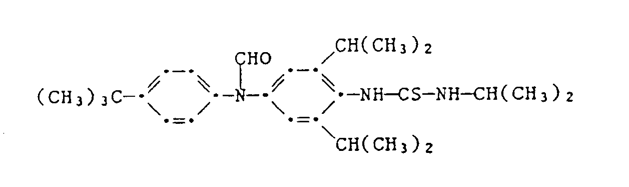

7. Procédé selon l'une des revendications 1, 2 ou 4 pour la préparation des composés de formule 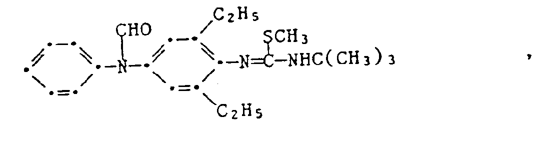 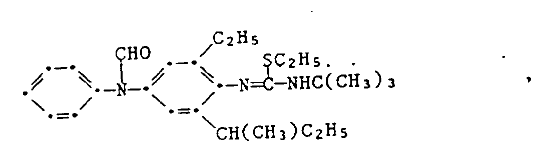 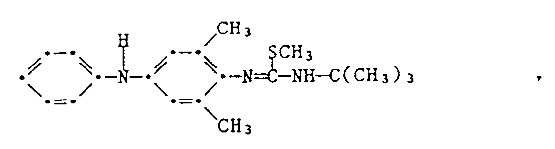 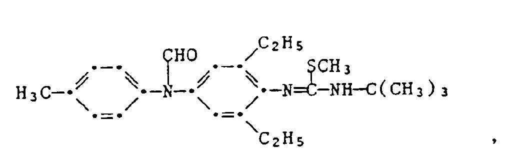 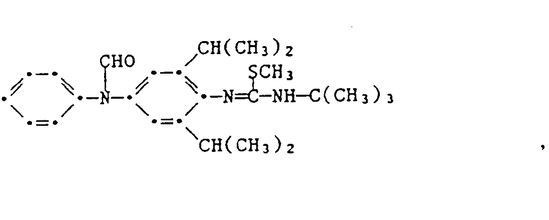 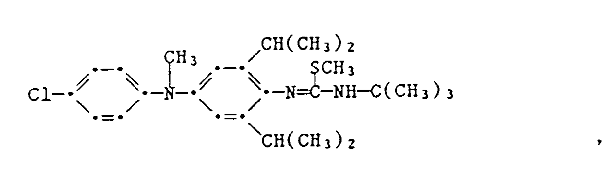 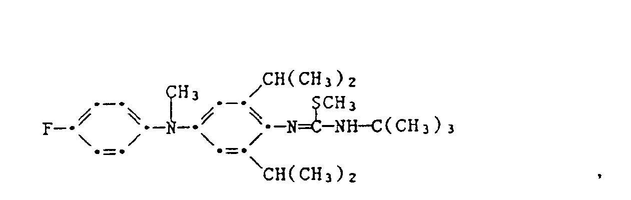 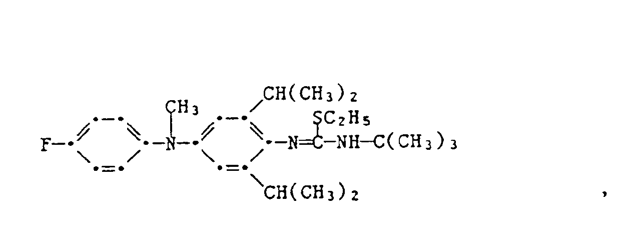 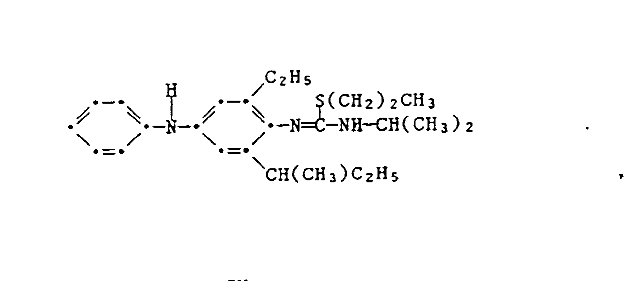 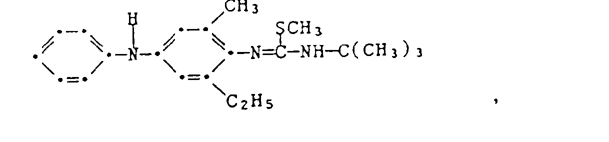 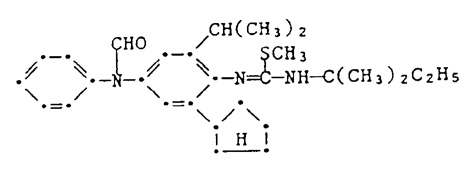 et

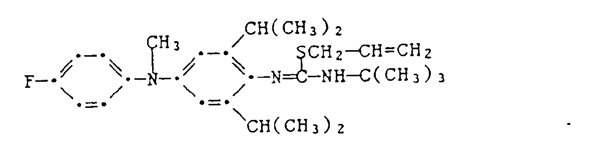

8. Procédé selon l'une des revendications 1, 2 ou 5, pour la préparation des composés de formules 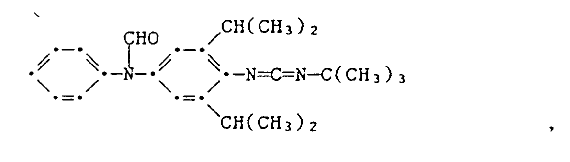 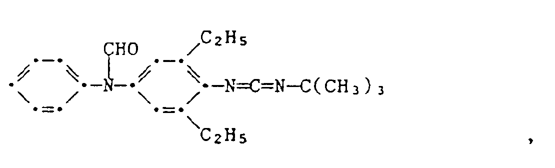 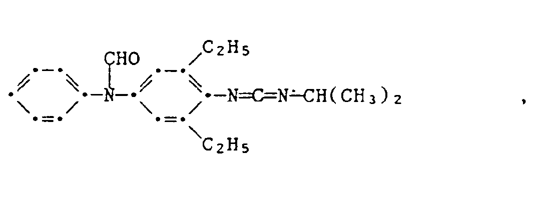 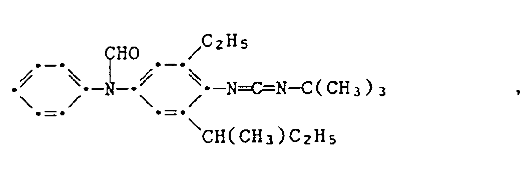 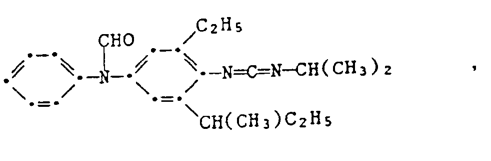 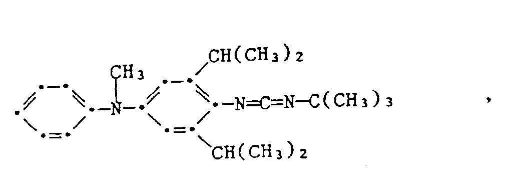 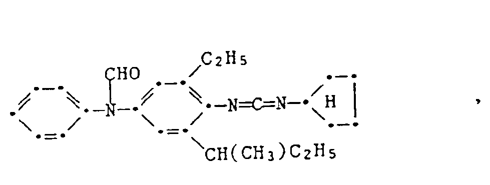 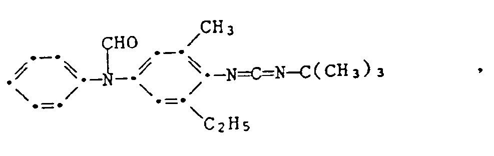 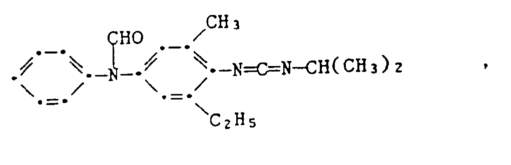 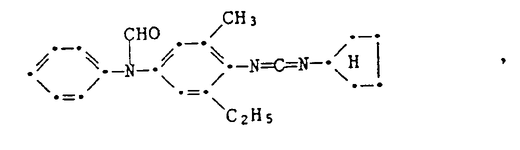 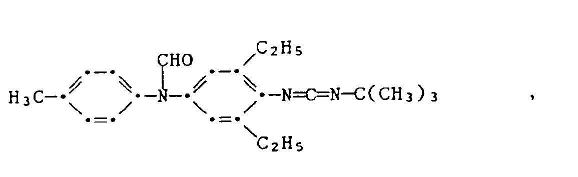 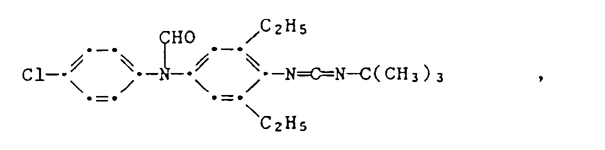 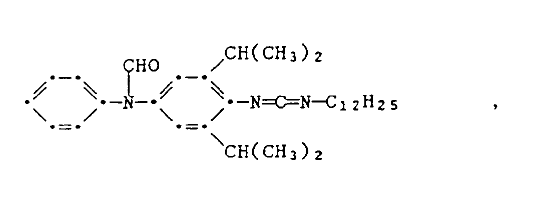 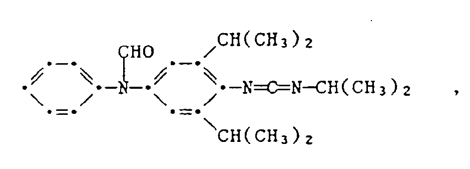 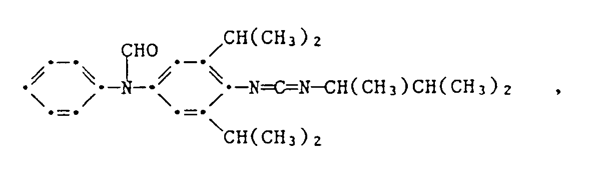 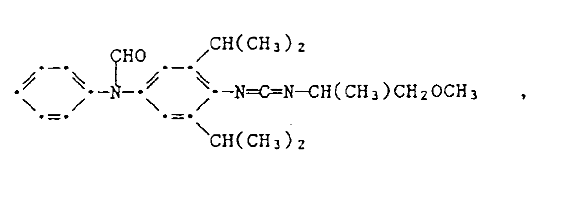 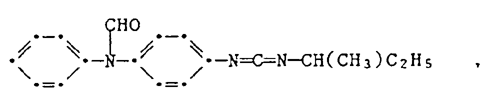 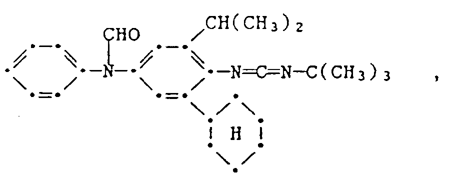 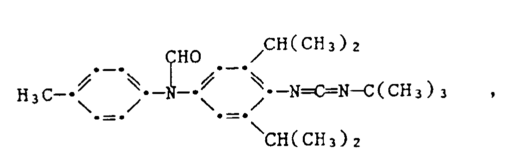 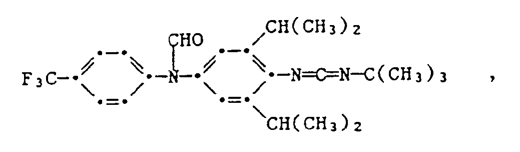 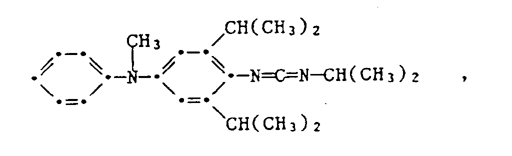 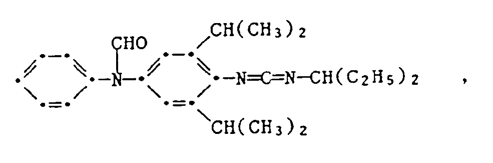 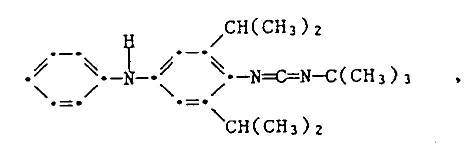 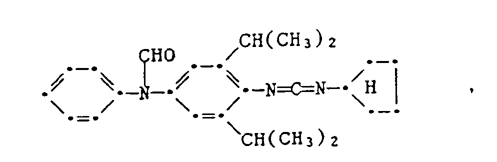 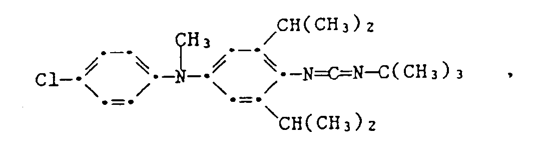 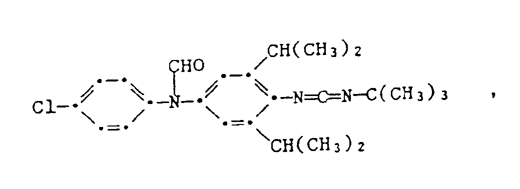 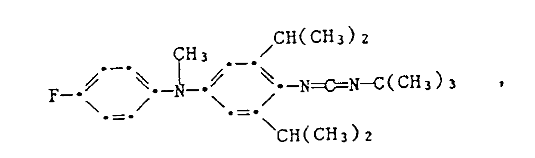 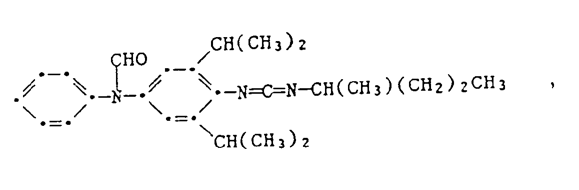 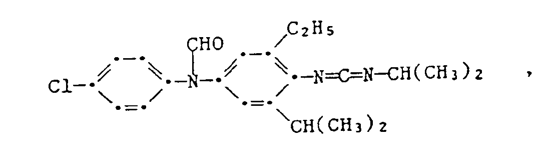 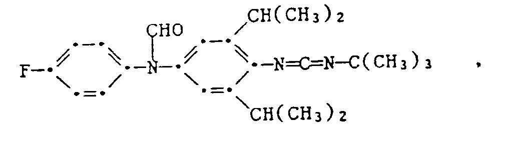 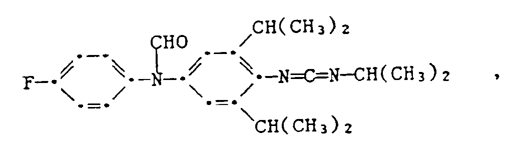 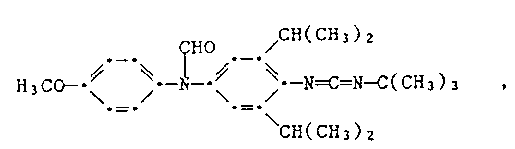 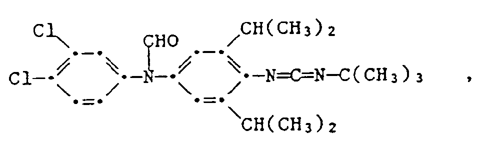 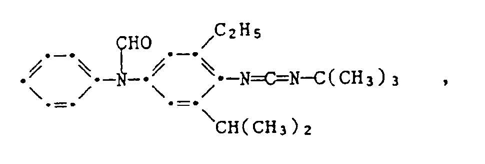 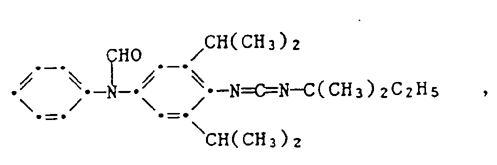 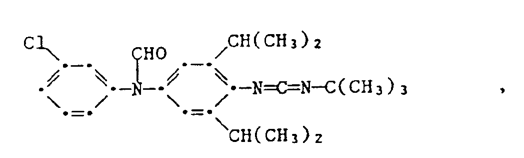 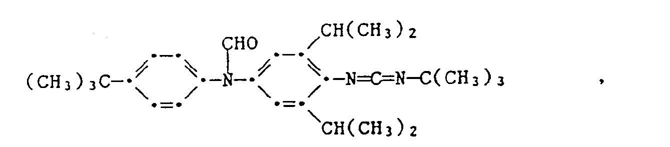 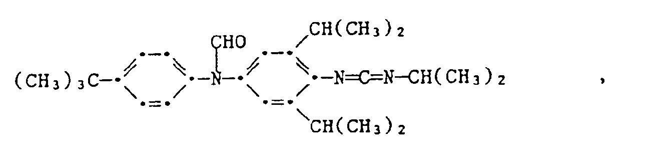 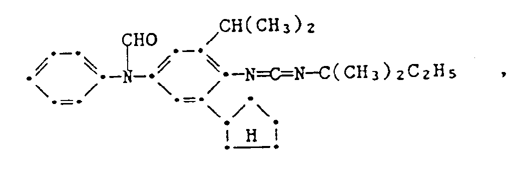 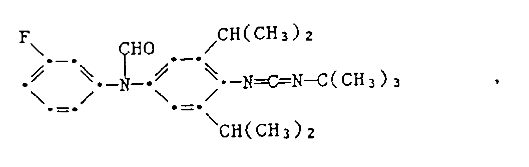 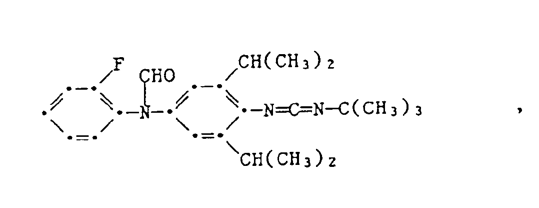 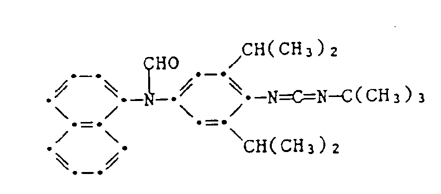 et

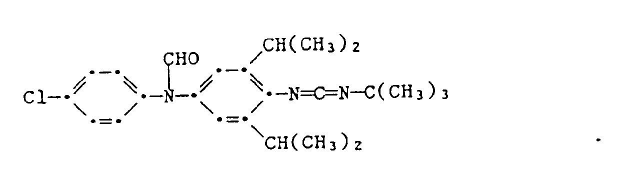

9. Produit pesticide contenant au moins un composant actif consistant en un composé de formule 1 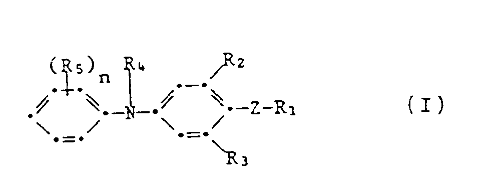 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou en l'un de ses sels d'acide organique ou minéral, avec des véhicules et/ou additifs appropriés.

10. Produit pesticide selon la revendication 9, contenant au moins un composant actif consistant en un composé de formule I dans laquelle R₁ représente un groupe alkyle en C1-C8, un groupe alkyle en C1-C8 portant un ou plusieurs substituants halogéno et/ou alcoxy en C1-C5, ou un groupe cycloalkyle en C3-C8 ; R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4; n est égal à 0, 1 ou 2 ; Z représente -NH-CS-NH-, -N=C(SR₆)-NH- ou -N=C=N- ; et R₆ représente un groupe alkyle en C1-C4.

11. Produit pesticide selon revendication 10, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -NH-CS-NH-.

12. Produit pesticide selon revendication 10, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; Z représente -N=C(SR₆)-NH- ; et R₆ représente un groupe alkyle en C1-C4.

13. Produit pesticide selon revendication 10, pour lequel R₁ représente un groupe alkyle en C1-C5 ou cyclopentyle ; R₂ et R₃ représentent chacun un groupe alkyle en C1-C4 ; R₄ représente l'hydrogène, un groupe méthyle ou -CHO ; R₅ représente un groupe méthyle ; n est égal à 0 ou 1 ; et Z représente -N=C=N-.

14. Utilisation d'un composé de formule 1 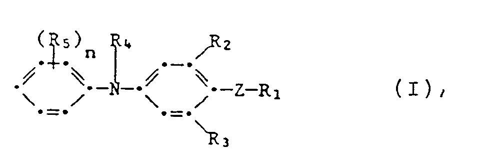 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou de l'un de ses sels d'acide organique ou minéral, pour la lutte contre les parasites des animaux et des végétaux.

15. Utilisation selon la revendication 14 pour la lutte contre les insectes et les arachnides.

16. Procédé pour combattre les parasites des animaux et des végétaux, caractérisé en ce que l'on met les parasites, dans leurs divers stades de développement, en contact avec un composé de formule 1 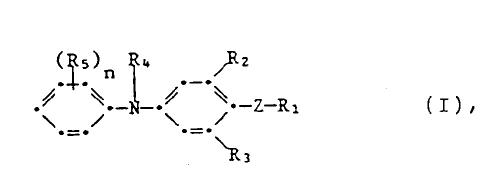 dans laquelle
R₁ représente un groupe alkyle en C1-C12, un groupe alkyle en C1-C12 portant un ou plusieurs substituants choisis parmi les halogènes et/ou les groupes alcoxy en C1-C6, un groupe cycloalkyle en C3-C8, un groupe cycloalkyle en C3-C8 portant un ou plusieurs substituants alkyle en C1-C3, ou un groupe (cycloalkyle en C3-C8)-alkyle en C1-C4 ;
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un halogène, un groupe alkyle en C1-C4, un groupe alkyle en C1-C4 portant un ou plusieurs substituants halogéno, un groupe alcoxy en C1-C4, un groupe alcoxy en C1-C4 portant un ou plusieurs substituants halogéno, ou un pont (̵CH=CH)̵₂, (̵CH₂)̵₃, (̵CH₂)̵₄ en position 2,3 ou 3,4 ;
n est égal à 0, 1, 2, 3 ou 4 ;
Z représente -NH-CS-NH-, -N=C(SR₆)-NH- ou -N=C=N- et
R₆ représente un groupe alkyle en C1-C6 ou allyle, ou l'un de ses sels d'acide organique ou minéral.

17. Procédé de préparation d'un composé de formule V 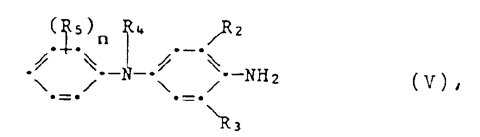 dans laquelle
R₂ et R₃ représentent chacun un groupe alkyle en C1-C5 ou cycloalkyle en C5-C6 ;
R₄ représente l'hydrogène, un groupe méthyle ou -CHO ; chacun des symboles
R₅ représente un groupe alkyle en C1-C4, un groupe alkyle en Cₗ-C₄ portant un ou plusieurs substituants halogéno, un groupe alcoxy en C1-C4 ou un pont (̵CH=CH)̵₂, (̵CH₂)̵₃ ou (̵CH₂)̵₄ en position 2,3 ou 3,4 et
n est égal à 0, 1, 2, 3 ou 4, caractérisé en ce que l'on fait réagir une aniline de formule VI 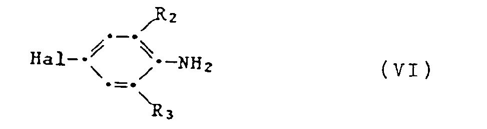 avec un formanilide de formule VII 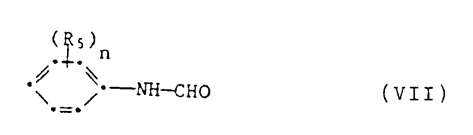 R₂, R₃, R₅ et n ayant les significations indiquées en référence à la formule 1 et Hal représentant un halogène, en particulier le chlore ou le brome.

18. Procédé selon la revendication 17, dans lequel R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4 ; et n est égal à 0, 1 ou 2.

19. Procédé selon la revendication 18, dans lequel R₂ et R₃ représentent chacun un groupe alkyle en C1-C4 ; R₄ représente l'hydrogène, un groupe méthyle ou -CHO ; R₅ représente un groupe méthyle et n est égal à 0 ou 1.

20. Procédé selon la revendication 17 pour la préparation des composés de formules

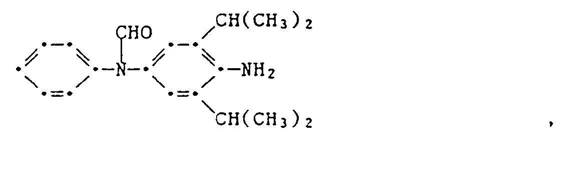

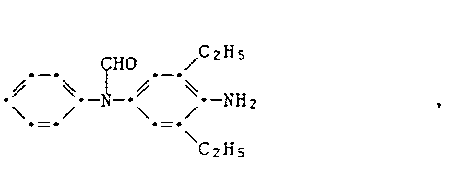

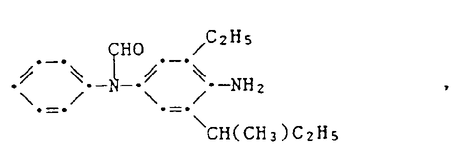

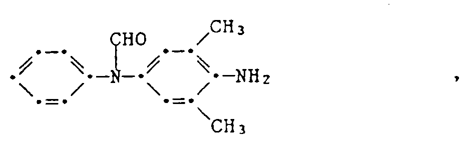

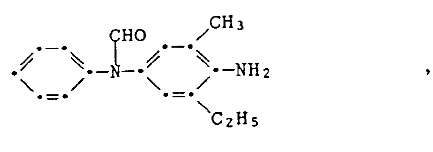

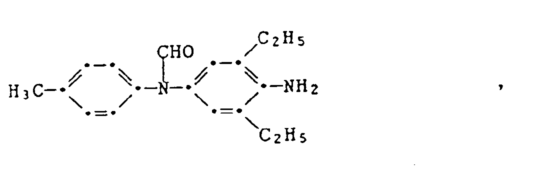

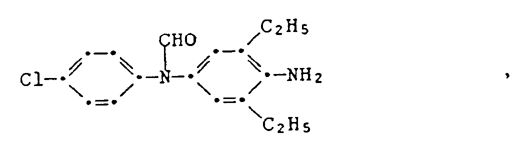

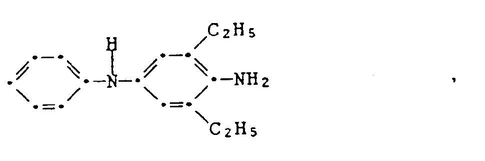

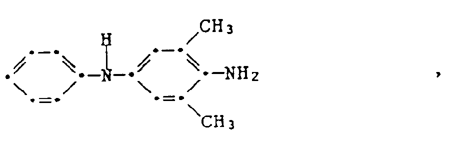

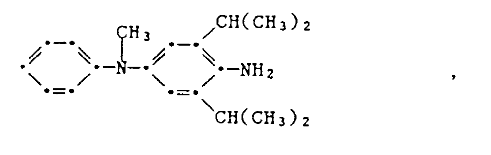

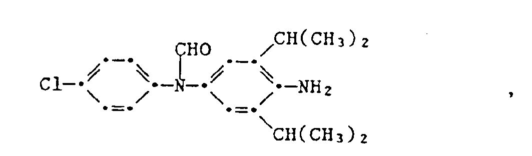

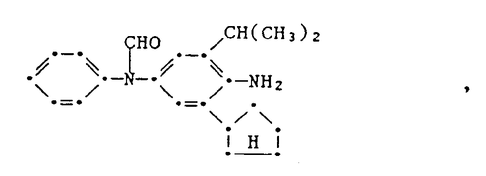

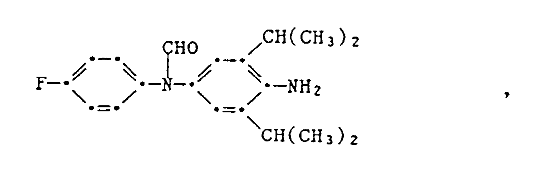

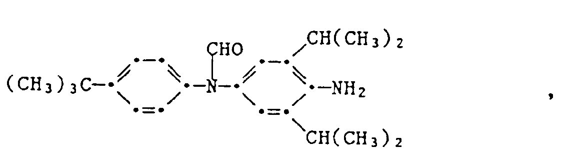

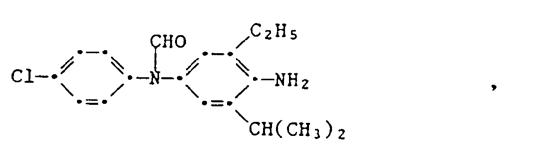

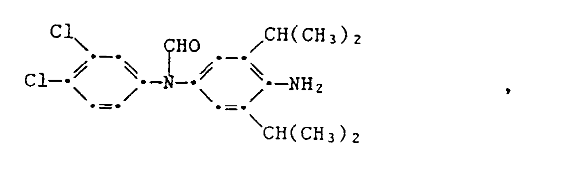

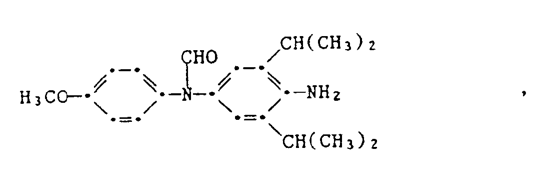

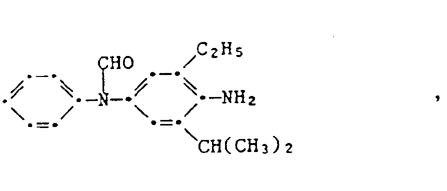

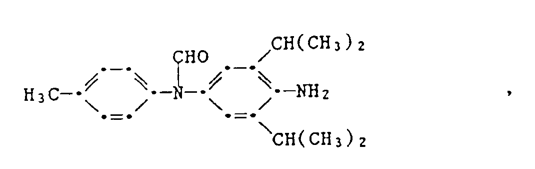

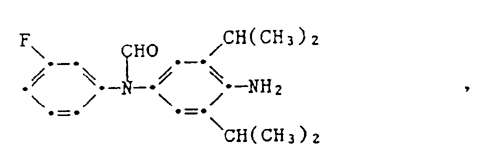

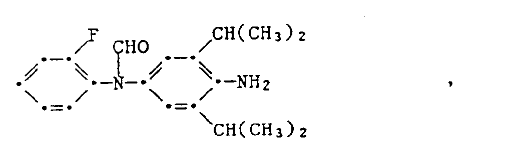

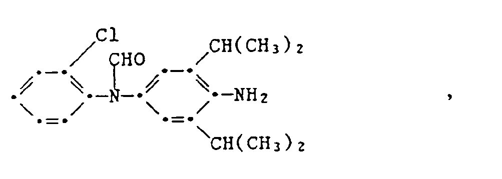

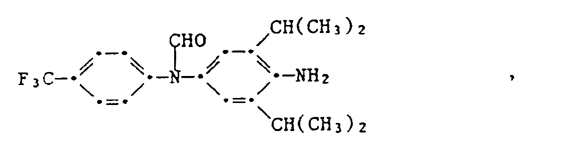

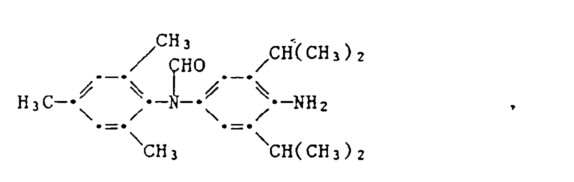

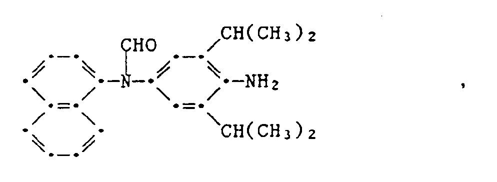

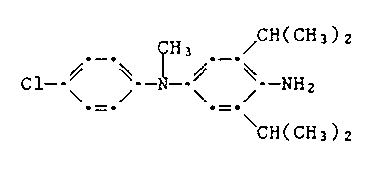

et

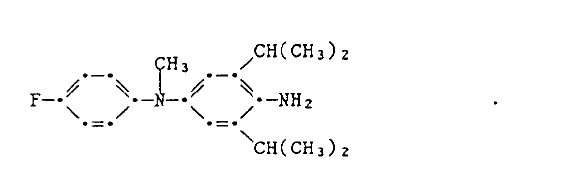

21. Procédé de préparation des composés de formule Il 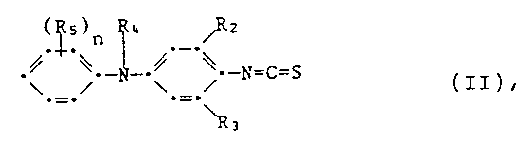 dans laquelle
R₂ et R₃ représentent chacun un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C4, un groupe alkyle en C1-C4 portant un ou plusieurs substituants halogéno, un groupe alcoxy en C1-C4 ou un pont (̵CH=CH)̵₂, (̵CH₂)̵₃ ou (̵CH₂)̵₄ en position 2,3 ou 3,4 et n est égal à 0, 1, 2, 3 ou 4, caractérisé en ce que l'on fait réagir une aminodiphénylamine de formule 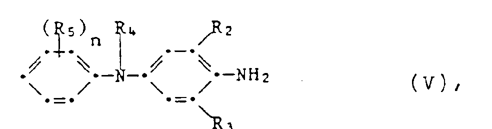 avec le thiophosgène, R₂, R₃, R₄, R₅ et n ayant les significations indiquées en référence à la formule I.

22. Procédé selon la revendication 21, dans lequel R₂ représente un groupe alkyle en C1-C5 ; R₃ représente un groupe alkyle en C1-C5 ou cycloalkyle en C5-C6 ; R₄ représente l'hydrogène, un groupe méthyle ou -CHO ; chacun des symboles R₅ représente un halogène, un groupe alkyle en C1-C3 ou un pont (̵CH=CH)̵₂ ou (̵CH₂)̵₃ en position 3,4 ; et n est égal à 0, 1 ou 2.

23. Procédé selon la revendication 22, dans lequel R₂ et R₃ représentent chacun un groupe alkyle en C1-C4 ; R₄ représente l'hydrogène, un groupe méthyle ou -CHO ; R₅ représente un groupe méthyle et n est égal 0 ou 1.

24. Procédé selon la revendication 21, pour la préparation des composés de formules 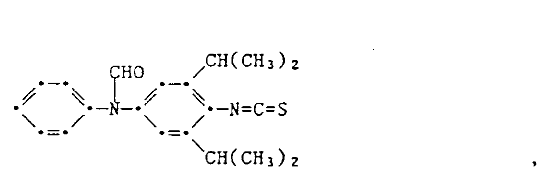 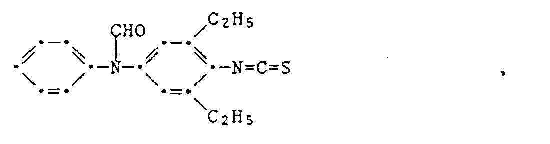 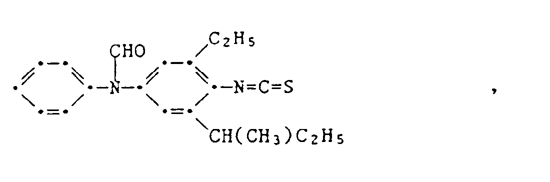 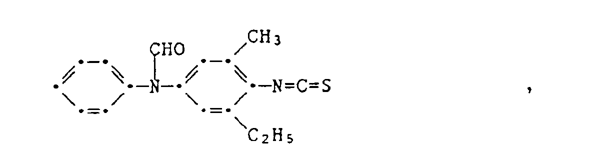 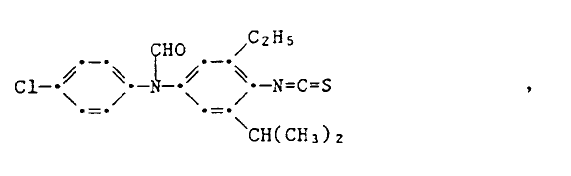 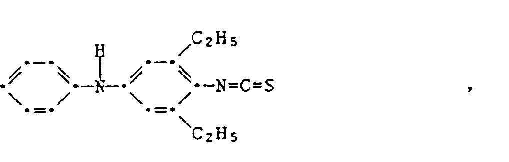 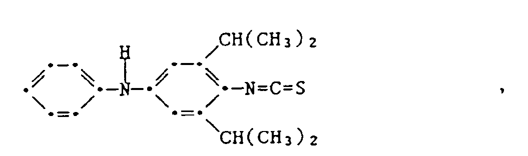 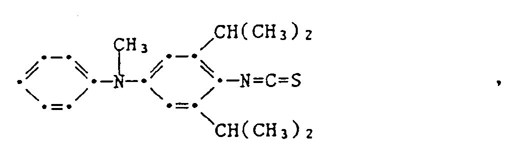 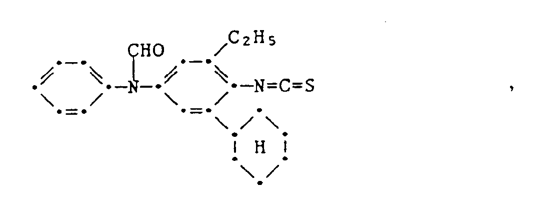 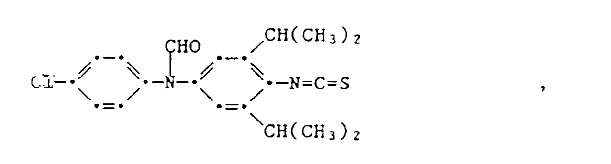 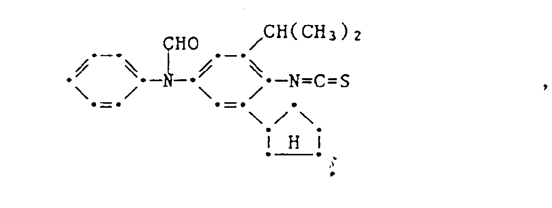 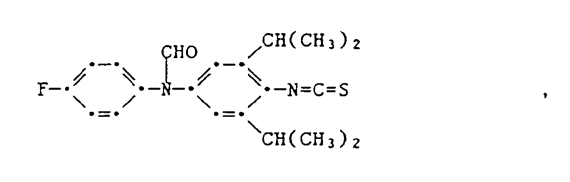 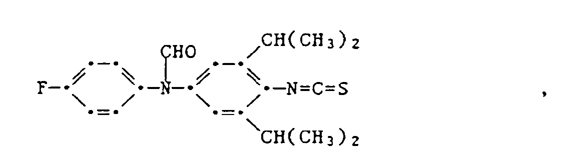 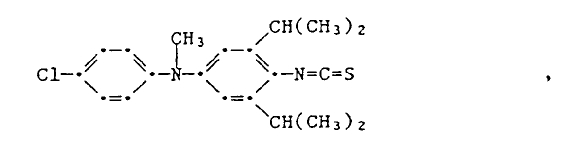 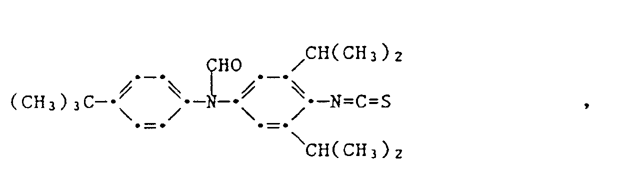 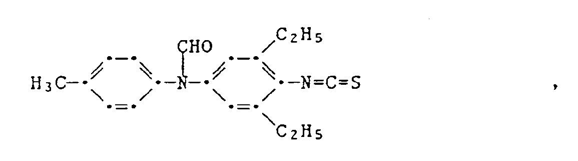 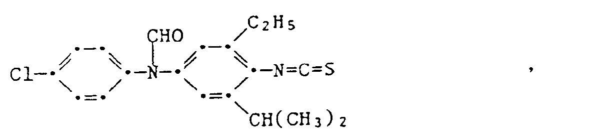 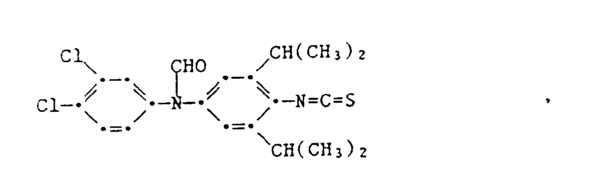 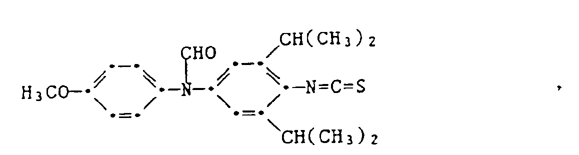 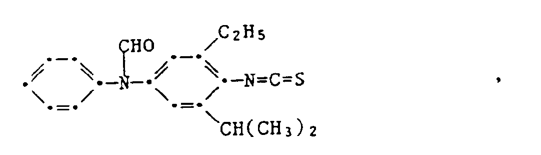 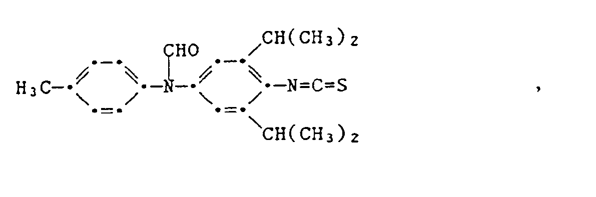 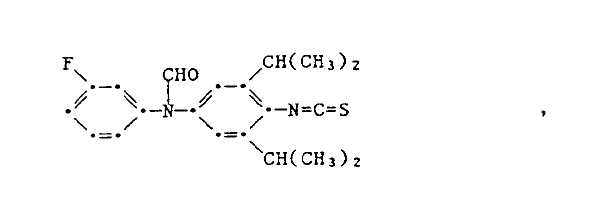 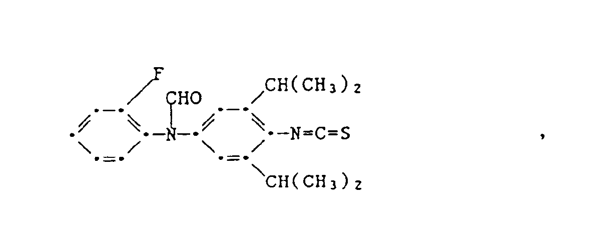 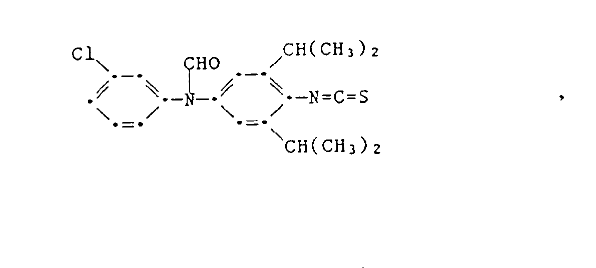 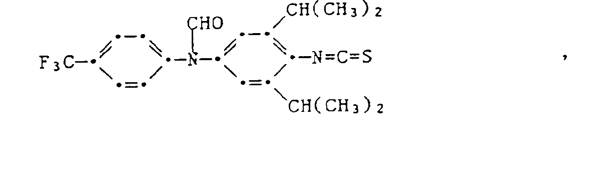 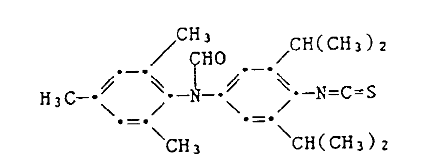 et
